(19) Europäisches Patentamt / European Patent Office / Office européen des brevets

(11) **EP 2 761 519 B1**

(12) **EUROPEAN PATENT SPECIFICATION**

(45) Date of publication and mention of the grant of the patent:
**18.03.2026 Bulletin 2026/12**

(21) Application number: **12835159.0**

(22) Date of filing: **26.09.2012**

(51) International Patent Classification (IPC):
***G16C 10/00*** *(2019.01)* ***G16B 15/00*** *(2019.01)*
***G16B 15/30*** *(2019.01)* ***G06F 30/20*** *(2020.01)*
*G16C 20/30* *(2019.01)*

(52) Cooperative Patent Classification (CPC):
**G16B 15/00; G06F 30/20; G16B 15/30; G16C 10/00;** G06F 2111/10; G16C 20/30

(86) International application number:
**PCT/JP2012/075576**

(87) International publication number:
**WO 2013/047881 (04.04.2013 Gazette 2013/14)**

(54) **SIMULATION APPARATUS FOR PREDICTING BEHAVIOR OF MASS POINT SYSTEM**

SIMULATIONSVORRICHTUNG ZUR VERHALTENSVORHERSAGE EINES MASSENPUNKTESYSTEMS

APPAREIL ET PROCÉDÉ DE SIMULATION POUR PRÉDIRE LE COMPORTEMENT D'UN SYSTÈME DE POINTS DE MASSE, ET PROGRAMME ET SUPPORT D'ENREGISTREMENT POUR METTRE EN OEUVRE LE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.09.2011 JP 2011208444**
**21.09.2012 JP 2012208211**

(43) Date of publication of application:
**06.08.2014 Bulletin 2014/32**

(73) Proprietor: **FUJIFILM Corporation**
**Tokyo 106-8620 (JP)**

(72) Inventors:
- **TSUMURA, Kyosuke**
  **Tokyo 107-0052 (JP)**
- **WATANABE, Hiroyuki**
  **Tokyo 107-0052 (JP)**
- **OGAWA, Tomohiro**
  **Tokyo 107-0052 (JP)**
- **NOGUCHI, Takafumi**
  **Tokyo 107-0052 (JP)**

(74) Representative: **Klunker IP**
**Patentanwälte PartG mbB**
**Destouchesstraße 68**
**80796 München (DE)**

(56) References cited:
**JP-A- 2004 519 026** **JP-A- 2004 527 027**

- **BARBATTI M.: "Nonadiabatic dynamics with trajectory surface hopping method", WILEY INTERDISCIPLINARY REVIEWS: COMPUTATIONAL MOLECULAR SCIENCE, vol. 1, no. 4, 6 May 2011 (2011-05-06), pages 620 - 633, XP055187134, ISSN: 1759-0876, DOI: 10.1002/wcms.64**
- **GIESE K. ET AL: "Multidimensional quantum dynamics and infrared spectroscopy of hydrogen bonds", PHYSICS REPORTS, NORTH-HOLLAND, AMSTERDAM, NL, vol. 430, no. 4, 1 August 2006 (2006-08-01), pages 211 - 276, XP024926265, ISSN: 0370-1573, [retrieved on 20060801], DOI: 10.1016/J.PHYSREP.2006.04.005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- VALORANI M. ET AL: "The G-Scheme: A framework for multi-scale adaptive model reduction", JOURNAL OF COMPUTATIONAL PHYSICS, LONDON, GB, vol. 228, no. 13, 20 July 2009 (2009-07-20), pages 4665 - 4701, XP026122563, ISSN: 0021-9991, [retrieved on 20090320], DOI: 10.1016/J.JCP.2009.03.011
- FUKUNISHI, Y.: "The Filling Potential Method: A Method for Estimating the Free Energy Surface forProtein-Ligand Docking", THE JOURNAL OF PHYSICAL CHEMISTRY B, vol. 107, no. 47, 2003, pages 13201 - 13210, XP055148882
- MARUMORI, T.: "Self-Consistent Collective-Coordinate Method for the Large-Amplitude Nuclear Collective Motion", PROGRESS OF THEORETICAL PHYSICS, vol. 64, no. 4, October 1980 (1980-10-01), pages 1294 - 1314, XP003031014
- KOMATSUZAKI, T.: "Dynamical hierarchy in transition states: Why andhow does a system climb over the mountain?", PROCEEDINGS OF THE NATIONAL ACADEMY OF SCIENCES OF THE UNITED STATES OF AMERICA, vol. 98, no. 14, 2001, pages 7666 - 7671, XP055063924
- OKUNBOR, D.I.: "Canonical numerical methods for molecular dynamics simulations", JOURNAL OF COMPUTATIONAL CHEMISTRY, vol. 15, no. 1, 1994, pages 72 - 79, XP055063928

## Description

### Technical Field

The present invention relates to a simulation apparatus and simulation method for predicting dynamic behavior of a modeled mass point system using a computer. The invention also relates to a program and recording medium for implementing the method.

### Background Art

Along with the development of computer technology, researches have been conducted intensively to try to logically clarify dynamic large deformation behavior of biological macromolecules, such as proteins, nucleic acids, lipids, and polysaccharides in atomic level through simulations using theoretical calculations. More specifically, such researches may include drug screening in which affinity between a target protein and a binding candidate molecule (target molecule for analysis as to whether or not having binding affinity with the target protein) is theoretically predicted and the analysis of folding mechanism of protein in which a construction mechanism of three-dimensional structure from primary sequence of protein is revealed to theoretically construct a higher dimensional structure from the primary structure.

Simulation methods for predicting dynamic behavior of biological macromolecules may include, for example, a molecular dynamics method capable of performing a simulation even for a macromolecule in atomic level and simulation methods based on Monte Carlo method as described, for example, in the following non-patent documents: T.J.A. Ewing and I.D. Kuntz, "Critical evaluation of search algorithms for automated molecular docking and database screening", Journal of Computational Chemistry, Vol. 18, Issue 9, pp. 1175-1189, 1997, G.M. Morris et al., "Automated docking using a Lamarckian genetic algorithm and an empirical binding free energy function", Journal of Computational Chemistry, Vol. 19, Issue 14, pp. 1639-1662, 1998, M. Rarey et al., "A Fast Flexible Docking Method using an Incremental Construction", Journal of Molecular Biology, Vol. 261, Issue 3, pp. 470-489, 1996, R. Abagyan et al., "ICM - A new method for protein modeling and design: Applications to docking and structure prediction from the distorted native conformation", Journal of Computational Chemistry, Vol. 15, Issue 5, pp. 488-506, 1994, G. Jones et al., "Development and validation of a genetic algorithm for flexible docking", Journal of Molecular Biology, Vol. 267, Issue 3, pp. 727-748, 1997, R.A. Friesner et al., "Glide: A New Approach for Rapid, Accurate Docking and Scoring. 1. Method and Assessment of Docking Accuracy", Journal of Medicinal Chemistry, Vol. 47, Issue 7, pp. 1739-1749, 2004, T.A. Halgren et al., "Glide: A New Approach for Rapid, Accurate Docking and Scoring. 2. Enrichment Factors in Database Screening", Journal of Medicinal Chemistry, Vol. 47, Issue 7, pp. 1750-1759, 2004. According to the molecular dynamics method, time evolution of a polyatomic system may be sequentially traced by a small time interval according to a motion equation. As many local minimums or many energy barriers are present on the potential surface of a polyatomic system that includes a biological macromolecule, the aforementioned method has a problem that the state of a polyatomic system is trapped by a local minimum close to the initial structure, thereby requiring a huge amount of time for the calculation. This problem also applies to the methods based on the Monte Carlo method.

### Summary of the Invention

The invention is defined in the appended claims.

### Disclosure of Invention

Consequently, as a method for solving the problem of trapping of the local minimum, a calculation method in which a "compulsive force" is applied to the calculation based on the molecular dynamics method to escape from the trap of local minimum is known. For example, Japanese Unexamined Patent Publication No. 2005-267592 and PCT Japanese Publication No. 2005-524129, PCT Japanese Republication No. 2006/068271, and non-patent documents, Y. Fukunishi et al., "The Filling Potential Method: A Method for Estimating the Free Energy Surface for Protein-Ligand Docking", THE JOURNAL OF PHYSICAL CHEMISTRY B, Vol. 107, Issue 47, pp. 13201-13210, 2003, and Y. Sugita and Y. Okamoto, "Replica-exchange molecular dynamics method for protein folding", Chemical Physics Letters, Vol. 314, Issues 1-2, pp. 141-151, 1999, disclose that virtual interactions are successively introduced so that a polyatomic system never takes the same structure again or structures found in accelerated motion of a polyatomic system in the high-temperature phase are successively reflected in the motion of the polyatomic system in the low-temperature phase. Such introduction of mutual interactions and the like may accelerate the route searching on the potential surface through which the polyatomic system passes, thereby allowing dynamic behavior of the biological macromolecule to be calculated.

The calculation based on a motion equation describing a mass point system representing a modeled biological macromolecule or the like and using molecular dynamics for predicting behavior of the mass point system through integration with respect to time, however, poses a problem that the calculation time of the simulation and calculation accuracy are in a trade-off relationship. More specifically, in the simulation methods described in Japanese Unexamined Patent Publication No. 2005-267592 and PCT Japanese Publication No. 2005-524129, PCT Japanese Republication No. 2006/068271, and non-patent documents, Y. Fukunishi et al., "The Filling Potential Method: A Method for Estimating the Free Energy Surface for Protein-Ligand Docking", THE JOURNAL OF PHYSICAL CHEMISTRY B, Vol. 107, Issue 47, pp. 13201-13210, 2003 and Y. Sugita and Y. Okamoto, "Replica-exchange molecular dynamics method for protein folding", Chemical Physics Letters, Vol. 314, Issues 1-2, pp. 141-151, 1999, the order of motion inherent to the mass point system is destroyed by the applied "compulsive force", whereby, as a calculation result, a behavior of unrealistically large deformation is calculated, although the calculation time is reduced. This tendency becomes more significant as the exertion of the "compulsive force" is increased in order to escape from a trap of local minimum more efficiently.

The present invention has been developed in view of the circumstances described above, and it is an object of the present invention to provide a simulation apparatus and simulation method capable of improving calculation accuracy while reducing calculation time in a simulation for predicting dynamic behavior of a mass point system. It is a further object of the present invention to provide a program and recording medium for implementing the method.

In order to solve the aforementioned problems, a simulation apparatus according to the present disclosure is an apparatus for predicting behavior of a mass point system constituted by modeled N mass points, the apparatus including:

a coordinate setting means for setting slow coordinates which are M coordinates mainly assuming a structural change in the mass point system based on 3N mass point coordinates describing a structure of the mass point system and fast coordinates which are coordinates describing the structure of the mass point system and being independent of the slow coordinates;

a coordinate extraction means for obtaining a structure of the fast coordinates as a function of the slow coordinates by subordinating the fast coordinates to the slow coordinates and obtaining, by taking into account influence of a change in the fast coordinates on the slow coordinates due to a change in the slow coordinates, a structure of the slow coordinates as a function of K collective coordinate(s) of a general coordinate which is associated with the slow coordinates by a canonical transformation, wherein the general coordinate is constituted by a variable component that varies with time and an invariable component that serves as a constant with respect to time and the K collective coordinate (s) is the variable component of the general coordinate; and

an inverse transformation means for predicting time evolution of the mass point system based on the collective coordinate (s) as a function of time, which can be obtained as a solution of a motion equation with respect to the collective coordinate (s), the structure of the slow coordinates, and the structure of the fast coordinates.

K, M, and N herein satisfy the relationship $K<M<3N$ and each representing an integer not less than 1.

The term "a structure of a mass point system" as used herein refers to a three-dimensional structure formed by N mass points constituting the mass point system.

The term "slow coordinates mainly assuming a structural change in the mass point system" as used herein refers to coordinates that exert large influence on the formation of three-dimensional structure of the mass point system.

The term "setting slow coordinates" as used herein refers to setting, as the slow coordinates, some of the mass point coordinates themselves, coordinates that can be defined by combining the mass point coordinates, or a combination thereof. The same applies to the fast coordinates.

Preferably, the coordinate extraction means of the simulation apparatus according to the present disclosure is a means that obtains the structure of the slow coordinates by:

performing a first step for obtaining potential energy V represented as a function of the slow coordinates and the fast coordinates;

performing a second step for subordinating the fast coordinates to the slow coordinates according to an adiabatic approximation condition using the potential energy;

performing, under a current state of the slow coordinates and the fast coordinates, a third step for obtaining a differential coefficient of the potential energy with respect to the slow coordinates by taking into account the influence described above;

performing, under the differential coefficient of the potential energy, a fourth step for obtaining a differential coefficient of the slow coordinates with respect to the collective coordinate(s) according to a basic equation of self-consistent collective coordinate method using the differential coefficient of the potential energy;

performing, under the differential coefficient of the slow coordinates, a fifth step for updating the collective coordinate (s) by a small amount and obtaining updated slow coordinates;

performing, under the updated slow coordinates, a sixth step for performing structural relaxation on the fast coordinates subordinated to the slow coordinates; and

thereafter, repeating the third to sixth steps based on the slow coordinates and the fast coordinates in a state after the structural relaxation of the fast coordinates.

The term "under the slow coordinates and the fast coordinates in a current state" as used herein refers to that the third step is performed under the slow coordinates and fast coordinates in a state set by the coordinate setting means in the first time, and under the slow coordinates and fast coordinates in a state after the structural relaxation performed on the fast coordinates in the immediately preceding sixth step in the second time on.

Preferably, in the simulation apparatus of the present disclosure, the influence described above is taken into account by a method that uses at least one of Formulae 1 to 3 given below.

$$\frac{\partial}{\partial Rs_i} V_{eff}(Rs) = \frac{\partial}{\partial Rs_i} V(Rs, R_F)\bigg|_{R_F = R_F(Rs)}$$

Formula 1

$$\begin{aligned}\frac{\partial^2}{\partial Rs_i \partial Rs_j} V_{eff}(Rs) &= \frac{\partial^2}{\partial Rs_i \partial Rs_j} V(Rs, R_F)\bigg|_{R_F = R_F(Rs)} \\ &+ \left( \sum_{\beta=1}^{3N-M} X_\beta{}^j(Rs) \frac{\partial^2}{\partial Rs_i \partial R_{F_\beta}} V(Rs, R_F)\bigg|_{R_F = R_F(Rs)} + (i \leftrightarrow j) \right) \\ &+ \sum_{\alpha=1}^{3N-M} \sum_{\beta=1}^{3N-M} X_\alpha{}^i(Rs) X_\beta{}^j(Rs) \frac{\partial^2}{\partial R_{F_\alpha} \partial R_{F_\beta}} V(Rs, R_F)\bigg|_{R_F = R_F(Rs)}\end{aligned}$$

Formula 2

$$\begin{aligned}\frac{\partial^3}{\partial Rs_i \partial Rs_j \partial Rs_k} V_{eff}(Rs) &= \frac{\partial^3}{\partial Rs_i \partial Rs_j \partial Rs_k} V(Rs, R_F)\bigg|_{R_F = R_F(Rs)} \\ &+ \left( \sum_{\gamma=1}^{3N-M} X_\gamma{}^k(Rs) \frac{\partial^3}{\partial Rs_i \partial Rs_j \partial R_{F_\gamma}} V(Rs, R_F)\bigg|_{R_F = R_F(Rs)} + (i \leftrightarrow k) + (j \leftrightarrow k) \right) \\ &+ \left( \sum_{\alpha=1}^{3N-M} \sum_{\beta=1}^{3N-M} X_\alpha{}^i(Rs) X_\beta{}^j(Rs) \frac{\partial^3}{\partial R_{F_\alpha} \partial R_{F_\beta} \partial Rs_k} V(Rs, R_F)\bigg|_{R_F = R_F(Rs)} + (i \leftrightarrow k) + (j \leftrightarrow k) \right) \\ &+ \sum_{\alpha=1}^{3N-M} \sum_{\beta=1}^{3N-M} \sum_{\gamma=1}^{3N-M} X_\alpha{}^i(Rs) X_\beta{}^j(Rs) X_\gamma{}^k(Rs) \frac{\partial^3}{\partial R_{F_\alpha} \partial R_{F_\beta} \partial R_{F_\gamma}} V(Rs, R_F)\bigg|_{R_F = R_F(Rs)}\end{aligned}$$

Formula 3

As used herein, the following refer to the following:

i, j, and k each represents an integer in the range from 1 to M;
$\alpha$, $\beta$, and $\gamma$ each represents an integer in the range from 1 to 3N-M;
$R_{Si}$ represents $i^{th}$ slow coordinate in the mass point system;
$R_{F\alpha}$ represents $\alpha^{th}$ fast coordinate in the mass point system;
$R_S$ represents ($R_{S1}$, $R_{S2}$, ------, $R_{SM}$);
$R_F$ represents ($R_{F1}$, $R_{F2}$, ------, $R_{F(3N-M)}$) ;
$R_F$ ($R_S$) represents the fast coordinates subordinated to the slow coordinates;
V ($R_S$, $R_F$) represents the potential energy of a mass point system represented by the slow coordinates and the fast coordinates; and
$V_{eff}(R_S)$ represents effective potential energy to be obtained by substituting $R_E(R_S)$ into V($R_S$, $R_F$).

In Formula 2, ($i \leftrightarrow j$) in the third term represents a term derived by mutually replacing alphabets i and j in the second term (that is, a term derived by replacing i with j and j with i in the second term, the same applies hereinafter).

In Formula 3, (i↔k) in the third term represents a term derived by mutually replacing alphabets i and k in the second term, (j↔k) in the fourth term represents a term derived by mutually replacing alphabets j and k in the second term, (i↔k) in the sixth term represents a term derived by mutually replacing alphabets i and k in the fifth term, and (j↔k) in the seventh term represents a term derived by mutually replacing alphabets j and k in the fifth term.

Further, in Formulae 1 to 3, Formula 4 given below is used.

$$X_\alpha{}^i(Rs) \equiv -\sum_{\beta=1}^{3N-M} K_{\alpha\beta}^{-1}(Rs) J^{\beta i}(Rs)$$

Formula 4

where: $K_{\alpha\beta}^{-1}(R_S)$ represents an inverse matrix of $K^{\alpha\beta}(R_S)$, and $K^{\alpha\beta}(R_S)$ and $J^{\alpha i}(R_S)$ are defined by Formulae 5 and 6 given below respectively.

$$K^{\alpha\beta}(Rs) \equiv \frac{\partial^2}{\partial R_{F_\alpha} \partial R_{F_\beta}} V(Rs, R_F)\Big|_{R_F = R_F(Rs)}$$

Formula 5

$$J^{\alpha j}(Rs) \equiv \frac{\partial^2}{\partial R_{F_\alpha} \partial Rs_j} V(Rs, R_F)\Big|_{R_F = R_F(Rs)}$$

Formula 6

Preferably, in the simulation apparatus of the present disclosure, the adiabatic approximation condition is Formula 7 given below.

$$\frac{\partial}{\partial R_{F_\alpha}} V(Rs, R_F) = 0 \qquad \textit{for} \quad \alpha = 1, \cdots, 3N - M$$

Formula 7

Preferably, in the simulation apparatus of the present disclosure, the number K of the collective coordinate (s) satisfies K=1, and the basic equation of self-consistent collective coordinate method is represented by Formulae 8 and 9 given below.

$$\frac{\mathrm{d}Rs_i}{\mathrm{d}q^1} = m_i^{-1/2} \varphi_i(Rs) \qquad \textit{for} \quad i = 1, \cdots, M$$

Formula 8

$$\sum_{j=1}^{M} \left( m_i^{-1/2} m_j^{-1/2} \frac{\partial^2}{\partial Rs_i \partial Rs_j} V_{eff}(Rs) - \Lambda(Rs)\, \delta_{ij} \right) \varphi_j(Rs) = 0 \qquad \textit{for} \quad i = 1, \cdots, M$$

Formula 9

As used herein, the following refer to the following:

$q^1$ represents the collective coordinate;
$m_i$ represents a mass of $i^{th}$ slow coordinate in the mass point system;
$\varphi_i(R_S)$ represents $i^{th}$ component of a function (eigenvector) that satisfies Formula 9; and
$\Lambda(R_S)$ represents a function (eigenvalue) that satisfies Formula 9.

Alternatively, it is preferable in the simulation apparatus of the present disclosure that the number K of the collective coordinate (s) satisfies K=1, and the basic equation of self-consistent collective coordinate method is represented by Formulae 10 to 12 given below.

$$\frac{\mathrm{d}Rs_i}{\mathrm{d}q^1} = m_i^{-1/2}\varphi_i(Rs,\lambda) \qquad \mathit{for} \quad i=1,\cdots,M$$

Formula 10

$$\frac{\mathrm{d}\lambda}{\mathrm{d}q^1} = \kappa(Rs,\lambda)$$

Formula 11

$$\sum_{j=1}^{M} m_i^{-1/2} m_j^{-1/2} \frac{\partial^2}{\partial Rs_i \partial Rs_j}\left(\frac{1}{2}\sum_{k=1}^{M}\left(m_k^{-1/2}\frac{\partial}{\partial Rs_k}V_{eff}(Rs)\right)^2 - \lambda\ V_{eff}(Rs)\right)\varphi_j(Rs,\lambda) = m_i^{-1/2}\frac{\partial}{\partial Rs_i}V_{eff}(Rs)\,\kappa(Rs,\lambda) \qquad \mathit{for} \quad i=1,\cdots,M$$

Formula 12

where, $\varphi_i(R_S, \lambda)$ and $\kappa(R_S, \lambda)$ are functions that satisfy Formula 12 and $\varphi_i(R_S, \lambda)$ represents $i^{th}$ component, and $\lambda$ represents an auxiliary coordinate (variable treated as independent of the slow coordinates and as function of the collective coordinate(s)).

Preferably, in the simulation apparatus of the present disclosure, the coordinate extraction means is a means that performs calculation in the fourth step by increasing the number of variables treated as independent of the slow coordinates and as functions of the collective coordinate(s) (i.e., auxiliary coordinates) in solving the basic equation in order to eliminate the arbitrariness of sign of a differential coefficient of the slow coordinates or auxiliary coordinates with respect to the collective coordinate (s) in the basic equation.

Further, in the case where the number of auxiliary coordinates is increased, it is preferable that the coordinate extraction means is a means that performs calculation according to a basic equation represented by Formula 13 given below obtained as a result thereof. Still further, in this case, the number K of the collective coordinate(s) satisfies K=1.

$$\frac{\mathrm{d}\mathbf{Y}}{\mathrm{d}q^\mu} = \mathbf{v}^\mu \qquad \mathit{for} \quad \mu=1,\cdots,K$$

Formula 13

where, Y is a MK+M+K dimensional vector defined by Formula 14 given below, and $v^\mu$ is a solution vector of the inhomogeneous linear equation of Formula 15 given below.

$$\mathbf{Y} \equiv \begin{pmatrix} \sqrt{m_i}Rs_i \\ \varphi_i^\mu \\ \Lambda^\mu \end{pmatrix}$$

Formula 14

$$C\mathbf{v}^\mu = \mathbf{s}^\mu \qquad \mathit{for} \quad \mu=1,\cdots,K$$

Formula 15

C and $s^\mu$ in Formula 15 are defined by Formulae 16 and 17 given below respectively.

$$C \equiv \begin{pmatrix} \sum_{k=1}^{M} V_{,ijk}(Rs)\varphi_k^{\mu} & (V_{,ij}(Rs) - \Lambda^{\mu}\delta_{ij})\delta^{\mu\nu} & -\varphi_i^{\mu}\delta^{\mu\nu} \\ 0 & \varphi_j^{\mu}\delta^{\mu\nu} & 0 \\ \delta_{ij} & 0 & 0 \end{pmatrix}$$

Formula 16

$$\mathbf{s}^{\mu} \equiv \begin{pmatrix} 0 \\ 0 \\ \varphi_i^{\mu} \end{pmatrix} \qquad for \quad \mu = 1, \cdots, K$$

Formula 17

where, $V_{ij}(R_S)$ and $V_{ijk}(R_S)$ are defined by Formulae 18 and 19 given below respectively.

$$V_{,ij}(Rs) \equiv (m_i m_j)^{-1/2} \frac{\partial^2}{\partial Rs_i \partial Rs_j} V_{eff}(Rs)$$

Formula 18

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formula 19

where, $\mu$ and $\nu$ each represents an integer in the range from 1 to K, $q^{\mu}$ represents $\mu^{th}$ collective coordinate, and $\varphi_i^{\mu}$ and $\Lambda^{\mu}$ each represents an auxiliary coordinate.

Alternatively, in the case where the number of auxiliary coordinates is increased, it is preferable that the coordinate extraction means is a means that performs calculation according to a basic equation represented by Formula 20 given below obtained as a result thereof. Further, in this case, the number K of the collective coordinate(s) satisfies K=1.

$$\frac{\mathrm{d}\mathbf{Z}}{\mathrm{d}q^{\mu}} = \sum_{\nu=1}^{K} c^{\mu\nu}\mathbf{w}^{\nu} \qquad for \quad \mu = 1, \cdots, K$$

Formula 20

where, Z is a MK+M+2K dimensional vector defined by Formula 21 given below, $c^{\mu\nu}$ is a constant uniquely determined such that the value represented by Formula 22 given below to be defined with respect to each $\mu$ is minimized, and $w^{\mu}$ represents MK+M+2K dimensional K unit vector (s) spanning a K dimensional singular value space of matrix D defined by Formula 23 given below. Note that $\lambda^{\mu}$ and $\rho^{\mu}$ each represents an auxiliary coordinate independent of $R_S$, like $\varphi_i^{\mu}$.

$$\mathbf{Z} \equiv \begin{pmatrix} \sqrt{m_i} Rs_i \\ \varphi_i^{\mu} \\ \lambda^{\mu} \\ \rho^{\mu} \end{pmatrix}$$

Formula 21

$$\sum_{i=1}^{M}\left(m_i^{1/2}\frac{dRs_i}{dq^{\mu}}-\varphi_i^{\mu}\right)^2$$

Formula 22

$$D \equiv \begin{pmatrix} \sum_{k=1}^{M} V_{,ijk}(Rs)\varphi_k^{\mu} & (V_{,ij}(Rs)-\lambda^{\mu}\delta_{ij})\delta^{\mu\nu} & -\varphi_i^{\mu}\delta^{\mu\nu} & 0 \\ V_{,ij}(Rs) & -\rho^{\nu}\delta_{ij} & 0 & -\varphi_i^{\nu} \\ 0 & \varphi_j^{\mu}\delta^{\mu\nu} & 0 & 0 \end{pmatrix}$$

Formula 23

Preferably, in the simulation apparatus of the present disclosure, the coordinate extraction means is a means that calculates the term of third derivative of the potential energy based on Formula 24 given below. $\Phi_i$ represents ith component of an arbitrary vector and n is defined by Formula 25 given below.

$$\sum_{k=1}^{M} V_{,ijk}(Rs)\cdot\phi_k = \lim_{\Delta x\to 0}\frac{V_{,ij}(Rs+\mathbf{n}\Delta x)-V_{,ij}(Rs-\mathbf{n}\Delta x)}{2\Delta x}$$

Formula 24

$$\mathbf{n} \equiv (m_1^{-1/2}\phi_1,\cdots,m_i^{-1/2}\phi_i,\cdots,m_M^{-1/2}\phi_M)$$

Formula 25

Preferably, in the simulation apparatus of the present disclosure, the coordinate setting means is a means that sets representative coordinates extracted from and representing each characteristic partial structure of the structure of the mass point system as the slow coordinates.

The term "characteristic partial structure" as used herein refers to a partial structure of the structure of the mass point system having morphological and/or functional characteristics.

In the simulation apparatus of the present disclosure, the mass point system may be a polyatomic system that includes a biological macromolecule, the partial structure may be a secondary structure, a building block, or a main chain of the biological macromolecule, and the representative coordinate of each partial structure is a coordinate of each of atoms constituting the partial structure, a coordinate defined by combining coordinates of the atoms, or a pitch of the partial structures.

In the simulation apparatus of the present disclosure, if the mass point system is a polyatomic system that includes a biological macromolecule, it is preferable that the biological macromolecule is a protein, the partial structure is a secondary structure of the protein, and the representative coordinate of the secondary structure is a coordinate of the center of gravity of a group of atoms constituting the secondary structure or a flexion angle of the secondary structure. In this case, it is preferable that the secondary structure is at least one of helix structure, β sheet, turn, loop, and random coil.

Further, in the simulation apparatus of the present disclosure, if the mass point system is a polyatomic system that includes a biological macromolecule, it is preferable that the biological macromolecule is a protein, the partial structure is a residue of the protein, and the representative coordinate of the residue is a coordinate of the center of gravity of a group of atoms constituting the residue.

Still further, in the simulation apparatus of the present disclosure, if the mass point system is a polyatomic system that includes a biological macromolecule, it is preferable that the biological macromolecule is a protein, the partial structure is a main chain of the protein, and the representative coordinate of the main chain is a coordinate of each atom constituting the main chain.

Further, in the simulation apparatus of the present disclosure, if the mass point system is a polyatomic system that includes a biological macromolecule, it is preferable that the biological macromolecule is a nucleic acid, the partial structure is a secondary structure of the nucleic acid, and the representative coordinate of the secondary structure is a coordinate of the center of gravity of a group of atoms constituting the secondary structure or a flexion angle of the secondary structure. In this case, it is preferable that the secondary structure is a helical structure.

Further, in the simulation apparatus of the present disclosure, if the mass point system is a polyatomic system that includes a biological macromolecule, it is preferable that the biological macromolecule is a nucleic acid, the partial structure is a residue of the nucleic acid, and the representative coordinate of the residue is a coordinate of the center of gravity of a group of atoms constituting the residue.

Still further, in the simulation apparatus of the present disclosure, if the mass point system is a polyatomic system that includes a biological macromolecule, it is preferable that the biological macromolecule is a nucleic acid, the partial structure is a main chain of the nucleic acid, and the representative coordinate of the main chain is a coordinate of each atom constituting the main chain.

Further, in the simulation apparatus of the present disclosure, if the mass point system is a polyatomic system that includes a biological macromolecule, it is preferable that the biological macromolecule is a nucleic acid, the partial structure is a helical structure of the nucleic acid, and the representative coordinate of the helical structure is a pitch of the helical structure.

Still further, in the simulation apparatus of the present disclosure, the polyatomic system may include a binding candidate molecule for the biological macromolecule.

A simulation method of the present disclosure is a method for use with the simulation apparatus described above for predicting behavior of a mass point system constituted by modeled N mass points, the method including the steps of:

setting slow coordinates which are M coordinates mainly assuming a structural change in the mass point system based on 3N mass point coordinates describing a structure of the mass point system;
setting fast coordinates which are coordinates describing the structure of the mass point system and being independent of the slow coordinates;
obtaining a structure of the fast coordinates as a function of the slow coordinates by subordinating the fast coordinates to the slow coordinates;
obtaining, by taking into account influence of a change in the fast coordinates on the slow coordinates due to a change in the slow coordinates, a structure of the slow coordinates as a function of K collective coordinate(s) of a general coordinate which is associated with the slow coordinates by a canonical transformation, wherein the general coordinate is constituted by a variable component that varies with time and an invariable component that serves as a constant with respect to time and the K collective coordinate (s) is the variable component of the general coordinate; and
predicting time evolution of the mass point system based on the collective coordinate(s) as a function of time, which can be obtained as a solution of a motion equation with respect to the collective coordinate(s), the structure of the slow coordinates, and the structure of the fast coordinates.

A simulation program of the present disclosure is a program for causing a computer to perform the simulation method described above.

A computer readable recording medium of the present disclosure is a medium on which is recorded the simulation program described above.

The simulation apparatus of the present disclosure includes the coordinate setting means, coordinate extraction means, and inverse transformation means described above, and predicts time evolution of mass point coordinates by introducing hierarchized slow coordinates, extracting, by taking into account influence of a change in fast coordinates on the slow coordinates due to a change in the slow coordinates, a collective coordinate in the collective motion theory that describes collective and intrinsic behavior of a mass point system, and solving the motion equation with respect to the collective coordinate. Thus, the extraction of collective coordinate allows the simulation to be performed in atomic level and the introduction of the slow coordinates allows the number of coordinates handled for extracting the collective coordinate to be reduced. As a result, both improvement of calculation accuracy and reduction in calculation time may be achieved in a simulation for predicting dynamic behavior of a mass point system.

The simulation method of the present disclosure is a method for use with the simulation apparatus described above and predicts time evolution of mass point coordinates by introducing hierarchized slow coordinates, extracting, by taking into account influence of a change in fast coordinates on the slow coordinates due to a change in the slow coordinates, a collective coordinate in the collective motion theory that describes collective and intrinsic behavior of a mass point system, and solving a motion equation with respect to the collective coordinate. Thus, the extraction of collective coordinate allows the simulation to be performed in atomic level and the introduction of the slow coordinates allows the number of coordinates handled for extracting the collective coordinate to be reduced. As a result, both improvement of calculation accuracy and reduction in calculation time may be achieved in a simulation for predicting dynamic behavior of a mass point system.

The program and recording medium of the present disclosure may cause the aforementioned simulation method to be performed, so that improved calculation accuracy and reduced calculation time may be achieved in a simulation for predicting dynamic behavior of a mass point system.

Brief Description of Drawings

Figure 1 is a schematic view illustrating a binding process of a protein and a binding candidate molecule in the case where the induced-fit is taken into account in the simulation method of the present invention.
Figure 2 is a schematic view illustrating a reaction path on a conceptual potential surface of a polyatomic system that includes a protein and a binding candidate molecule in which the induced-fit is taken into account.
Figure 3 is a view conceptually illustrating a process for obtaining a reaction path of a polyatomic system using extraction of collective coordinate.
Figure 4 illustrates a concept of variable transformation and inverse transformation.
Figure 5 is a block diagram of a simulation apparatus according to an embodiment, schematically illustrating a configuration thereof.
Figure 6A is a flowchart schematically illustrating calculation steps of a simulation method according to an embodiment.
Figure 6B is a flowchart schematically illustrating calculation steps of a simulation method according to an embodiment.
Figure 7 schematically illustrates a relationship between slow coordinates $R_S$, fast coordinates $R_F$, and atom coordinates x.
Figure 8 is a graph illustrating a reaction path of a predetermined composite body obtained by a simulation method according to an embodiment.
Figure 9 is a schematic view illustrating a binding process of the composite body shown in the graph of Figure 8, in which A and B illustrate a starting state and a final state of the binding process of the composite body respectively.
Figure 10 is a graph illustrating a result of comparison between calculation in which the arbitrariness of the sign is eliminated and calculation in which the arbitrariness of the sign is not eliminated.

Best Mode for Carrying Out the Invention

Hereinafter, an embodiment of the present invention will be described with reference to the accompanying drawings, but it is to be understood that the present invention is not limited to the embodiment. Note that each component in the drawings is not necessarily drawn to scale for ease of visual recognition.
Before going into details, the technical idea on which the present invention bases and the background that has led to the present invention will be described first in order to clarify technical advantages of the present invention over the prior art. For the sake of clarity of the explanation, a specific description will be made of a case in which dynamic behavior of a polyatomic system that includes a composite body of a biological macromolecule and a binding candidate molecule is predicted.
Prediction of dynamic behavior of a polyatomic system like that described above plays an important role, for example, in a new drug development from a viewpoint of development period and cost reduction. A physiologically active substance, such as a drug, may exhibit the chemical property by binding to a specific protein. As such, it is necessary, in the new drug development, to narrow down the number of candidates that are likely to bind to the target protein from a huge number of compounds ranging from several hundreds of thousands to several millions (drug screening). Further, it is ultimately required to narrow down the drug candidates to about several candidates. In order to efficiently narrow down the drug candidates from a huge number of compounds, it is necessary to treat the behavior of a polyatomic system in atomic level and to accurately evaluate interactions between molecules and, further, between atoms.
Figure 1 is a schematic view illustrating a binding process of a protein and a binding candidate molecule in the case where dynamic behavior of the protein is considered. The protein 2 has a pocket 4 for binding a binding candidate molecule 6. That is, the binding candidate molecule 6 can be said to be a physiologically active substance of the protein 2. Note that, however, the pocket 4 does not necessarily have a shape that allows the binding candidate molecule 6 to be directly fitted therein (a of Figure 1) . For example, Figure 1 illustrates that the size of the opening of the pocket 4 is smaller than that of the binding candidate molecule 6. Consequently, in such a case, the protein 2 changes its structure in order to adapt the shape and size of the opening to those of the binding candidate molecule 6 according to the interaction with the approaching binding candidate molecule 6(b and c of Figure 1).
The phenomenon that a biological macromolecule, such as a protein, nucleic acid, or the like, changes its structure as a result of an interaction with a physiologically active substance in the manner described above is referred to as the "induced-fit". In order to accurately calculate the interaction between molecules, the "induced-fit" should naturally be taken into account. This can be realized by treating the polyatomic system in atomic level.
Figure 2 is a schematic view illustrating a reaction path on a conceptual potential surface of a polyatomic system in which induced-fit is taken into account. The horizontal axis $X_1$ of Figure 2 conceptually illustrates a structural change in the protein and the vertical axis $X_2$ illustrates a distance between a protein and a binding candidate molecule. That is, Figure 2 represents a potential surface of a polyatomic system according to the structural change in the protein and the distance between molecules. As illustrated in Figure 2, the system in which the induced-fit occurs has an energy barrier B (saddle point) on a reaction path RP connecting two stable points (point A at which the composite body is in dissociated bodies and point B at which the binding of the composite body is completed).

Thus, to analyze the binding process of the protein 2 and the binding candidate molecule 6 by taking into account the induced-fit is, in effect, searching for the reaction path connecting the two stable points A and C over the energy barrier B on the potential surface with respect to the protein 2 and the binding candidate molecule 6.

In view of the above, in order to achieve improved calculation accuracy and reduced calculation time in a simulation for predicting dynamic behavior of a polyatomic system, it is necessary to make it possible to treat the polyatomic system in atomic level and to make it easy to search for an ab initio reaction path.

The behavior of a polyatomic system having several thousands to several millions of atoms, however, is a slow and large deformation that occurs in a time scale on the order from one second to one hour. This has caused a problem on the conventional simulation methods that the theoretical calculation requires a huge amount of time of several decades, although they may treat the polyatomic system in atomic level. Consequently, in order to make it possible to perform theoretical calculation for the behavior of a polyatomic system within a practical time period, various simulation methods have been studied or developed. The molecular dynamics method in which the "compulsive force" is applied is one of such simulation methods, but it has the aforementioned problem. Another method that reduces the amount of calculations by approximating the target protein as a rigid body and reducing the number of variables used is also being studied. But such coarse approximation naturally cannot take into account the influence of the dynamic behavior of the protein so that interactions acting between molecules cannot be calculated correctly. In such a case, for example, one million candidate compounds may only be narrowed down to about ten thousand compounds as drug candidates. The problem of the trade-off relationship between calculation time and calculation accuracy still remains also in other simulation methods.

The present inventor has come up with an idea of modeling a polyatomic system that includes a biological macromolecule into a mass point system having N mass points, then treating behavior of the mass point system as collective motion, and extracting a collective coordinate having a smaller degree of freedom than that of mass point coordinates (coordinates of mass points that one-dimensionally describe the structure of the mass point system) from the collective motion based on the mass point coordinates.

(Collective Coordinate)

The collective coordinate will now be described. Generally, the term "collective coordinate" refers to a coordinate element of a collective variable. The term "collective variable" refers to one of general variables (q, p) associated with canonical variables of a mass point system (coordinates of a mass points and momentum of the mass points in the mass point system) by canonical transformation having a smaller degree of freedom than the canonical variable. In a general variable (q, p) associated by the canonical transformation, q represents a coordinate element and p represents a momentum element. The coordinate element q and momentum element p are defined by Formulae 26 and 27 given below respectively.

$$q \equiv \left(q^1, \cdots, q^\eta, \cdots, q^{3N}\right)$$

Formula 26

$$p \equiv \left(p_1, \cdots, p_\eta, \cdots, p_{3N}\right)$$

Formula 27

In Formulae 26 and 27, η represents an integer in the range from 1 to 3N.

Therefore, using $q^\dagger$ and pt defined respectively by Formulae 28 and 29 given below, the collective variable may be represented as ($q^\dagger$,$p_\dagger$).

$$q^\dagger \equiv \left(q^1, \cdots, q^\mu, \cdots, q^K, 0, \cdots, 0\right) = \left(q^1, \cdots, q^K\right)$$

Formula 28

$$p_\dagger \equiv (p_1, \cdots, p_\mu, \cdots, p_K, 0, \cdots, 0) = (p_1, \cdots, p_K)$$

Formula 29

Formulae 28 and 29 indicate that the collective variable ($q^\dagger, p_\dagger$) includes 2K valuables indicated by $\mu$=1 to K in each of Formulae 28 and 29. In other words, the collective variable ($q^\dagger, p_\dagger$) can be said to be of a general coordinate constituted by a variable component that varies with time ($q^1$, $q^2$, -----, $q^K$, $p_1$, $p_2$, -----, $p_K$) and an invariable component which serves as a constant with respect to time ($q^{K+1}$=0, $q^{K+2}$=0, -----, $q^{3N}$=0, $p_{K+1}$=0, $p_{K+2}$=0, -----, $p_{3N}$=0), the variable component. Although Formulae 28 and 29 indicate that the invariable component takes a value of zero as constant, but the constant is not limited to zero.

Then, as the collective coordinate is the coordinate element of the collective variable ($q^\dagger, p_t$), it may be represented as $q^\dagger$. More specifically, the collective coordinate is a set of variables constituted by ($q^1$, $q^2$, -----, $q^K$). Note that the collective coordinate may be obtained only from mass point coordinates by canonical transformation.

The variable transformation from coordinates to be treated as the collective motion to collective coordinates having a smaller degree of freedom in the manner described above is also referred to as the "variable separation".

There is not any restriction on the degree of freedom of the collective coordinate as long as it is smaller than that of the target coordinates of variable separation. But, as a smaller degree of freedom makes the following calculation operations easier, the degree of freedom of the collective coordinate is preferable to be 1 (that is, K=1).

(Treatment as Collective Motion)

Performance of variable separation on mass point coordinates allows intrinsic motion, that is, lower dimensional collective motion in the behavior of the mass point system to be described by the collective coordinate $q^\dagger$. As a result, the description of motion by the 3N dimensional mass point coordinates is replaced with the description of motion by the K dimensional collective coordinate $q^\dagger$, whereby the description of motion of the mass point system is simplified. More specifically, the motion equation described by the mass point coordinates is simplified to a combination of a structure x=x ($q^\dagger$) of a mass point coordinate x having a collective coordinate $q^\dagger$ as an argument and a motion equation with respect to $q^\dagger$. Note that x represents ($x_1$, $x_2$, -----, $x_{3N}$). The specific structure of the mass point coordinates represents the way of behavior as the collective motion of the mass point system and the motion equation with respect to the collective coordinate $q^\dagger$ represents the basic law of the behavior. Thus, as the degree of freedom of treated variables for solving the motion equation is further reduced, theoretical calculations become easier.

Then, variable separation is performed on the mass point coordinate x to find out a reaction path on the potential surface with respect to the collective coordinate $q^\dagger$ and the collective coordinate $q^\dagger$ is inversely transformed into the mass point coordinate x to represent the reaction path on the potential surface with respect to the mass point coordinate x. In this way, the reaction path of the mass point system may be obtained. According to the aforementioned method, only a variable transformation is performed from the collective coordinate $q^\dagger$ to the mass point coordinate x after the lower dimensional motion equation is solved. Therefore, the mass point system will not inherently be trapped by a local minimum.

For example, a conceptual process for obtaining a reaction path of a polyatomic system that includes the composite body shown in Figure 2 is as follows. Figure 3 is a drawing conceptually illustrating a process for obtaining a reaction path of a polyatomic system using extraction of a collective coordinate. First, the potential surface with respect to the mass point coordinates $X_1$, $X_2$ (a in Figure 3) is transformed into the potential surface of the extracted collective coordinates $q^1$, $q^2$ (b in Figure 3). Here, as the collective coordinates, it is important to employ collective coordinates in which two stable points A, C and energy barrier B are aligned on a straight line. The reaction path RP connecting two stable points A, C may be drawn at once (b in Figure 3) on the potential surface with respect to such collective coordinates $q^1$, $q^2$. Thereafter, the coordinates are inversely transformed and the reaction path RP on the potential surface with respect to variables $X_1$, $X_2$ is obtained (c in Figure 3) . The one-dimensional trajectory obtained here may be variables identified as the "reaction coordinate" which has long been used in the theoretical chemistry.

(Problem in Treating Behavior of Polyatomic System that Includes Biological Macromolecule as Collective Motion)

The variable separation is carried out based on the collective motion theory. As one of the collective motion theories, for example, a self-consistent collective coordinate (SCC) method may be cited. The SCC method is one of the collective motion theories in physics which is being studied in the field of atomic nucleus. More specifically, the SCC method is a motion theory that obtains an inherent collective variables describing collective motion of a system, then finds a discrete manifold from a 6N dimensional phase space spanned by canonical variables included in the Hamiltonian of the system, and describes the collective motion by the Hamiltonian defined on the discrete manifold or adjacent thereof. The term "discrete manifold" as used herein refers to a partial space of 6N dimensional phase space spanned by canonical variables included in the Hamiltonian of the system in which the trajectory representing the behavior of the system is confined. That is, in the case where any one arbitrary point in the discrete manifold is taken as the initial value, the trajectory is always confined in the discrete manifold. For more about the SCC method, refer to S. Tomonaga, "Elementary Theory of Quantum-Mechanical Collective Motion of Particles, II", Progress of Theoretical Physics, Vol. 13, No. 5, pp. 482-496, 1955, T. Marumori et al., "Self-Consistent Collective-Coordinate Method for the Large-Amplitude Nuclear Collective Motion", Progress of Theoretical Physics, Vol. 64, No. 4, pp. 1294-1314, 1980, and G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000, and the like.

Consequently, it might seem that replacement of a nucleon (proton and neutron) treated in nucleus reaction with an atom treated in chemical reaction and application of the SCC method, a dynamic behavior of a polyatomic system may be treated as collective motion and the reaction path of the polyatomic system may be obtained.

The present inventor has newly found out that direct application of a conventional collective motion theory, such as the SCC method, to a large scale system like the system that includes a biological macromolecule, which is the subject matter of the present invention, is inadequate in order to improve calculation accuracy and reduce calculation time. This is due to the fact that while the nucleus reaction needs to treat only several hundred nucleons at most, the chemical reaction of a polyatomic system that includes a biological macromolecule in water needs to treat several thousands to several million atoms. That is, in the existing method, though it is a collective motion theory, control of variables, i.e., the variable separation becomes complicated.

The aforementioned problem is not limited to the case in which a reaction path of a polyatomic system that includes a composite body is obtained. That is, in more general sense, it is easy to imagine that the same problem occurs in the case where a reaction path of a system that includes a biological macromolecule is obtained.

[Description of the Invention]

Through the study described above, the present inventor has invented a simulation apparatus and simulation method for predicting dynamic behavior of a mass point system based on a novel collective motion theory that describes collective motion by collective coordinates having a smaller degree of freedom than that of the mass point coordinates and is applicable to structural calculation of a polyatomic system that includes a biological macromolecule which is a huge group of atoms, and a program and recording medium for implementing the method.

More specifically, a simulation apparatus according to the present invention is an apparatus for predicting behavior of a mass point system constituted by modeled N mass points, the apparatus including:

a coordinate setting means for setting slow coordinates which are M coordinates mainly assuming a structural change in the mass point system based on 3N mass point coordinates describing a structure of the mass point system and fast coordinates which are coordinates describing the structure of the mass point system and being independent of the slow coordinates;
a coordinate extraction means for obtaining a structure of the fast coordinates as a function of the slow coordinates by subordinating the fast coordinates to the slow coordinates and obtaining, by taking into account influence of a change in the fast coordinate on the slow coordinates due to a change in the slow coordinates, a structure of the slow coordinates as a function of K collective coordinate (s) of a general coordinate which is associated with the slow coordinates by a canonical transformation, wherein the general coordinate is constituted by a variable component that varies with time and an invariable component that serves as a constant with respect to time and the K collective coordinate (s) is the variable component of the general coordinate; and
an inverse transformation means for predicting time evolution of the mass point system based on the collective coordinate(s) as a function of time, which can be obtained as a solution of a motion equation with respect to the collective coordinate (s), the structure of the slow coordinates, and the structure of the fast coordinates.

A simulation method of the present invention is a method for use with the aforementioned simulation apparatus for predicting behavior of a mass point system constituted by modeled N mass points performed, the method including the steps of:

setting slow coordinates which are M coordinates mainly assuming a structural change in the mass point system based on 3N mass point coordinates describing a structure of the mass point system;
setting fast coordinates which are coordinates describing the structure of the mass point system and being independent of the slow coordinates;
obtaining a structure of the fast coordinates as a function of the slow coordinates by subordinating the fast coordinates to the slow coordinates;
obtaining, by taking into account influence of a change in the fast coordinates on the slow coordinates due to a change in the slow coordinates, a structure of the slow coordinates as a function of K collective coordinate(s) of a general coordinate which is associated with the slow coordinates by a canonical transformation, wherein the general coordinate is constituted by a variable component that varies with time and an invariable component that serves as a constant with respect to time and the K collective coordinate (s) is the variable component of the general coordinate; and

predicting time evolution of the mass point system based on the collective coordinate(s) as a function of time, which can be obtained as a solution of a motion equation with respect to the collective coordinate(s), the structure of the slow coordinates, and the structure of the fast coordinates.

A simulation program according to the present invention is a program for causing a computer to perform the simulation method described above.

A computer readable recording medium according to the present invention is a medium on which is recorded the simulation program described above.

The term "a mass point system having N mass points" as used herein refers to that the total number of mass points, which are constituent elements of the mass point system, is N.

<Coordinate Setting Means>

The coordinate setting means is a means for setting slow coordinates which are M coordinates mainly assuming a structural change in a mass point system based on 3N mass point coordinates describing a structure of the mass point system and fast coordinates which are coordinates describing the structure of the mass point system and being independent of the slow coordinates. The term "3N mass point coordinates" as used herein specifically refers to coordinates of N mass points in a three-dimensional space.

First, the coordinate setting means sets M slow coordinates that primarily assume a structural change in the mass point system in describing macroscopic collective motion (large amplitude collective motion) of the mass point system based on a structure of the mass point coordinates. Then, the coordinate extraction means performs variable separation on the slow coordinates. In other words, in the present invention, independent coordinates of those describing a mass point system having little influence on a structural change in the mass point system due to the large amplitude collective motion of the mass point coordinates are excluded, as they are known, in advance from the target of the variable separation in the collective motion theory. That is, calculation for the variable separation is simplified by extracting the collective coordinates from the M dimensional slow coordinates instead of extracting from the 3N dimensional mass point coordinates. Here, M is an integer that satisfies $K<M<3N$. K represents the number of elements of the collective coordinate $q^{\dagger}$ as in Formulae 28 and 29.

Note that further hierarchization may be performed from the slow coordinates. That is, based on the structure of the slow coordinates set in the manner described above, secondary slow coordinates which are lower in dimension may be set and a collective coordinate may be extracted from the secondary slow coordinates.

The slow coordinates are set based on the degree of influence of the large amplitude collective motion on the structural change in the mass point system. Coordinates having large influence are more suitable as the slow coordinates. In other words, the setting method of slow coordinates and specific contents thereof depend on what type of large amplitude collective motion of a mass point system is targeted.

More specifically, the slow coordinates are set using some of the mass point coordinates, coordinates which can be defined by combining mass point coordinates, or a combination thereof. Preferably, the slow coordinates are set, for example, with respect to a characteristic partial structure of the structure of the mass point system. In this case, it is more preferable that each of the slow coordinates is a representative coordinate extracted from each characteristic partial structure and represents the partial structure. A plurality of representative coordinates may be set to one characteristic partial structure.

The term "characteristic partial structure" as used herein refers to a part of the structure of the mass point system having morphological and/or functional characteristics. In the case where the mass point system is a polyatomic system that includes a biological macromolecule, the characteristic partial structure is, for example, a secondary structure (partial folding structure) of the biological macromolecule, building block of the biological macromolecule, and main chain of the biological macromolecule. The term "representative coordinate" of the characteristic partial structure as used herein refers to a coordinate representatively indicates the characteristic partial structure. The representative coordinate is, for example, the coordinate itself of a mass point (atom) constituting the characteristic partial structure, a coordinate that can be defined by combining coordinates of the mass points (atoms), a cyclic interval of the partial structures, or the like.

More specifically, in the case where the biological macromolecule is a protein, a secondary structure of the protein may be cited as the characteristic partial structure. Specific secondary structures include helix structures ($3_{10}$ helix, $\alpha$ helix, $\pi$ helix, $\beta$ helix, and the like), $\beta$ sheet, turn, loop, random coil, and the like. As for the representative coordinate of the secondary structure, a coordinate of the center of gravity of a group of atoms constituting the secondary structure or a flexion angle of the secondary structure may be cited. For example, the number of characteristic partial structures (higher dimensional structures) included in a general protein is several to a few dozen at most. This may largely reduce the number of target variables for variable separation. In the case where behavior of a binding reaction between a biological macromolecule and a binding candidate molecule is predicted, the structure itself of the binding candidate may be treated as one of the characteristic partial structures of the polyatomic system.

Otherwise, in the case where the biological macromolecule is a protein, the characteristic partial structure may be a residue of the protein (amino acid portion which is the building block of the protein, including n-terminal residue and c-terminal residue) . Then, as for the representative coordinate with respect to the residue of the protein, the coordinate of the center of gravity of the group of atoms constituting the residue may be cited.

Further, in the case where the biological macromolecule is a protein, the characteristic partial structure may be the main chain of the protein. Then, as for the representative coordinate with respect to the main chain of the protein, coordinates of the respective atoms constituting the main chain may be cited.

Otherwise, in the case where the biological macromolecule is a nucleic acid, the characteristic partial structure may be a secondary structure of the nucleic acid. A specific secondary structure may be a helical structure or the like. In the case of helical structure, each predetermined pitch is treated as the characteristic partial structure. Then, as for the representative coordinate with respect to the secondary structure of the nucleic acid, a coordinate of the center of gravity of the group of atoms constituting the secondary structure or a flexion angle of the secondary structure may be cited.

Otherwise, in the case where the biological macromolecule is a nucleic acid, the characteristic partial structure may be a residue of the nucleic acid (nucleotide portion which is the building block of the nucleic acid). Then, as for the representative coordinate with respect to the residue of the nucleic acid, the coordinate of the center of gravity of the group of atoms constituting the residue may be cited.

Further, in the case where the biological macromolecule is a nucleic acid, the characteristic partial structure may be the main chain of the nucleic acid. Then, as for the representative coordinate with respect to the main chain of the nucleic acid, coordinates of the respective atoms constituting the main chain may be cited.

Otherwise, in the case where the biological macromolecule is a nucleic acid and a helical structure of the nucleic acid, the pitch interval of the helical structure may be used as the representative coordinate.

A determination on how to actually set the characteristic partial structure is made appropriately according to the structure and/or phenomenon of the simulation target mass point system. For example, in the case where a formation process of the folding structure of a protein or a formation process of the helical structure of a nucleic acid is the analysis target, it is preferable to use a main chain that can be said to be a building block of the biological macromolecule or a fundamental skeleton of the biological macromolecule as a characteristic partial structure. With respect to the main chain of the biological macromolecule, the entirety of one main chain linked by covalent binding may be used as one characteristic partial structure or each partial structure obtained by dividing the one main chain may be used as a characteristic partial structure. In the case where behavior of binding reaction between a protein and a binding candidate molecule is the analysis target, each of the helix structure, β sheet, turn, loop, random coil, and the like or a combination thereof may be used as a characteristic partial structure, other than the main chain described above. The characteristic partial structure may include a plurality of secondary structures. That is, each of the secondary structures may be treated as one characteristic partial structure or a plurality of adjacent secondary structures along a main chain may be grouped as one characteristic partial structure. Further, in the case where the reaction in which an inhibitor is caught and bound to a helical structure of a nucleic acid is the analysis target, the main chain described above, the periodic structure of the helical structure, or a combination thereof may also be used. With respect to the helical structure, the entirety may be used as one characteristic partial structure or each partial structure obtained by dividing the helical structure by a predetermined pitch may be used as a characteristic partial structure.

The "fast coordinates" are coordinates describing the structure of the mass point system and being independent of the slow coordinates. As in the slow coordinates, the fast coordinates are set using some of the mass point coordinates themselves included in the mass point system, coordinates that can be defined by combining the mass point coordinates, or a combination thereof. Depending on the combination of mass point coordinates, a portion of the formation of the fast coordinates may be represented by some slow coordinates. As the fast coordinates are those independent of the slow coordinates, however, the formation of the fast coordinates is never represented by only some slow coordinates. In the case where coordinates of some of the mass points are set as slow coordinates, the fast coordinates are coordinates of N mass points minus the mass points set as the slow coordinates. In the case where only coordinates that can be defined by combining coordinates of mass points are set as the slow coordinates, the fast coordinates are coordinates that can be defined by combining all or some of the coordinates of mass points and independent of the slow coordinates.

Two examples of specific methods for setting slow coordinates will be described.

One example method is a method in which N atoms included in a polyatomic system are grouped with respect to each characteristic partial structure to extract the center of gravity for each group and only the coordinates of these centers of gravity are set as slow coordinates. In this case, it is preferable that each fast coordinate is a relative coordinate with respect to the coordinates of the center of gravity of the partial structure to which the atoms belong and the fast coordinates are independent from each other. Thus, the number of fast coordinates is 3N-M. This is due to the fact that, when 3N mass point coordinates are separated into M coordinates of the centers of gravity and 3N relative coordinates, M relational expressions occur between the relative coordinates by definition, i.e., M relative coordinates of all relative coordinates are not independent coordinates. This method considers that the positional relationship between characteristic partial structures has significant influence on the structural change in the polyatomic system due to large amplitude collective motion. The deformation scale of a characteristic partial structure itself is small and the deformation time scale is shorter than that of the chemical reaction, so that the influence of the deformation on the structural change in the polyatomic system is small.

The other example method is a method in which each coordinate of atoms constituting a main chain of a biological macromolecule is set as a slow coordinate. In this case, it is preferable that the coordinates of other atoms, such as a side chain and a solvation, are set as fast coordinates. Thus, the number of fast coordinates is 3N-M. This method considers that the shape of the polyatomic system has significant influence on the structural change in the polyatomic system due to large amplitude collective motion. As the side chain moves dependently with the main chain, the influence of the deformation of the side chain on the structural change in the polyatomic system is small. In this case, if a molecule other than a biological macromolecule, such as a binding candidate molecule or the like, is included in the polyatomic system, the gravity point of the molecule may be set as a slow coordinate. In this case, for example, the relative coordinates of the atoms constituting the molecule to the gravity point of the molecule are set as fast coordinates.

Note that the first embodiment to be described later uses the former setting method.

Preferably, slow coordinates are set after total structural relaxation is performed under the state in which all mass points are freed (state not being fixed to the mass point system) as required. The term "total structural relaxation" as used herein refers to that the coordinates of mass points are moved such that the potential energy of the mass point system is consistently reduced from an appropriate starting state until the gradient of the potential energy becomes zero. The term "total structural relaxation" is synonymous with a generally so-called structural relaxation and this term is used in order to make distinction from partial structural relaxation, to be described later. The state of the mass point system found out by the total structural relaxation is one of the local minimums near the starting state described above. That is, the total structural relaxation is not the operation to find out a reaction path connecting between stable points and a minimum point by repeating ups and downs on the potential surface. In particular, the total structural relaxation is distinguished from the "partial structural relaxation" in that it moves all atoms constituting the structural relaxation target. The total structural relaxation is performed using a known method, such as conjugate gradient method, steepest descent method, inverse Hessian method, and the like.

<Coordinate Extraction Means>

The coordinate extraction means is a means for obtaining a structure of the fast coordinates as a function of the slow coordinates and a structure of the slow coordinates as a function of collective coordinates. The term "a structure of the fast coordinates as a function of the slow coordinates" as used herein refers to a specific content of a function $R_F(R_S)$ with respect to the fast coordinates $R_F$ represented by the slow coordinates $R_S$, and the term "a structure of the slow coordinates as a function of collective coordinates" as used herein refers to a specific content of function $R_S(q^\dagger)$ with respect to the slow coordinates $R_S$ represented by the collective coordinate $q^\dagger$.

(Structure of Fast Coordinates as Function of Slow Coordinates)

The structure $R_F(R_S)$ of the fast coordinates may be obtained by subordinating the fast coordinates $R_F$ to the slow coordinates $R_S$, i.e., by giving a conditional expression that defines the relationship between the fast coordinates $R_F$ and slow coordinates $R_S$. There is not any specific restriction on the conditional expression and, for example, a conditional expression of Formula 30 given below obtained from the adiabatic approximation condition may be used. The term "adiabatic approximation" as used herein refers to the approximation in which fast coordinates $R_F$ are assumed to be able to instantaneously follow a change in the slow coordinates $R_S$ (refer to M. Born, and J.R. Oppenheimer, "On the Quantum Theory of Molecules", Ann. Physik, Vol. 84, pp. 457-484, 1927 and H. Haken, "Nonequilibrium Phase Transitions and Self-Organization in Physics, Chemistry, and Biology", Synergetics 2nd Edition, Springer-Verlag, 1978) . Formula 30 represents a condition that, for a given $R_S$, the $R_F$ is at a local stable point of the potential energy V, i.e., at a point where the gradient of the potential energy V with respect to $R_F$ is zero.

$$\frac{\partial}{\partial R_{F_\alpha}} V(R_S, R_F) = 0 \qquad for \quad \alpha = 1, \cdots, 3N - M$$

Formula 30

In Formula 30, $V(R_S, R_F)$ represents potential energy of a mass point system represented by the slow coordinates $R_S$ and fast coordinates $R_F$. The $V(R_S,R_F)$ may be obtained by substituting the structure $x(R_S,R_F)$ of the mass point coordinate x determined when the slow coordinates $R_S$ are set in the potential energy V (x) represented by the mass point coordinate x. For example, the structure $x(R_S,R_F)$ of the mass point coordinate x may be obtained by obtaining, based on the structure $R_S(x)$ of the slow coordinates $R_S$ and the structure $R_F(x)$ of the fast coordinates $R_F$ determined when the slow coordinates $R_S$ are set, inverse functions of these.

Note that it is necessary to perform partial differentiation on $V(R_S,R_F)$ with respect to all fast coordinates $R_{F1}$ to $RF_{(3N-M)}$ in order to subordinate all fast coordinates $R_{F1}$ to $RF_{(3N-M)}$ to the slow coordinates $R_S$.

In a mass point system in which the fast coordinates $R_F$ are subordinated to the slow coordinates $R_S$, the fast coordinates $R_F$ are represented as the slow coordinates $R_S$ as in Formula 31 given below, and the potential energy V of the mass point system is represented as Formula 32 given below. Hereinafter, potential energy $V_{eff}$ of the mass point system represented as a function of only the slow coordinates $R_S$ under the condition that the fast coordinates $R_F$ is subordinated to the slow coordinates $R_S$ is referred to also as the effective potential energy.

$$R_F = R_F(R_S)$$

Formula 31

$$V_{eff}(R_S) \equiv V(R_S, R_F = (R_S))$$

Formula 32

In the case where the fast coordinates $R_F$ are subordinated to the slow coordinates $R_S$ as in Formula 31, the mass point coordinate x may be represented as a function of the slow coordinates as in Formula 33 given below.

$$x = x(R_S, R_F = R_F(R_S))$$

Formula 33

(Structure of Slow Coordinates as Function of Collective Coordinates)

The structure $R_S(q^\dagger)$ of the slow coordinates may be obtained by solving the basic equation of the collective motion theory with respect to the slow coordinates $R_S$ under the condition in which the fast coordinates $R_F$ are subordinated to the slow coordinates $R_S$ taking into account the influence of a change in the fast coordinates $R_F$ on the slow coordinates $R_S$ due to a change in the slow coordinates $R_S$. The important points in obtaining the structure $R_S(q^\dagger)$ are "solving the basic equation of the collective motion theory with respect to the slow coordinates $R_S$" and "taking into account influence (feedback)of a change in the fast coordinates $R_F$ on the slow coordinates $R_S$ due to a change in the slow coordinates Rs" when solving the basic equation.

Hereinafter, the two important points described above will be described.

The reason why the basic equation of the collective motion theory is solved with respect to the slow coordinates $R_S$ is to simplify the calculation of the variable separation by extracting the collective coordinate $q^\dagger$ from the M dimensional slow coordinates $R_S$ instead of extracting the collective coordinate $q^\dagger$ from the 3N dimensional mass point coordinate x as described above.

The most fundamental basic equation in the SCC method is represented by Formulae 34 to 36 given blow (G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000).

$$\frac{\mathrm{d}R_{S_i}}{\mathrm{d}q^{\mu}} = m_i^{-1/2}\varphi_i^{\mu}(R_S) \qquad \mathit{for} \quad i = 1,\cdots,M \quad \mu = 1,\cdots,K$$

Formula 34

$$\sum_{j=1}^{M}(V_{,ij}(Rs)-\lambda^{\mu}(Rs)\delta_{ij})\varphi_{j}^{\mu}(Rs)=0 \qquad for \quad i=1,\cdots,M \quad \mu=1,\cdots,K$$

Formula 35

..

$$V_{,i}(Rs)=\sum_{\mu=1}^{K}\rho^{\mu}(Rs)\varphi_{i}^{\mu}(Rs) \qquad for \quad i=1,\cdots,M$$

Formula 36

where, $m_i$ represents a mass of $i^{th}$ slow coordinate in the mass point system, Note that, if $i^{th}$ slow coordinate is the coordinate of the gravity of a plurality of mass points, $m_i$ is the total mass of these mass points. In the case where the slow coordinate is a coordinate of combined mass point coordinates and not a gravity coordinate, $m_i$ is not the simple total mass thereof. In such a case, general momentum corresponding to the slow coordinate is obtained by a formula of the general analytical dynamics, then a Hamiltonian is written, and the mass of the slow coordinate is obtained as a coefficient of the term involving the general momentum of the Hamiltonian. $\varphi_i^{\mu}(R_S)$, $\lambda^{\mu}(R_S)$, and $\rho^{\mu}(R_S)$ are functions that satisfy Formulae 35 and 36, in which $\varphi_i^{\mu}(R_S)$ is $(i,\mu)^{th}$ component while $\lambda^{\mu}(R_S)$ and $\rho^{\mu}(R_S)$ are $\mu^{th}$ components. $V_{,i}(R_S)$ and $V_{,ij}(R_S)$ are defined respectively by Formulae 37 and 38 given below.

$$V_{,i}(Rs)\equiv(m_i)^{-1/2}\frac{\partial}{\partial Rs_i}V_{eff}(Rs)$$

Formula 37

$$V_{,ij}(Rs)\equiv(m_i m_j)^{-1/2}\frac{\partial^2}{\partial Rs_i\partial Rs_j}V_{eff}(Rs)$$

Formula 38

Generally, therefore, the function $R_{Si}(q^{\mu})$ may be obtained by forming a K dimensional hyperplane (hereinafter, simply referred to as "plane") parameterized by K collective coordinates ($q^1$, $q^2$, -----, $q^K$) in a M dimensional superspace spanned by M coordinates ($R_{S1}$, $R_{S2}$, -----, $R_{SM}$) according to Formulae 34 to 36.

As for the basic equation, the basic equation for approximate calculation in G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000 may also be used. Here, for example, in the case where the structure $R_S(q^1)$ of slow coordinates is obtained as a function of the collective coordinate $q^{\dagger}$ with a degree of freedom of 1 (K=1), the basic equation in the collective motion theory using the slow coordinates $R_S$ may be represented as Formulae 39 to 40 given blow.

$$\frac{dRs_i}{dq^1}=m_i^{-1/2}\varphi_i(Rs) \qquad for \quad i=1,\cdots,M$$

Formula 39

$$\sum_{j=1}^{M}\left(m_i^{-1/2}m_j^{-1/2}\frac{\partial^2}{\partial Rs_i\partial Rs_j}V_{eff}(Rs)-\Lambda(Rs)\delta_{ij}\right)\varphi_j(Rs)=0 \qquad for \quad i=1,\cdots,M$$

Formula 40

where, $\varphi_i(R_S)$ represents $i^{th}$ component of a function (eignevector) that satisfies Formula 40, and $\Lambda(R_S)$ represents a function (eigenvalue) that satisfies Formula 40.

In the mean time, approximate calculation is performed in the conventional SCC method in G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000, which may not allow a variable separation high accurately. Consequently, the present inventor has derived a new basic equation based on a new approximation method that allows more accurate calculation of the variable separation with respect to the slow coordinates $R_S$ and established a new SCC method (SCC 2 method) theory based on the new basic equation.

For example, in the case where the structure $R_S(q^1)$ of slow coordinates is obtained as a function of the collective coordinate $q^\dagger$ with a degree of freedom of 1 (K=1) based on the SCC 2 method, the basic equation in the collective motion theory using the slow coordinates $R_S$ may be represented as Formulae 41 to 43 given blow.

$$\frac{\mathrm{d}Rs_i}{\mathrm{d}q^1} = m_i^{-1/2}\varphi_i(Rs,\lambda) \qquad for \quad i=1,\cdots,M$$

Formula 41

$$\frac{\mathrm{d}\lambda}{\mathrm{d}q^1} = \kappa(Rs,\lambda)$$

Formula 42

$$\sum_{j=1}^{M} m_i^{-1/2} m_j^{-1/2} \frac{\partial^2}{\partial Rs_i \partial Rs_j}\left(\frac{1}{2}\sum_{k=1}^{M}\left(m_k^{-1/2}\frac{\partial}{\partial Rs_k}V_{eff}(Rs)\right)^2 - \lambda\ V_{eff}(Rs)\right)\varphi_j(Rs,\lambda) = m_i^{-1/2}\frac{\partial}{\partial Rs_i}V_{eff}(Rs)\,\kappa(Rs,\lambda) \qquad for \quad i=1,\cdots,M$$

Formula 43

where, $\varphi_i(R_S, \lambda)$ and $\kappa(R_S, \lambda)$ are functions that satisfy Formula 43 in which $\varphi_i(R_S, \lambda)$ represents $i^{th}$ component and $\lambda$ represents an auxiliary coordinate.

As the solution of Formula 39 or 41, the structure of the slow coordinates $R_S$ represented by Formula 44 is eventually obtained.

$$Rs = Rs\left(q^\dagger\right)$$

Formula 44

Here, the basic equation has been described in the case where K=1, but the present invention is not limited to the case where K=1. More specifically, where K≥2, Formulae 39 and 40 may be extended to the basic equation of SCC method by using the Frobenius theorem as described, for example, in G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000. Also, Formulae 41 to 43 may obviously be extended to the basic equation of SCC 2 method in the same manner where K≥2.

As described above, the introduction of hierarchized variables called slow coordinates (lower dimensional variables secondarily describing the structure of the mass point system) may simplify the contraction problem from 3N dimensions to K dimensions to the contraction problem from M dimensions to K dimensions, whereby the calculation for the variable separation becomes easier.

In the present invention, however, it is necessary to perform variable separation by taking into account the feedback in order to accurately calculate the structure of a mass point system. Disregarding the feedback is synonymous to performing calculation by assuming existing mass points (or fast coordinates) to not to exist, and if the variable separation is performed without considering the influence of this, a discrepancy may occur in the calculation and the calculation result may be ruined. Consequently, the existing collective motion theory is modified based on the new theory in order to take into account the feedback. The new theory is a theory in which the differential coefficient of the coordinates of potential energy in the basic equation of collective motion theory is accurately calculated under the condition that the fast coordinates $R_F$ are subordinated to the slow coordinates $R_S$, and partial structural relaxation is performed on the fast coordinates $R_F$ each time a small update is made to the slow coordinate $R_S(q^\dagger)$ ($R_S(q^\dagger) \rightarrow R_S(q^\dagger+\Delta q^\dagger)$).

In the conventional collective motion theory, only a small update ($x(q^\dagger) \rightarrow x(q^\dagger+\Delta q^\dagger)$) of the mass point coordinate x is repeated in the direction of a predetermined eigenvector φ of the differential coefficient with respect to mass point coordinate x of the potential energy V(x).

In the present invention, the feedback equations of Formulae 45 to 47 may be applied when calculating the differential coefficient with respect to the coordinates of potential energy V.

$$\frac{\partial}{\partial Rs_i} V_{eff}(Rs) = \frac{\partial}{\partial Rs_i} V(Rs, R_F)\Big|_{R_F=R_F(Rs)}$$

Formula 45

$$\frac{\partial^2}{\partial Rs_i \partial Rs_j} V_{eff}(Rs) = \frac{\partial^2}{\partial Rs_i \partial Rs_j} V(Rs, R_F)\Big|_{R_F=R_F(Rs)} + \left( \sum_{\beta=1}^{3N-M} X_\beta{}^j(Rs) \frac{\partial^2}{\partial Rs_i \partial R_{F_\beta}} V(Rs, R_F)\Big|_{R_F=R_F(Rs)} + (i \leftrightarrow j) \right) + \sum_{\alpha=1}^{3N-M} \sum_{\beta=1}^{3N-M} X_\alpha{}^i(Rs) X_\beta{}^j(Rs) \frac{\partial^2}{\partial R_{F_\alpha} \partial R_{F_\beta}} V(Rs, R_F)\Big|_{R_F=R_F(Rs)}$$

Formula 46

$$\frac{\partial^3}{\partial Rs_i \partial Rs_j \partial Rs_k} V_{eff}(Rs) = \frac{\partial^3}{\partial Rs_i \partial Rs_j \partial Rs_k} V(Rs, R_F)\Big|_{R_F=R_F(Rs)} + \left( \sum_{\gamma=1}^{3N-M} X_\gamma{}^k(Rs) \frac{\partial^3}{\partial Rs_i \partial Rs_j \partial R_{F_\gamma}} V(Rs, R_F)\Big|_{R_F=R_F(Rs)} + (i \leftrightarrow k) + (j \leftrightarrow k) \right) + \left( \sum_{\alpha=1}^{3N-M} \sum_{\beta=1}^{3N-M} X_\alpha{}^i(Rs) X_\beta{}^j(Rs) \frac{\partial^3}{\partial R_{F_\alpha} \partial R_{F_\beta} \partial Rs_k} V(Rs, R_F)\Big|_{R_F=R_F(Rs)} + (i \leftrightarrow k) + (j \leftrightarrow k) \right) + \sum_{\alpha=1}^{3N-M} \sum_{\beta=1}^{3N-M} \sum_{\gamma=1}^{3N-M} X_\alpha{}^i(Rs) X_\beta{}^j(Rs) X_\gamma{}^k(Rs) \frac{\partial^3}{\partial R_{F_\alpha} \partial R_{F_\beta} \partial R_{F_\gamma}} V(Rs, R_F)\Big|_{R_F=R_F(Rs)}$$

Formula 47

In Formula 46, (i↔j) in the third term represents a term derived by mutually replacing alphabets i and j in the second term.

In Formula 47, (i↔k) in the third term represents a term derived by mutually replacing alphabets i and k in the second term, (j↔k) in the fourth term represents a term derived by mutually replacing alphabets j and k in the second term, (i↔k) in the sixth term represents a term derived by mutually replacing alphabets i and k in the fifth term, and (j↔k) in the seventh term represents a term derived by mutually replacing alphabets j and k in the fifth term.

In Formulae 45 to 47, Formula 48 given below is used.

$$X_\alpha{}^i(Rs) \equiv -\sum_{\beta=1}^{3N-M} K_{\alpha\beta}^{-1}(Rs) J^{\beta i}(Rs)$$

Formula 48

where, $K_{\alpha\beta}^{-1}(R_S)$ represents an inverse matrix of $K^{\alpha\beta}(R_S)$, and $K^{\alpha\beta}(R_S)$ and $J^{\alpha i}(R_S)$ are defined by Formulae 49 and 50 given below respectively.

$$K^{\alpha\beta}(Rs) \equiv \frac{\partial^2}{\partial R_{F_\alpha} \partial R_{F_\beta}} V(Rs, R_F)\Big|_{R_F = R_F(Rs)}$$

Formula 49

$$J^{\alpha j}(Rs) \equiv \frac{\partial^2}{\partial R_{F_\alpha} \partial Rs_j} V(Rs, R_F)\Big|_{R_F = R_F(Rs)}$$

Formula 50

In each of Formulae 45 to 47, those other than the first term on the right-hand side represent the influence of a change in the fast coordinates $R_F$ on the slow coordinates $R_S$ due to a change in the slow coordinates $R_S$. Such configuration of feedback equation has not been known heretofore and found out by the present inventor for the first time. The feedback equation includes three formulae of first order, second order, and third order differential equations and only a required equation may be used according to the content of the basic equation in the collective motion theory. For example, in the case where Formulae 39 and 40 are employed as the basic equation in the collective motion theory, only the second order differential equation of Formula 46 is sufficient as the feedback equation.

Then, partial structural relaxation for the fast coordinates $R_F$ is performed by performing a small update ($R_S(q^\dagger) \to R_S(q^\dagger + \Delta q^\dagger)$) on the slow coordinates $R_S(q^\dagger)$ in the direction of eigenvector of the differential coefficient defined by the feedback equation and performing structural relaxation according to the condition in which the fast coordinates $R_F$ are subordinated to the slow coordinates $R_S$ under the updated slow coordinates ($q^\dagger + \Delta q^\dagger$). The term "under the updated slow coordinates ($q^\dagger + \Delta q^\dagger$) " as used herein refers to "under the state in which the updated slow coordinates ($q\dagger + \Delta q^\dagger$) are fixed in the mass point system". Further, the term "performing structural relaxation according to the condition that the fast coordinates $R_F$ are subordinated to the slow coordinates Rs" as used herein refers to moving the fast coordinates $R_F$ such that the potential energy of the mass point system is consistently reduced until the gradient of the potential energy becomes zero while keeping the state in which the fast coordinates $R_F$ are subordinated to the slow coordinates $R_S$. The structural relaxation performed with the slow coordinates $R_S$ being fixed to the mass point system is referred to as the "partial structural relaxation". As described above, the partial structural relaxation differs from the total structural relaxation in that it is performed with the slow coordinates $R_S$ being fixed to the mass point system. But, as for the method of performing the partial structural relaxation, an existing method, such as the conjugate gradient method, steepest descent method, or inverse Hessian method, may be used, as in the total structural relaxation. Preferably, the eigenvector $\varphi$ that determines the update direction corresponds to that of the minimal eigenvalue. The reason is that the subject matter of the present invention is large amplitude collective motion.

In the case where K=1, the starting state of the fast coordinates $R_F$ in the partial structural relaxation may be $R_{F\alpha}(R_S(q^1))$, but it is preferable to be the state in which fast coordinates $R_F$ are changed by a small amount given by Formula 51 given below, i.e., the state $R_{F\alpha}{}^\dagger$ represented by Formula 52 given below.

$$\frac{\mathrm{d}R_{F_\alpha}}{\mathrm{d}q^1} = \sum_{i=1}^{M} X_\alpha{}^i(Rs) \frac{\mathrm{d}Rs_i}{\mathrm{d}q^1}$$

Formula 51

$$R_{F_\alpha}{}^\dagger = R_{F_\alpha}(Rs(q^1)) + \sum_{i=1}^{M} X_\alpha{}^i(Rs) \cdot (Rs_i(q^1 + \Delta q^1) - Rs_i(q^1))$$

Formula 52

The coordinate $R_{F\alpha}{}^\dagger$ represented by Formula 52 corresponds to the fast coordinate $R_F(R_S(q^1 + \Delta q^1))$ if the small update is infinitesimal. In the actual calculation, however, the small update is finite. By setting the starting state of the fast coordinates $R_F$ in the partial structural relaxation to $R_{F\alpha}{}^\dagger$, the safety for the partial structural relaxation may be enhanced.

(Process Performed by Coordinate Extraction Means)

A series of specific steps performed by the coordinate extraction means will now be described.

First, the coordinate extraction means performs a first step for obtaining potential energy V ($R_S$, $R_F$) represented by a function of the slow coordinates $R_S$ and fast coordinates $R_F$.

Next, the coordinate extraction means performs a second step for subordinating the fast coordinates $R_F$ to the slow coordinates $R_S$. The structure of the fast coordinates $R_F$ is obtained as a function of the slow coordinates $R_S$. Here, by way of example, it is assumed that the fast coordinates $R_F$ are subordinated to the slow coordinates $R_S$ according to the adiabatic approximation condition of Formula 30.

Next, under the state of the slow coordinates $R_S$ and fast coordinates $R_F$ set by the coordinate setting means, the coordinate extraction means performs a third step for obtaining a differential coefficient of the potential energy V with respect to the slow coordinates $R_S$ according to, for example, the feedback equation of Formula 46.

Next, under the differential coefficient of the potential energy V, the coordinate extraction means performs a fourth step for obtaining a differential coefficient of slow coordinate $R_S(q)$ with respect to the collective coordinate q according to, for example, the basic equation of the SCC method of Formulae 39 and 40 when K=1.

Next, the coordinate extraction means performs a fifth step for updating the collective coordinate q by a small amount $\Delta q$ under the differential coefficient of the slow coordinate $R_S(q)$ and obtaining the updated slow coordinate $R_S$ $(q+\Delta)$.

Next, under the updated slow coordinate $R_S(q+\Delta q)$, the coordinate extraction means performs a sixth step for performing partial structural relaxation on the fast coordinates $R_F$ subordinated to the slow coordinates $R_S$ according to the adiabatic approximation condition. In this case, if the state $R_{F\alpha}{}^{\dagger}$ represented by Formula 52 is used as the starting state of the partial structural relaxation, the differential coefficient of the fast coordinate $R_F(q)$ with respect to the collective coordinate q is obtained from the differential coefficient of the slow coordinate $R_S(q)$ obtained in the fourth step according to Formula 51 and the fast coordinates are updated by a small amount $\Delta q$ under the obtained differential coefficient.

Then, the coordinate extraction means obtains a structure of the slow coordinates $R_S$ as a function of the collective coordinate q by repeating the third to sixth steps until, for example, reaching to a next local minimum while alternately updating the slow coordinates $R_S$ and fast coordinates $R_F$. That is, in the third step from the second time on, a differential coefficient of potential energy V with respect to the slow coordinates $R_S$ will be obtained, as in the third step described above, under the slow coordinates $R_S$ and fast coordinates $R_F$ in the state after the partial structural relaxation of the fast coordinates $R_F$ performed in the immediately preceding sixth step. There is not any specific restriction on the method of determining the local minimum and, for example, a method that makes a determination as to whether or not the differential coefficient of potential energy V becomes equivalent to zero may be cited as an example.

<Inverse Transformation Means>

The inverse transformation means predicts time evolution of the mass point coordinate x based on collective coordinate $q^{\dagger}(t)$ as a function of time t that can be obtained as the solution of the motion equation with respect to the collective coordinate $q^{\dagger}$, the structure $R_S(q)$ represented by Formula 44, and the structure $R_F(R_S)$ of the fast coordinates $R_F$ represented by Formula 31.

More specific description will be provided in the following. Figure 4 illustrates the concept of variable transformation and inverse transformation. Figure 4 illustrates, by way of example, the case where some of the mass point coordinate x are used as the slow coordinates $R_S$ and collective coordinate $q^{\dagger}=q^1$ with a degree of freedom of 1 is extracted from the slow coordinates $R_S$.

a of Figure 4 illustrates the setting of slow coordinates $R_S$ and fast coordinates $R_F$ based on the mass point coordinate x. In a of Figure 4, M mass point coordinates are set as the slow coordinates $R_S$ and the rest of 3N-M mass point coordinates are set as the fast coordinates $R_F$. As described above, the slow coordinates $R_S$ and fast coordinates $R_F$ are set by the coordinate setting means. b of Figure 4 indicates that the fast coordinates $R_F$ are given as a function of the slow coordinates $R_S$. c of Figure 4 illustrates that the slow coordinates $R_S$ are given as a function of the collective coordinate $q^1$. As described above, the structures of the fast coordinates $R_F$ and slow coordinates $R_S$ are obtained by the coordinate extraction means.

d of Figure 4 indicates that the collective coordinate $q^1$ is given as a function of time t, which can be obtained by solving the motion equation with respect to the collective coordinate $q^1$. The motion equation with respect to the collective coordinate $q^1$ may be obtained by obtaining motion equation with respect to the slow coordinates $R_S$ and fast coordinates $R_F$ by substituting the structure $x(R_S, R_F)$ determined when the slow coordinates $R_S$ are set to the motion equation with respect to the mass point coordinate x and substituting Formulae 31 and 44 to the obtained motion equation.

The use of the relationships from a to d of Figure 4 allows all mass point coordinate x to be obtained as a function of time t (e of Figure 4). That is, the inverse transformation means is a means that obtains the structure of the mass point coordinate x as a function of time t, x(t), by obtaining the collective coordinate $q^1$ as a function of time t and inversely transforming the variable transformation from the mass point coordinate x to collective coordinate $q^1$ via the slow coordinates $R_S$. As the function x(t) represents behavior of the mass point coordinate x with respect to a change in time, so that the function x(t) is the very thing corresponding the reaction path to be obtained. Thus, by observing the change in the function x(t) with time, the time evolution may be predicted.

[First Embodiment of the Present Invention]

Figure 5 is a block diagram of a simulation apparatus 10 according to the present embodiment, schematically illustrating a configuration thereof. Figures 6A, 6B are flowcharts schematically illustrating calculation steps in a simulation method according to the present embodiment. In the embodiment, a description will be made of a case in which coordinates of gravity points extracted from each characteristic partial structure of the structure of a polyatomic system constituted by N atoms, including a biological macromolecule and a binding candidate molecule for the biological macromolecule, are set as M slow coordinates and the degree of freedom of the collective coordinate is 1 (K=1).

The simulation apparatus 10 of the present embodiment is an apparatus for predicting behavior of a composite body constituted by a biological macromolecule and a binding candidate molecule. As illustrated in Figure 5, the simulation apparatus 10 includes an input means 12 for inputting data required for prescribing an analysis target polyatomic system (input data), a control means 14 for controlling each section of the apparatus, a coordinate setting means 16, a coordinate extraction means 18, an inverse transformation means 20, and display means 22 for displaying an analysis result.

The simulation method of the present embodiment is a method performed by the simulation apparatus 10, including the steps of inputting the input data by the input means 12, setting M slow coordinates $R_S$ and 3N-M fast coordinates $R_F$ by the coordinate setting means 16, obtaining a structure of the slow coordinates $R_S$ as a function of collective coordinate $q^1$ and a structure of the fast coordinates $R_F$ as a function of the slow coordinates $R_S$ by the coordinate extraction means 18, obtaining coordinates of the atoms as a function of time x(t) by the inverse transformation means 20, and displaying an analysis result on the display means 22.

The simulation program of the present embodiment is a program for causing a computer to perform the simulation method described above.

The computer readable recording medium of the present embodiment is a medium on which is recorded the simulation program described above.

<Input Means>

The input means 12 is a section from which the input data are inputted by the user. The inputted input data are outputted to the control means 14. There is not any specific restriction on the method of inputting the input data and, for example, a manual method through operation of the simulation apparatus 10 by the user, a method of reading from a predetermined recording medium, or the like may be cited as example.

<Control Means>

The control means 14 is a section that controls processing, including exchange of data with each section and the like. The input data inputted to the control means 14 from the input means 12 are outputted to the coordinate setting means 16. When the input data are outputted to the coordinate setting means 16, the calculation for behavior of the polyatomic system is started. The control means includes a storage medium, such as a memory, for recording intermediate and final calculation results generated through the exchange of data with each section.

Further, the control means 14 controls the display means 22 to display the result using a diagram or a graph based on the data of coordinates of the atoms x(t) obtained by the inverse transformation means 20.

<Coordinate Setting Means>

The coordinate setting means 16 is a means that optimizes a composite body based on the input data received from the control means 14 and sets M slow coordinates $R_S$ and 3N-M fast coordinates $R_F$. The data with respect to the obtained slow coordinates $R_S$ and fast coordinates $R_F$ are outputted to the coordinate extraction means 18 via the control means 14.

<Coordinate Extraction Means>

The coordinate extraction means 18 is a means that obtains potential energy $V(R_S, R_F)$ of the entire polyatomic system based on the data with respect to the slow coordinate $R_S$ and fast coordinates $R_F$ obtained by the coordinate setting means 16 and, based on the obtained $V(R_S, R_F)$, obtains a structure of the slow coordinates $R_S$ as a function of collective coordinate $q^1$ and a structure of the fast coordinates $R_F$ as a function of the slow coordinates $R_S$. The data with respect to the obtained structures of the slow coordinates $R_S$ and fast coordinates $R_F$ are outputted to the inverse transformation means 20 via the control means 14.

<Inverse Transformation Means>

The inverse transformation means 20 is a means that obtains coordinates of the atoms as a function of time x(t) based on the data with respect to the structures of the slow coordinates $R_S$ and fast coordinates $R_F$ obtained by the coordinate extraction means 18. The data with respect to the obtained coordinates of the atoms x(t) are outputted to the control means 14.

<Display Means>

The display means is a means that displays, for example, the potential energy V of the polyatomic system as a graph, the structure in the binding process of the composite body and/or the structure of the final state as an image, binding process of the composite body as a motion picture, and the like according to the instruction from the control means 14. There is not any specific restriction on the display method and any known method, such as 2D display, 3D display, or the like, may be used.

Hereinafter, a process of the simulation method using the apparatus of the present embodiment will be described with reference to Figures 6A and 6B.

<STEP1>

First, input data are inputted from the input means 12 to the simulation apparatus 10 (ST1 in Figure 6A). Specific contents of the input data include the type, number and coordinates x representing the initial arrangement of N atoms constituting a polyatomic system that includes a biological macromolecule and a binding candidate molecule, the rule for forming potential energy V(x) from the coordinates x, and the like.

<STEP2>

Next, the polyatomic system that includes the composite body is optimized and an initial state structure is obtained (ST2 in Figure 6A) .

More specific description will be provided in the following. First, the coordinate setting means 16 performs total structural relaxation on the biological macromolecule and binding candidate molecule independently to optimize the structures of the biological macromolecule and binding candidate molecule respectively. Then, the biological macromolecule and binding candidate molecule optimized in the structures are disposed at a distance that allows an interaction to be initiated between them (e.g., the binding candidate molecule slightly touches to the biological macromolecule). This state is the initial state of the composite body constituted by the biological macromolecule and binding candidate molecule.

<STEP3>

Next, slow coordinates $R_S$ and fast coordinates $R_F$ are set based on the initial state structure obtained in STEP 2 (ST3 in Figure 6A) .

The coordinate setting means 16 divides the structure of the biological macromolecule into (M-3) /3 characteristic partial structures while treating the entire binding candidate molecule as one partial structure, and extracts the center of gravity from each of M/3 partial structures. Then, coordinates of the M/3 gravity points are set as the slow coordinates $R_S$ and coordinates relative to each center of gravity of atoms included in each of M/3 partial structures are set as 3N-M fast coordinates $R_F$. In the present embodiment, as a fast coordinate $R_F$, a coordinate relative to the coordinate of the center of gravity of the partial structure in which the atoms are included is set.

<STEP4>

Next, potential energy $V(R_S, R_F)$ of the polyatomic system represented by the slow coordinates $R_S$ and fast coordinates $R_F$ is obtained (ST4 in Figure 6A).

Figure 7 schematically illustrates the relationship between slow coordinates $R_S$, fast coordinates $R_F$, and atom coordinates x of the present embodiment. In Figure 7, the number of atoms included in m^th (m is an integer that satisfies $1 \le m \le M/3$) characteristic partial structure is represented as $N_m$. That is, $N_1+N_2+\cdots+N_m+\cdots N_{M/3}=N$. In the present invention, M represents the number of atom coordinates (mass points) in a three-dimensional space, so that M/3 is normally an integer. Further, in the present embodiment, it is known from Figure 7 that the slow coordinates $R_S$, fast coordinates $R_F$, and atom coordinates x have relationships represented by Formulae 53 and 54 given below.

$$\mathrm{m}=1:\left(Rs_1, Rs_2, Rs_3\right)=\frac{1}{N_1}\cdot\left(\sum_{n=1}^{N_1} x_{3n-2}, \sum_{n=1}^{N_1} x_{3n-1}, \sum_{n=1}^{N_1} x_{3n}\right)$$

$$\vdots$$

$$\mathrm{m}=\mathrm{m}:$$

$$\left(Rs_{3m-2}, Rs_{3m-1}, Rs_{3m}\right)=\frac{1}{N_m}\cdot\left(\sum_{n=1}^{N_m} x_{3N_1+\cdots+3N_{m-1}+3n-2}, \sum_{n=1}^{N_m} x_{3N_1+\cdots+3N_{m-1}+3n-1}, \sum_{n=1}^{N_m} x_{3N_1+\cdots+3N_{m-1}+3n}\right)$$

$$\vdots$$

$$\mathrm{m}=\mathrm{M}/3:$$

$$\left(Rs_{M-2}, Rs_{M-1}, Rs_M\right)=\frac{1}{N_{M/3}}\cdot\left(\sum_{n=1}^{N_{M/3}} x_{3N-3N_{M/3}-3n-2}, \sum_{n=1}^{N_{M/3}} x_{3N-3N_{M/3}+3n-1}, \sum_{n=1}^{N_{M/3}} x_{3N-3N_{M/3}+3n}\right)$$

Formula 53

$$\mathrm{m}=1,\ \mathrm{n}=1:(x_1, x_2, x_3)=(Rs_1, Rs_2, Rs_3)+(R_{F1}, R_{F2}, R_{F3})$$

$$\vdots$$

$$\mathrm{m}=\mathrm{m},\ \mathrm{n}=\mathrm{n}:\begin{pmatrix} x_{3N_1+\cdots+3N_{m-1}+3n-2} \\ x_{3N_1+\cdots+3N_{m-1}+3n-1} \\ x_{3N_1+\cdots+3N_{m-1}+3n} \end{pmatrix}=\begin{pmatrix} Rs_{3m-2} \\ Rs_{3m-1} \\ Rs_{3m} \end{pmatrix}+\begin{pmatrix} R_{F3N_1+\cdots+3N_{m-1}+3n-2} \\ R_{F3N_1+\cdots+3N_{m-1}+3n-1} \\ R_{F3N_1+\cdots+3N_{m-1}+3n} \end{pmatrix}$$

$$\vdots$$

$$\mathrm{m}=\mathrm{M}/3,\ \mathrm{n}=\mathrm{N}_{M/3}:(x_{3N-2}, x_{3N-1}, x_{3N})=(Rs_{M-2}, Rs_{M-1}, Rs_M)+(R_{F3N-2}, R_{F3N-1}, R_{F3N})$$

Formula 54

Formula 53 is a formula representing the relationship between m^th slow coordinate $R_S$, i.e., the coordinate of the center of gravity of m^th characteristic partial structure, and atom coordinates x. Formula 54 is a formula representing the relationship between the coordinate x of n^th atom belonging to m^th characteristic partial structure, slow coordinates $R_S$, and fast coordinates $R_F$. In Formulae 53 and 54, n represents the serial number of atoms included in each characteristic partial structure. Thus, n with respect to m^th characteristic partial structure takes an integer in the range from 1 to $N_m$. Further, $3N_1+3N_2+\cdots 3N_{m-1}$ is zero when m=1.

Therefore, the potential energy $V(R_S,R_F)$ may be obtained by applying Formula 54 to potential energy V(x).

<STEP5>

Next, the fast coordinates $R_F$ are subordinated to the slow coordinates $R_S$ according to the adiabatic approximation condition of Formula 30 using the potential energy $V(R_S, R_F)$ (ST5 in Figure 6A) . In the present embodiment, $V(R_S,R_F)$ is partially differentiated with respect to all fast coordinates $R_{F1}$ to $R_{F3N}$.

<STEP6>

Next, under the current slow coordinates $R_S$ and fast coordinates $R_F$, second order differential coefficient of the potential energy V with respect to the slow coordinates $R_S$ is obtained according to the feedback equation of Formula 46 (ST6 in Figure 6A) .

<STEP7>

Next, under the second order differential coefficient obtain in STEP 6, a differential coefficient $dR_S/dq^1$ with respect to collective coordinate $q^1$ is obtained according to the basic equation of SCC method in Formulae 39 and 40 (ST7 in Figure 6A).

<STEP8>

Next, under the differential coefficient $dR_S/dq^1$ obtained in STEP 7, differential coefficient $dR_F/dq^1$ of the fast coordinates $R_F$ with respect to the collective coordinate $q^1$ is obtained according to Formula 51 (ST8 in Figure 6B).

<STEP9>

Next, under the differential coefficient $dR_S/dq^1$ obtained in STEP 7 and the differential coefficient $dR_F/dq^1$ obtained in STEP 8, the collective coordinate $q^1$ is updated by a small amount $\Delta q^1$ (ST9 in Figure 6B).

<STEP10>

Next, slow coordinate $R_S$ $(q^1+\Delta q^1)$ updated by $\Delta q^1$ and updated fast coordinate $R_F(q^1+\Delta q^1)$ are obtained (ST10 in Figure 6B).

For example, in the case where the initial state in STEP2 is set as the initial condition of collective coordinate $q^1$ (that is, the polyatomic system takes the initial state when $q^1=0$), the slow coordinate $R_S$ $(q^1+\Delta q^1)$ and fast coordinate $R_F(q^1+\Delta q^1)$ may be represented as Formula 55 given below.

$$Rs_i(\Delta q^1) = Rs_i(0) + \frac{\mathrm{d}Rs_i}{\mathrm{d}q^1} \cdot \Delta q^1$$

$$\begin{aligned} R_{F_\alpha}(\Delta q^1) &= R_{F_\alpha}(0) + \frac{\mathrm{d}R_{F_\alpha}}{\mathrm{d}q^1} \cdot \Delta q^1 \\ &= R_{F_\alpha}(0) + \sum_{i=1}^{M} X_\alpha{}^i(Rs)\frac{\mathrm{d}Rs_i}{\mathrm{d}q^1} \cdot \Delta q^1 \end{aligned}$$

Formula 55

In the case where STEP6 to STEP12 are repeated according to the determination result in STEP13, $R_{Si}$ $(2\Delta q^1)$, $R_{Si}$ $(3\Delta q^1)$----- which may be represented as Formula 56 are obtained one at a time in STEP10 from the second time on provided, however, that the differential coefficient $dR_S/dq^1$ does not always take the same value in each time. By repeating the operation described above, a structure $R_S(q^1)$ of the slow coordinates $R_S$ as a function of the collective coordinate $q^1$ is eventually obtained.

$$Rs_i(2\Delta q^1) = Rs_i(\Delta q^1) + \frac{\mathrm{d}Rs_i}{\mathrm{d}q^1} \cdot \Delta q^1$$

$$Rs_i(3\Delta q^1) = Rs_i(2\Delta q^1) + \frac{\mathrm{d}Rs_i}{\mathrm{d}q^1} \cdot \Delta q^1$$

$$\vdots$$

Formula 56

<STEP11>

In STEP11, under the slow coordinate $R_S(q^1+\Delta q^1)$ updated in STEP10, partial structural relaxation is performed on the fast coordinate $R_F$ with the fast coordinate $R_F(q^1+\Delta q^1)$ updated in STEP10 as the starting state (ST11 in Figure 6B).

<STEP12>

Next, in STEP12, potential energy V of the polyatomic system in a state after the partial structural relaxation is calculated (ST12 in Figure 6B). Note that, according to the determination result in STEP13, potential energy V is calculated in each time of repetition. The numerical calculation of the potential energy may be calculation based on the effective potential energy $V_{eff}$.

<STEP13>

Next, in STEP13, a determination is made as to whether or not the potential energy V calculated in STEP 12 has reached a local minimum (ST13 in Figure 6B). In view of the chemical reaction between the biological macromolecule and binding candidate molecule, the end point of the chemical reaction is the point at which the potential energy V will become a local minimum next time, i.e., a next stable point on the potential surface. Consequently, the end point of the chemical reaction is determined with reference to the value of the potential energy. If a determination is made that the potential energy V has reached a next local minimum, the process proceeds to STEP14, otherwise STEP6 to STEP12 are repeated again. Further, even in the case where the potential energy is determined to have reached a local minimum, it is also possible to continue the analysis until the potential energy V will reach the next local minimum, as required.

<STEP14>

If the potential energy V is determined to have reached a local minimum in STEP13, a structure of the polyatomic system at the potential energy V in STEP14 is the final state structure to be obtained (ST14 in Figure 6B).
Then, in addition to the final state structure, a structure $R_S(q^1)$ of the slow coordinates $R_S$ represented by Formula 44 and a structure $R_F(R_{sS})$ of the fast coordinates $R_F$ represented by Formula 31 are obtained. Thereafter, the coordinates x(t) as a function of time are obtained

<Advantageous Effects>

Figure 8 is a graph illustrating a reaction path of a composite body of a predetermined protein 2 and a physiologically active substance 6 (drug) obtained by the simulation method of the present embodiment. Figure 9 schematically illustrates the binding process of the composite body shown in Figure 8. a and b of Figure 9 illustrate an initial state and a final state of the binding process of the composite body respectively.
In Figure 8, the horizontal axis represents the distance between residues 2a, 2b of the predetermined protein 2 while the vertical axis represents the distance between the drug 6 and residue 2c. The residues 2a, 2b are residues located at the opening of the pocket 4 of the protein 2, while the residue 2c is a residue located at a position opposite to the drug 6 when the protein and drug are bound (Figure 9). The points A and C in the graph correspond to, for example, the stable points A and C of Figure 2, and the point B in the graph corresponds to, for example, the energy barrier B of Figure 2. The plot in the graph indicates equally spaced points on a time axis. The graph shows that the reaction proceeds slowly before reaching to the energy barrier B and the reaction proceeds quickly after the energy barrier B. The time required by the simulation method of the present embodiment to obtain the reaction path in a polyatomic system that includes about 2000 atoms is about 30 minutes.
The position of "X-ray analysis" in the graph shown in Figure 8 indicates a measured value actually obtained as a result of crystal structural analysis by X-ray diffraction measurement. This shows that the simulation method may calculate the binding state of the composite body with very high accuracy even though the present invention performs the simulation non-empirically.
As described above, the simulation apparatus of the present embodiment includes a coordinate setting means, a coordinate extraction means, and an inverse transformation means, and predicts time evolution of mass point coordinates by introducing hierarchized slow coordinates, extracting, by taking into account influence of a change in fast coordinates on the slow coordinates due to a change in the slow coordinates, a collective coordinate in the collective motion theory that describes collective and intrinsic behavior of a mass point system, and solving the motion equation with respect to the collective coordinate. Thus, the extraction of collective coordinate allows the simulation to be performed in atomic level and the introduction of the slow coordinates allows the number of coordinates handled for extracting the collective coordinate to be reduced. As a result, both improvement of calculation accuracy and reduction in calculation time may be achieved in a simulation for predicting dynamic behavior of a mass point system.

The simulation method of the present embodiment is a method for use with the simulation apparatus described above and predicts time evolution of mass point coordinates by introducing hierarchized slow coordinates, extracting, by taking into account influence of a change in fast coordinates on the slow coordinates due to a change in the slow coordinates, a collective coordinate in the collective motion theory that describes collective and intrinsic behavior of a mass point system, and solving a motion equation with respect to the collective coordinate. Thus, the extraction of collective coordinate allows the simulation to be performed in atomic level and the introduction of the slow coordinates allows the number of coordinates handled for extracting the collective coordinate to be reduced. As a result, both improvement of calculation accuracy and reduction in calculation time may be achieved in a simulation for predicting dynamic behavior of a mass point system.

The program and recording medium of the present embodiment may cause the aforementioned simulation method to be performed, so that improved calculation accuracy and reduced calculation time may be achieved in a simulation for predicting dynamic behavior of a mass point system.

[Second Embodiment of the Present Invention]

Next, a second embodiment will be described. The present embodiment differs from the first embodiment in that, for obtaining the solution of the basic equations (Formulae 39 and 40) employing the SCC method described in G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000, or the solution of the SCC 2 method (Formulae 41 to 43), it solves an equation equivalent to those basic equations. Therefore, components identical those of the first embodiment are not elaborated upon further here unless otherwise specifically required.

The simulation apparatus 10 of the present embodiment includes an input means 12, a control means 14, a coordinate setting means 16, a coordinate extraction means 18, an inverse transformation means 20, and display means 22 for displaying an analysis result, as in the first embodiment.

In the present embodiment, the coordinate extraction means 18 uses Formula 57 as the basic equation of SCC method in order to obtain the same solution as that of Formulae 39 and 40.

$$\frac{d\mathbf{Y}}{dq^{\mu}} = \mathbf{v}^{\mu} \qquad \textit{for} \quad \mu = 1, \cdots, K$$

Formula 57

where, Y is a MK+M+K dimensional vector defined by Formula 58 given below. That is, Y includes each component obtained by sweeping each of i and $\mu$: $m_1^{1/2}R_{S1}$, -----, $m_M^{1/2}R_{SM}$; $\varphi_1^1$, -----,$\varphi_M^1$, -----, $\varphi_1^K$, -----, $\varphi_M^K$; and $\Lambda^1$, ------,$\Lambda^K$. Note that $\varphi_i^{\mu}$ and $\Lambda^{\mu}$ represent auxiliary coordinates.

$$\mathbf{Y} \equiv \begin{pmatrix} \sqrt{m_i} Rs_i \\ \varphi_i^{\mu} \\ \Lambda^{\mu} \end{pmatrix}$$

Formula 58

where, $v^{\mu}$ is a solution vector of the inhomogeneous linear equation of Formula 59 given below. Note that $v^{\mu}$ is a MK+M+K dimensional vector.

$$C\mathbf{v}^{\mu} = \mathbf{s}^{\mu} \qquad \textit{for} \quad \mu = 1, \cdots, K$$

Formula 59

C and $s^{\mu}$ in Formula 59 are defined by Formulae 60 and 61 given below respectively. C is a square matrix of (MK+M+K)×(MK+M+K). Although C appears as a 3×3 matrix, each component further has a matrix. That is, in C represented as a 3×3 matrix, the component in the first row and first column is a MK×M matrix (that is, with MK rows and M columns), the component in the first row and second column is a MK×MK matrix, the component in the first row and third column is a MK×K matrix, component in the second row and second column is s a K×MK matrix, the component in the third row and first column is a M×M matrix, and other components are zero matrices. Elements of each component are formed by sweeping i or μ, i=1 to M or μ=1 to K, as the variable in the rows and j or v, j=1 to M or v=1 to K, as the variable in the columns. $s^{\mu}$ is a MK+M+K dimensional vector. Like C, $s^{\mu}$ also appears as a three-dimensional vector, but each component further has a vector. That is, the third component as the three-dimensional vector of $s^{\mu}$ is a M dimensional vector and other components are zero vectors. Elements of the third component are formed by sweeping i from 1 to M.

$$C \equiv \begin{pmatrix} \sum_{k=1}^{M} V_{,ijk}(Rs)\varphi_k^{\mu} & (V_{,ij}(Rs) - \Lambda^{\mu}\delta_{ij})\delta^{\mu\nu} & -\varphi_i^{\mu}\delta^{\mu\nu} \\ 0 & \varphi_j^{\mu}\delta^{\mu\nu} & 0 \\ \delta_{ij} & 0 & 0 \end{pmatrix}$$

Formula 60

$$\mathbf{s}^{\mu} \equiv \begin{pmatrix} 0 \\ 0 \\ \varphi_i^{\mu} \end{pmatrix} \qquad for \quad \mu = 1, \cdots, K$$

Formula 61

$V_{,ijk}$ ($R_S$) is defined by Formula 62 given below.

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formula 62

Formula 57 is a basic equation when K is generalized, and if K=1, Formula 57 is equivalent to Formula 63 given below. Further, as described in G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000, Formula 57 with respect to generalized K may be derived from Formula 63 where K=1 by using the Frobenius theorem.

$$\begin{aligned} \frac{\mathrm{d}Rs_i}{\mathrm{d}q^1} &= m_i^{-1/2}\chi_i \\ \frac{\mathrm{d}\varphi_i^1}{\mathrm{d}q^1} &= \psi_i \qquad for \quad i = 1, \cdots, M \\ \frac{\mathrm{d}\Lambda^1}{\mathrm{d}q^1} &= \kappa \end{aligned}$$

Formula 63

In Formula 63, ($\chi_1$, -----,$\chi_M$, $\psi_1$, -----, $\psi_M$, $\kappa$) is a 2M+1 dimensional solution vector of the inhomogeneous linear equation represented by Formula 64 given below.

$$\begin{pmatrix} V_{,ijk}(Rs)\varphi_k^1 & V_{,ij}(Rs)-\Lambda^1\delta_{ij} & -\varphi_i^1 \\ 0 & \varphi_j^1 & 0 \\ \delta_{ij} & 0 & 0 \end{pmatrix}\begin{pmatrix} \chi_j \\ \psi_j \\ \kappa \end{pmatrix} = \begin{pmatrix} 0 \\ 0 \\ \varphi_i^1 \end{pmatrix} \qquad for \quad i=1,\cdots,M$$

Formula 64

In Formula 64, the product of the matrix and eigenvector is taken and when the Formula 60 is represented by three equations, the sum of 1 to M is taken with respect to j and k.

Equivalence of Formulae 63 and 64 to Formulae 39 and 40 may be proved in the following manner. If Formula 64 is integrated by $q^1$ with respect to ($\varphi_i^1$, $\Lambda^1$) by paying attention to Formulae 38, 62, and 63, Formula 65 given below may be obtained.

$$\sum_{j=1}^{M}\left( m_i^{-1/2} m_j^{-1/2} \frac{\partial^2}{\partial Rs_i \partial Rs_j} V - \Lambda^1 \delta_{ij} \right) \varphi_i^1 = 0 \qquad for \quad i=1,\cdots,M$$

$$\sum_{i=1}^{M} \varphi_i \varphi_i = \text{const.}$$

Formula 65

Further, if the identity $\chi_i=\varphi_i^1$ included in Formula 64 is substituted to the first equation of Formula 63, then Formula 66 given below may be obtained.

$$\frac{\mathrm{d}Rs_i}{\mathrm{d}q^1} = m_i^{-1/2}\varphi_i^1 \qquad for \quad i=1,\cdots,M$$

Formula 66

Here, by replacing $\varphi_i^1$ and $\Lambda^1$ in the first equation of Formula 65 and in Formula 66 with $\varphi_i$ ($R_S$) and $\Lambda(R_S)$ (i.e., when $\varphi_i^1$ and $\Lambda^1$ are treated as functions of $R_S$), Formulae 39 and 40 are reproduced.

In the case where the same solutions as those of Formulae 41 to 43 are obtained, the coordinate extraction means 18 uses Formula 67 as the basic equation of SCC 2 method.

$$\frac{\mathrm{d}\mathbf{Z}}{\mathrm{d}q^{\mu}} = \sum_{v=1}^{K} c^{\mu v}\mathbf{w}^{v} \qquad for \quad \mu=1,\cdots,K$$

Formula 67

where, Z is a MK+M+2K dimensional vector defined by Formula 68 given below. That is, Z includes each component obtained by sweeping each of i and $\mu$: $m_1^{1/2}R_{S1}$, -----, $m_M^{1/2}R_{SM}$; $\varphi_1^1$, -----,$\varphi_M^1$, -----, $\varphi_1^K$, -----,$\varphi_M^K$; $\lambda^1$, -----, $\lambda^K$; and $\rho^1$, -----, $\rho^K$. Note that$\lambda^\mu$ and $\rho^\mu$ represent auxiliary coordinates like $\varphi_i^\mu$. $c^{\mu v}$ is a constant uniquely determined such that the value represented by Formula 65 given below to be defined with respect to each $\mu$ is minimized, and $w^\mu$ represents MK+M+2K dimensional K unit vector (s) spanning a K dimensional singular value space of (MK+M+K) $\times$ (MK+M+2K) dimensional degenerate matrix D defined by Formula 70 given below. Although D appears as a 3$\times$4 matrix, each component further has a matrix. That is, in D represented as a 3$\times$4 matrix, the component in the first row and first column is a MK$\times$M matrix, the component in the first row and second column is a MK$\times$MK matrix, the component in the first row and third column is a MK$\times$K matrix, component in the second row and first column is s a M$\times$M matrix, the component in the second row and second column is a M$\times$MK matrix, the component in the second row and fourth column is a M$\times$K matrix, the component in the third row and second column is a K$\times$MK matrix, and other components are zero matrices. Elements of each component are formed by sweeping i or $\mu$, i=1 to M or $\mu$=1 to K, as the variable in the rows and j or v, j=1 to M or v=1 to K, as the variable in the columns.

$$\mathbf{Z} \equiv \begin{pmatrix} \sqrt{m_i} Rs_i \\ \varphi_i^{\mu} \\ \lambda^{\mu} \\ \rho^{\mu} \end{pmatrix}$$

Formula 68

$$\sum_{i=1}^{M} \left( m_i^{1/2} \frac{dRs_i}{dq^{\mu}} - \varphi_i^{\mu} \right)^2$$

Formula 69

$$D \equiv \begin{pmatrix} \sum_{k=1}^{M} V_{,ijk}(Rs)\varphi_k^{\mu} & (V_{,ij}(Rs) - \lambda^{\mu}\delta_{ij})\delta^{\mu\nu} & -\varphi_i^{\mu}\delta^{\mu\nu} & 0 \\ V_{,ij}(Rs) & -\rho^{\nu}\delta_{ij} & 0 & -\varphi_i^{\nu} \\ 0 & \varphi_j^{\mu}\delta^{\mu\nu} & 0 & 0 \end{pmatrix}$$

Formula 70

Formula 67 is a basic equation when K is generalized, and if K=1, Formula 67 is equivalent to Formula 71 given below. Further, as described in G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000, Formula 67 with respect to generalized K may be derived from Formula 71 where K=1 by using the Frobenius theorem.

$$\begin{aligned} \frac{\mathrm{d}Rs_i}{\mathrm{d}q^1} &= m_i^{-1/2}\chi_i \\ \frac{\mathrm{d}\varphi_i^1}{\mathrm{d}q^1} &= \psi_i \\ \frac{\mathrm{d}\lambda^1}{\mathrm{d}q^1} &= \kappa \\ \frac{\mathrm{d}\rho^1}{\mathrm{d}q^1} &= \sigma \end{aligned} \qquad for \quad i = 1, \cdots, M$$

Formula 71

In Formula 71, ($\chi_1$, -----, $\chi_M$, $\psi_1$, -----, $\psi_M$, $\kappa$, $\sigma$) is a 2M+2 dimensional solution vector (indeterminate solution) of homogeneous linear equation by the degenerate matrix of (2M+1)×(2M+2) represented by Formula 72 given below.

$$\begin{pmatrix} V_{,ijk}(Rs)\varphi_k^1 & V_{,ij}(Rs)-\lambda^1\delta_{ij} & -\varphi_i^1 & 0 \\ V_{,ij}(Rs) & -\rho^1\delta_{ij} & 0 & -\varphi_i^1 \\ 0 & \varphi_j^1 & 0 & 0 \end{pmatrix} \begin{pmatrix} \chi_i \\ \psi_i \\ \kappa \\ \sigma \end{pmatrix} = \begin{pmatrix} 0 \\ 0 \\ 0 \end{pmatrix}$$

Formula 72

In Formula 72, the product of the matrix and eigenvector is taken and when the Formula 68 is represented by three equations, the sum of 1 to M is taken with respect to j and k. That is, ($\chi_1$, -----,$\chi_M$, $\psi_1$, -----, $\psi_M$, K, $\sigma$) may be represented by Formula 73 by using a unit vector $\mu$ belonging to one-dimensional singular value space of the degenerate matrix of Formula 72. Here, c is a constant uniquely determined such that the value represented by Formula 74 given below is minimized.

$$\begin{pmatrix} \chi_i \\ \psi_i \\ \kappa \\ \sigma \end{pmatrix} = c\mathbf{u}$$

Formula 73

$$\sum_{i=1}^{M} \left(\chi_i - \varphi_i^1\right)^2$$

Formula 74

Equivalence of Formulae 71 and 72 to Formulae 41 to 43 may be proved in the following manner. If Formula 72 is integrated by $q^1$ with respect to ($\varphi_i^1$, $\lambda^1$, $\rho^1$) by paying attention to Formulae 38, 62, 71, and 73, Formula 75 given below may be obtained.

$$\sum_{j=1}^{M} \left( m_i^{-1/2} m_j^{-1/2} \frac{\partial^2}{\partial Rs_i \partial Rs_j} V - \lambda^1\, \delta_{ij} \right) \varphi_i^1 = 0 \qquad for \quad i = 1, \cdots, M$$

$$m_i^{-1/2} \frac{\partial}{\partial Rs_i} V - \rho^1 \varphi_i^1 = 0$$

$$\sum_{i=1}^{M} \varphi_i \varphi_i = \text{const.}$$

Formula 75

If $\varphi_i^1$ and $\rho^1$ are eliminated from the first and second equations of Formula 75, then Formula 76 given below may be obtained. Then, if Formula 76 is differentiated by presuming that $R_{Si}$ and $\lambda^1$ are functions of $q^1$, and first and third equations of Formula 71 are used, then Formula 77 given below may be obtained.

$$\sum_{j=1}^{M}\left(m_i^{-1/2}m_j^{-1/2}\frac{\partial^2}{\partial Rs_i \partial Rs_j}V-\lambda^1\delta_{ij}\right)m_j^{-1/2}\frac{\partial}{\partial Rs_j}V=0 \qquad for \quad i=1,\cdots,M$$

Formula 76

$$\sum_{j=1}^{M}m_i^{-1/2}m_j^{-1/2}\frac{\partial^2}{\partial Rs_i \partial Rs_j}\left(\frac{1}{2}\sum_{k=1}^{M}\left(m_k^{-1/2}\frac{\partial}{\partial Rs_k}V\right)^2-\lambda^1 V\right)\chi_i=m_i^{-1/2}\frac{\partial}{\partial Rs_i}V\kappa$$

$$for \quad i=1,\cdots,M$$

Formula 77

Here, by replacing $\lambda^1$, $\chi_i$, $\kappa$, and V in the first and third equations of Formula 71 and in Formula 77 with $\lambda$, $\varphi_i(R_S,\lambda)$, $\kappa(R_S, \lambda)$, and $V_{eff}(R_S)$, Formulae 41 to 43 are reproduced.

In the present embodiment, as is known from, for example, Formula 63 or 71, the number of variables as functions of $q^1$ treated as independent coordinates in solving the basic equation (total of the slow coordinates $R_S$ and auxiliary coordinates, and hereinafter referred to as the "independent variables") is increased. More specifically, in Formulae 39 and 40, the independent variables are only M $R_{Si}$ (i=1 to M), while in Formula 63 which is equivalent to Formulae 39 and 40, the independent variables are M $R_{Si}$ (i=1 to M), M $\varphi_i^1$ (i=1 to M), and $\Lambda^1$, totaling 2M+1. In the mean time, in Formulae 41 to 43, the independent variables are only M $R_{Si}$ (i=1 to M) and $\lambda$, while in Formula 71 which is equivalent to Formulae 41 to 43, the independent variables are M $R_{Si}$ (i=1 to M), M $\varphi_i^1$ (i=1 to M), $\lambda^1$, and $\rho^1$, totaling 2M+2.

Advantages of increasing the number of independent variables (more specifically, auxiliary coordinates) to equivalently transform the equations in the manner as described above will be described below.

For example, in the case where $\varphi_i(R_S)$ is obtained from Formula 40, arbitrariness remains in the sign of $\varphi_i(R_S)$, reflecting that Formula 40 is invariant to sign inversion of $\varphi_i(R_S)$. Further, the same applies to the case where $\varphi_i(R_S, \lambda)$ and $\kappa(R_S,\lambda)$ are obtained from Formula 43. The arbitrariness of the sign implies that a path that reversely runs the reaction path is also included. Given the fact that if a path that has reversely run once may return to forward direction by making a reverse run again, the reaction path may eventually be found out, it can be said that the arbitrariness of the sign is rather a natural phenomenon. But, the arbitrariness of the sign may cause a problem that the reverse run makes it difficult to efficiently form a reaction path. Further, Formulae 40 and 43 are solved by differencing in practice and the degree of the differencing $\Delta q^1$ has a finite value, so that when a reverse run occurs, the state of the system may not return to the original reaction path, that is, such reverse run may become a trigger formation of an erroneous reaction path.

Consequently, in the present embodiment, the arbitrariness of the sign is eliminated by increasing the number of independent variables $\varphi_i^\mu(R_S)$. As a result, the problem of the reverse run is solved and a reaction path may be formed more efficiently and accurately. Note that even if the number of independent variables is doubled, the burden due to increase in calculation load is negligible.

Figure 10 is a graph illustrating a result of comparison between calculation in which the arbitrariness of the sign is eliminated and calculation in which the arbitrariness of the sign is not eliminated. More specifically, the graph indicated by the reference numeral 24 illustrates a calculation result of the potential energy of a protein called melittin with respect to each structural change thereof using Formulae 71 to 74, while the graph indicated by the reference numeral 26 illustrates a calculation result of the potential energy of the same protein with respect to each structural change thereof using Formulae 41 to 43. The horizontal axis represents the flexion angle $\theta$ of the melittin for facilitating visualization of the structural change and the vertical axis represents the potential energy of the system. The state where $\theta$=140 degrees corresponds to the initial state.

In the calculation with the arbitrariness of the sign being remained, the energy rises sharply at about $\theta$=95 degrees where a reverse run has occurred. In contrast, in the calculation with the arbitrariness of the sign been eliminated, such a phenomenon does not appear and the energy reaches a local maximum point at about $\theta$=80 degrees. A separate point at $\theta$=60 degrees in the graph 24 is another local stable point of the system obtained from the state in the local maximum point by applying overall structural relaxation to the system.

As described above, the simulation apparatus and simulation method according to the present embodiment also predicts time evolution of mass point coordinates by introducing hierarchized slow coordinates, extracting, by taking into account influence of a change in fast coordinates on the slow coordinates due to a change in the slow coordinates, a collective coordinate in the collective motion theory that describes collective and intrinsic behavior of a mass point system, and solving the motion equation with respect to the collective coordinate. Thus, the present embodiment may provide advantageous effects identical to those of the first embodiment.

<Design Change>

In each embodiment described above, a term of the third derivative of potential energy $V(R_S)$ appears in the basic equations (e.g., Formulae 43, 60, 64, 70, and 72) . The order of time required for the calculation of the third derivative is proportional to $M^3$ (M represents the number of slow coordinates) and in view of the fact that the order of time required for the calculation of the second derivative is proportional to $M^2$, the calculation of the third derivate is a very heavy burden for the simulation. Thus, for example, with respect to $V_{,ijk}(R_S) \cdot \varphi_k^1$ in Formula 64, it takes a lot of time if $V_{,ijk}(R_S)$ is calculated first and then a product of the calculated value and $\varphi_k^1$ is taken. Consequently, the present inventor has developed Formula 78 given below, thereby allowing the $V_{,ijk}(R_S) \cdot \varphi_k^1$ to be calculated directly by taking the difference between the two second derivatives. The application of Formula 78 allows the order of calculation time to be reduced from about $M^3$ to about $M^2$. Further, it is preferable that the feedback equation of Formula 41 is applied to the second derivatives on the right hand side of Formula 78.

$$\sum_{k=1}^{M} V_{,ijk}(Rs)\cdot\phi_k = \lim_{\Delta x\to 0}\frac{V_{,ij}(Rs+\mathbf{n}\Delta x)-V_{,ij}(Rs-\mathbf{n}\Delta x)}{2\Delta x}$$

Formula 78

where, $\Phi_i$ represents $i^{th}$ component of an arbitrary vector and n is defined by Formula 79 given below.

$$\mathbf{n} \equiv (m_1^{-1/2}\phi_1,\cdots,m_i^{-1/2}\phi_i,\cdots,m_M^{-1/2}\phi_M)$$

Formula 79

(Basic Equation of SCC Method)

Finally, the relationship between the most fundamental basic equation (Formulae 34 to 36) and four sets of basic equations (set of Formulae 39 and 40, set of Formulae 41 to 43, Formula 57, and Formula 67) will be described briefly.

As described above, the function $R_{Si}(q^\mu)$ may generally be obtained by forming the plane according to the most fundamental basic equations. There are actually two problems, however, in forming the plane. One of them is a fundamental problem that a plane that strictly satisfies Formulae 34 to 36 is not always present for general potential energy. The other is a practical problem that a reverse run may occur if trying to form a plane according to Formulae 34 to 36 (problem of revere run described above). In particular, the cause of the latter problem is clear. That is, Formulae 35 and 36 are unable to determine the sign of $\varphi_i^\mu(R_S)$ and $\rho^\mu(R_S)$, in other words, they are invariant to sign inversion.

Consequently, for the fundamental problem, a method in which a plane that satisfies Formulae 34 to 36 is found by approximation is effective. In fact, in the description of G.D. Dang et al., "Self-consistent theory of large-amplitude collective motion: applications to approximate quantization of nonseparable systems and to nuclear physics", Physics Reports, Vol. 335, Issues 3-5, pp. 93-274, 2000, Formula 36 is abandoned and tries to form a plane only by Formulae 34 and 35. This measure will give Formulae 39 and 40 when K=1. Further, the present inventor has developed a method in which a plane is formed by Formulae 35 and 36 while Formula 34 is being satisfied to a maximum extent. This measure will give Formula 43 from Formula 41 when K=1. More specifically, if Formula 80 obtained by eliminating $\varphi_i^1(R_S)$ and $\rho^1(R_3)$ from Formulae 35 and 36 is differentiated by $q^1$ by presuming that $\lambda^1$ is a function of $q^1$, as well as $R_S$, Formula 43 may be obtained from Formula 41.

$$\sum_{j=1}^{M}(V_{,ij}(Rs)-\lambda^1(Rs)\delta_{ij})V_{,i}(Rs)=0 \qquad for \quad i=1,\cdots,M$$

Formula 80

With regard to the practical problem, a method in which $\varphi_i^\mu$, $\lambda^\mu$, and $\rho^\mu$ in Formulae 34 to 36 are presumed to be independent of $R_S$ and variables as functions of $q^\dagger$ (i.e., auxiliary coordinates) is effective. The reason is that if $\varphi_i^\mu$, $\lambda^\mu$, and $\rho^\mu$ are presumed to be independent of $R_S$, each of them becomes an amount that continuously varies little by little, like $R_S$, when forming the plane, so that a sudden change in the sign does not occur. In order to treat $\varphi_i^\mu$, $\lambda^\mu$, and $\rho^\mu$ as auxiliary coordinates, a formula that includes differentiation by $q^v$, like Formula 34, is required. Consequently, each of Formulae 35 and 36 is totally differentiated by $q^v$ and the result is divided by $dq^v$ to obtain Formulae 81 and 82 given below. In this way, the most fundamental basic equation is converted to the basic equation constituted by Formulae 34, 81, and 82 with the practical problem being eliminated.

$$\sum_{j=1}^{M}\left[\left(\sum_{k=1}^{M}\left(V_{,ijk}(Rs)\frac{dRs_k}{dq^{\nu}}\right)-\frac{d\lambda^{\mu}}{dq^{\nu}}\delta_{ij}\right)\varphi_j^{\mu}+(V_{,ij}(Rs)-\lambda^{\mu}\delta_{ij})\frac{d\varphi_j^{\mu}}{dq^{\nu}}\right]=0$$

$$for \quad i=1,\cdots,M \quad \mu,\nu=1,\cdots,K$$

Formula 81

$$\sum_{j=1}^{M}\left(V_{,ij}(Rs)\frac{dRs_j}{dq^{\nu}}\right)=\sum_{\mu=1}^{K}\left(\frac{d\rho^{\mu}}{dq^{\nu}}\varphi_i^{\mu}+\rho^{\mu}\frac{d\varphi_i^{\mu}}{dq^{\nu}}\right) \qquad for \quad i=1,\cdots,M \quad \nu=1,\cdots,K$$

Formula 82

Then, a consideration is given to address the fundamental problem in the basic equation constituted by Formulae 34, 81, and 82. The method of addressing the problem is, for example, a method in which a plane that satisfies Formulae 34, 81, and 82 is found by approximation as described above. More specifically, the measure in which the plane is formed only by Formulae 34 and 81 will give Formula 57, while the measure in which the plane is formed by Formulae 81 and 82 while Formula 34 is being satisfied to a maximum extent will give Formula 67.

[Industrial Applicability]

In each embodiment, the description has been made of a case in which the present invention is applied to the binding process of a composite body constituted by a protein and a drug, but the invention is not limited to this. That is, the present invention may be applied more commonly to the formation processes of composite bodies constituted by a biological macromolecule and a binding candidate molecule. The invention is also applicable to the analysis of behavior of polyatomic systems that includes a biological macromolecule, such as a dissociation process of composite body, a structure of apo body of protein, a folding mechanism of a biological macromolecule, and the like, other than the binding process of a composite body.

## Claims

**1.** A simulation apparatus (10) adapted to predict behavior of a modeled polyatomic system that includes a composite body constituted by N atoms of a biological macromolecule and a binding candidate molecule, the apparatus comprising:

a coordinate setting means (16) adapted to set slow coordinates which are M coordinates given by coordinates of centers of gravity of partial structures of the polyatomic system based on 3N mass point coordinates describing a structure of the polyatomic system, and fast coordinates which are coordinates of atoms and coordinates describing the structure of the polyatomic system and being independent of the centers of gravity of the partial structures;

a coordinate extraction means (18) adapted to obtain a structure of the fast coordinates as a function of the slow coordinates by subordinating the fast coordinates to the slow coordinates, and to obtain, by taking into account influence of a change of the position in the atoms on the partial structures due to a change of the position in the partial structures, a structure of the slow coordinates as a function of K collective coordinate(s) of a generalized coordinate which is associated with the slow coordinates by a canonical transformation, wherein the generalized coordinate is constituted by a variable component for which relative positional relationships between the partial structures vary with time and an invariable component that serves as a constant with respect to time and the K collective coordinate(s) is the variable component of the generalized coordinate; and

an inverse transformation means (20) adapted to predict time evolution of the polyatomic system based on the collective coordinate(s) as a function of time, which can be obtained as a solution of a motion equation with respect to the collective coordinate(s), the slow coordinates, and the fast coordinates, wherein, K, M, and N satisfy the relationship K<M<3N and each representing an integer not less than 1, the coordinate extraction means (18) is adapted to obtain the structure of the slow coordinates by:

performing a first step for obtaining potential energy V represented as a function of the slow coordinates and the fast coordinates;
performing a second step for representing the fast coordinates as functions of the slow coordinates according to an adiabatic approximation condition using the potential energy;
performing, under a current state of the slow coordinates and the fast coordinates, a third step for obtaining a differential coefficient of the potential energy with respect to the slow coordinates by taking into account the influence;
performing, under the differential coefficient of the potential energy, a fourth step for obtaining a differential coefficient of the slow coordinates with respect to the collective coordinate(s) according to a basic equation of self-consistent collective coordinate method using the differential coefficient of the potential energy;
performing, under the differential coefficient of the slow coordinates, a fifth step for updating the collective coordinate(s) by a small amount and obtaining updated slow coordinates;
performing, under the updated slow coordinates, a sixth step for performing structural relaxation on the fast coordinates subordinated to the slow coordinates; and
thereafter, repeating the third to sixth steps based on the slow coordinates and the fast coordinates in a state after the structural relaxation of the fast coordinates.

2. The simulation apparatus (10) of claim 1, **characterized in that** the influence is taken into account by a method that uses at least one of Formulae 1 to 3 given below:

$$\frac{\partial}{\partial R_{S_i}} V_{eff}(R_S) = \frac{\partial}{\partial R_{S_i}} V(R_S, R_F)\Big|_{R_F=R_F(R_S)}$$

Formula 1

$$\begin{aligned}\frac{\partial^2}{\partial R_{S_i}\partial R_{S_j}} V_{eff}(R_S) &= \frac{\partial^2}{\partial R_{S_i}\partial R_{S_j}} V(R_S, R_F)\Big|_{R_F=R_F(R_S)} \\ &+ \left(\sum_{\beta=1}^{3N-M} X_\beta{}^j(R_S) \frac{\partial^2}{\partial R_{S_i}\partial R_{F_\beta}} V(R_S, R_F)\Big|_{R_F=R_F(R_S)} + (i \leftrightarrow j)\right) \\ &+ \sum_{\alpha=1}^{3N-M}\sum_{\beta=1}^{3N-M} X_\alpha{}^i(R_S) X_\beta{}^j(R_S) \frac{\partial^2}{\partial R_{F_\alpha}\partial R_{F_\beta}} V(R_S, R_F)\Big|_{R_F=R_F(R_S)}\end{aligned}$$

Formula 2

$$\begin{aligned}\frac{\partial^3}{\partial R_{S_i}\partial R_{S_j}\partial R_{S_k}} V_{eff}(R_S) &= \frac{\partial^3}{\partial R_{S_i}\partial R_{S_j}\partial R_{S_k}} V(R_S, R_F)\Big|_{R_F=R_F(R_S)} \\ &+ \left(\sum_{\gamma=1}^{3N-M} X_\gamma{}^k(R_S) \frac{\partial^3}{\partial R_{S_i}\partial R_{S_j}\partial R_{F_\gamma}} V(R_S, R_F)\Big|_{R_F=R_F(R_S)} + (i \leftrightarrow k) + (j \leftrightarrow k)\right) \\ &+ \left(\sum_{\alpha=1}^{3N-M}\sum_{\beta=1}^{3N-M} X_\alpha{}^i(R_S) X_\beta{}^j(R_S) \frac{\partial^3}{\partial R_{F_\alpha}\partial R_{F_\beta}\partial R_{S_k}} V(R_S, R_F)\Big|_{R_F=R_F(R_S)} + (i \leftrightarrow k) + (j \leftrightarrow k)\right) \\ &+ \sum_{\alpha=1}^{3N-M}\sum_{\beta=1}^{3N-M}\sum_{\gamma=1}^{3N-M} X_\alpha{}^i(R_S) X_\beta{}^j(R_S) X_\gamma{}^k(R_S) \frac{\partial^3}{\partial R_{F_\alpha}\partial R_{F_\beta}\partial R_{F_\gamma}} V(R_S, R_F)\Big|_{R_F=R_F(R_S)}\end{aligned}$$

Formula 3

where: i, j, and k each represents an integer in the range from 1 to M;

$\alpha$, $\beta$, and $\gamma$ each represents an integer in the range from 1 to 3N-M;
$R_{Si}$ represents $i^{th}$ slow coordinate in the polyatomic system;
$R_{F\alpha}$ represents $\alpha^{th}$ fast coordinate in the polyatomic system;

$R_{Fß}$ represents ß$^{th}$ fast coordinate in the polyatomic system
$R_S$ represents ($R_{S1}$, $R_{S2}$, ------, $R_{SM}$);
$R_F$ represents ($R_{F1}$, $R_{F2}$, ------, $R_{F(3N-M)}$);
$R_F$ ($R_S$) represents the fast coordinates as functions of the slow coordinates;
V ($R_S$,$R_F$) represents the potential energy represented by the slow coordinates and the fast coordinates;
$V_{eff}(R_S)$ represents effective potential energy to be obtained by substituting $R_F(R_{sS})$ into V($R_S$, $R_F$);
in Formula 2, (i↔j) in the third term represents a term derived by mutually replacing alphabets i and j in the second term;
in Formula 3, (i↔k) in the third term represents a term derived by mutually replacing alphabets i and k in the second term, (j↔k) in the fourth term represents a term derived by mutually replacing alphabets j and k in the second term, (i↔k) in the sixth term represents a term derived by mutually replacing alphabets i and k in the fifth term, and (j↔k) in the seventh term represents a term derived by mutually replacing alphabets j and k in the fifth term; and further
in Formulae 1 to 3, Formula 4 given below is used.

$$X_\alpha{}^i(Rs) \equiv -\sum_{\beta=1}^{3N-M} K_{\alpha\beta}^{-1}(Rs) J^{\alpha i}(Rs)$$

Formula 4

where: $K_{\alpha\beta}^{-1}$ ($R_S$) represents an inverse matrix of $K^{\alpha\beta}$ ($R_S$) , and
$K^{\alpha\beta}$ ($R_S$) and $J^{\alpha i}$ ($R_S$) are defined by Formulae 5 and 6 given below respectively.

$$K^{\alpha\beta}(Rs) \equiv \frac{\partial^2}{\partial R_{F_\alpha} \partial R_{F_\beta}} V(Rs, R_F)\Big|_{R_F = R_F(Rs)}$$

Formula 5

$$J^{\alpha j}(Rs) \equiv \frac{\partial^2}{\partial R_{F_\alpha} \partial Rs_j} V(Rs, R_F)\Big|_{R_F = R_F(Rs)}$$

Formula 6

3. The simulation apparatus (10) of claim 1 or 2, **characterized in that** the adiabatic approximation condition is Formula 7 given below.

$$\frac{\partial}{\partial R_{F_\alpha}} V(Rs, R_F) = 0 \qquad for \quad \alpha = 1, \cdots, 3N - M$$

Formula 7

where: $R_{F\alpha}$ represents $\alpha^{th}$ fast coordinate in the polyatomic system;

$R_S$ represents ($R_{S1}$, $R_{S2}$, ------, $R_{SM}$);
$R_F$ represents ($R_{F1}$, $R_{F2}$, ------, $R_{F(3N-M)}$); and
V ($R_S$,$R_F$) represents the potential energy represented by the slow coordinates and the fast coordinates.

4. The simulation apparatus (10) of any of claims 1 to 3, **characterized in that** the number K of the collective coordinate (s) satisfies K=1, and the basic equation is represented by Formulae 8 and 9 given below.

$$\frac{dRs_i}{dq^1} = m_i^{-1/2} \varphi_i(Rs) \qquad for \quad i = 1, \cdots, M$$

Formula 8

$$\sum_{j=1}^{M}\left( m_i^{-1/2} m_j^{-1/2} \frac{\partial^2}{\partial Rs_i \partial Rs_j} V_{eff}(Rs) - \Lambda(Rs)\,\delta_{ij} \right)\varphi_i\,(Rs) = 0 \qquad for \quad i = 1,\cdots,M$$

Formula 9

where: i and j each represents an integer in the range from 1 to M;

$R_{Si}$ represents ith slow coordinate in the polyatomic system;
$R_S$ represents ($R_{S1}$, $R_{S2}$, ------, $R_{SM}$);
$q^1$ represents the collective coordinate;
$m_i$ represents a mass of ith slow coordinate in the polyatomic system;
$\varphi_i(R_S)$ represents ith component of a function (eigenvector) that satisfies Formula 9;
$\Lambda$(Rs) represents a function (eigenvalue) that satisfies Formula 9; and
$V_{eff}(R_S)$ represents effective potential energy.

5. The simulation apparatus (10) of any of claims 1 to 3, **characterized in that** the number K of the collective coordinate (s) satisfies K=1, and the basic equation is represented by Formulae 10 to 12 given below.

$$\frac{\mathrm{d}Rs_i}{\mathrm{d}q^1} = m_i^{-1/2}\varphi_i(Rs,\lambda) \qquad for \quad i = 1,\cdots,M$$

Formula 10

$$\frac{\mathrm{d}\lambda}{\mathrm{d}q^1} = \kappa\,(Rs,\lambda)$$

Formula 11

$$\sum_{j=1}^{M} m_i^{-1/2} m_j^{-1/2} \frac{\partial^2}{\partial Rs_i \partial Rs_j}\left( \frac{1}{2}\sum_{k=1}^{M}\left( m_k^{-1/2}\frac{\partial}{\partial Rs_k} V_{eff}(Rs)\right)^2 - \lambda\ V_{eff}(Rs)\right)\varphi_i\,(Rs,\lambda) = m_i^{-1/2}\frac{\partial}{\partial Rs_i} V_{eff}(Rs)\,\kappa\,(Rs,\lambda)$$

$$for \quad i = 1,\cdots,M$$

Formula 12

where: i, j, and k each represents an integer in the range from 1 to M;

$R_{Si}$ represents ith slow coordinate in the polyatomic system;
$R_S$ represents ($R_{S1}$, $R_{S2}$, ------, $R_{SM}$);
$q^1$ represents the collective coordinate;
$m_i$ represents a mass of ith slow coordinate in the polyatomic system;
$\varphi_i$ ($R_S$,$\lambda$) and $\kappa$ ($R_S$,$\lambda$) represent ith component of a function that satisfies Formula 12 and $\lambda$ represents an auxiliary coordinate; and
$V_{eff}(R_S)$ represents effective potential energy.

6. The simulation apparatus (10) of any of claims 1 to 3, **characterized in that** the coordinate extraction means (18) is a means adapted to perform calculation in the fourth step by increasing the number of variables treated as independent of the slow coordinates and as functions of the collective coordinate(s) in solving the basic equation in order to eliminate the arbitrariness of sign of a differential coefficient of the slow coordinates or auxiliary coordinates with respect to the collective coordinate(s) in the basic equation.

7. The simulation apparatus (10) of claim 6, **characterized in that** the coordinate extraction means (18) is a means adapted to perform calculation according to a basic equation represented by Formula 13 given below obtained as a result of increasing the number of variables treated as independent of the slow coordinates and as functions of the collective coordinate(s).

$$\frac{\mathrm{d}\mathbf{Y}}{\mathrm{d}q^{\mu}} = \mathbf{v}^{\mu} \qquad for \quad \mu = 1, \cdots, K$$

Formula 13

where, Y is a MK+M+K dimensional vector defined by Formula 14 given below, and $v^{\mu}$ is a solution vector of the inhomogeneous linear equation of Formula 15 given below.

$$\mathbf{Y} \equiv \begin{pmatrix} \sqrt{m_i} Rs_i \\ \varphi_i^{\mu} \\ \Lambda^{\mu} \end{pmatrix}$$

Formula 14

$$C\mathbf{v}^{\mu} = \mathbf{s}^{\mu} \qquad for \quad \mu = 1, \cdots, K$$

Formula 15

C and $s^{\mu}$ in Formula 15 are defined by Formulae 16 and 17 given below respectively.

$$C \equiv \begin{pmatrix} \sum_{k=1}^{M} V_{,ijk}(Rs)\varphi_i^{\mu} & (V_{,ij}(Rs) - \Lambda^{\mu}\delta_{ij})\delta^{\mu\nu} & -\varphi_i^{\mu}\delta^{\mu\nu} \\ 0 & \varphi_j^{\mu}\delta^{\mu\nu} & 0 \\ \delta_{ij} & 0 & 0 \end{pmatrix}$$

Formula 16

$$\mathbf{s}^{\mu} \equiv \begin{pmatrix} 0 \\ 0 \\ \varphi_i^{\mu} \end{pmatrix} \qquad for \quad \mu = 1, \cdots, K$$

Formula 17

where, $V_{,ij}(R_S)$ and $V_{,ijk}(R_S)$ are defined by Formulae 18 and 19 given below respectively.

$$V_{,ij}(Rs) \equiv (m_i m_j)^{-1/2} \frac{\partial^2}{\partial Rs_i \partial Rs_j} V_{eff}(Rs)$$

Formula 18

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formula 19

where: i, j, and k each represents an integer in the range from 1 to M;

$\mu$ and v each represents an integer in the range from 1 to K;
$q^\mu$ represents $\mu^{th}$ collective coordinate;
$R_{Si}$ represents $i^{th}$ slow coordinate in the polyatomic system;
$R_S$ represents ($R_{S1}$, $R_{S2}$, ------, $R_{SM}$);
$m_i$ represents a mass of $i^{th}$ slow coordinate in the polyatomic system;
$\varphi_i^\mu$ and $\Lambda^u$ each represents an auxiliary coordinate independent of $R_S$; and
$V_{eff}(R_S)$ represents effective potential energy.

**8.** The simulation apparatus (10) of claim 7, **characterized in that** the number K of the collective coordinate(s) satisfies K=1.

**9.** The simulation apparatus (10) of claim 6, **characterized in that** the coordinate extraction means (18) is a means adapted to perform calculation according to a basic equation represented by Formula 20 given below obtained as a result of increasing the number of variables treated as independent of the slow coordinates and as functions of the collective coordinate (s) .

$$\frac{d\mathbf{Z}}{dq^\mu} = \sum_{v=1}^{K} c^{\mu v} \mathbf{w}^\mu \qquad for \quad \mu = 1, \cdots, K$$

Formula 20

where: Z is a MK+M+2K dimensional vector defined by Formula 21 given below;

$c^{\mu v}$ is a constant uniquely determined such that the value represented by Formula 22 given below to be defined with respect to each $\mu$ is minimized; and
$w^\mu$ represents MK+M+2K dimensional K unit vector(s) spanning a K dimensional singular value space of matrix D defined by Formula 23 given below.

$$\mathbf{Z} \equiv \begin{pmatrix} \sqrt{m_i} Rs_i \\ \varphi_i^\mu \\ \lambda^\mu \\ \rho^\mu \end{pmatrix}$$

Formula 21

$$\sum_{i=1}^{M} \left( m_i^{1/2} \frac{dRs_i}{dq^\mu} - \varphi_i^\mu \right)^2$$

Formula 22

$$D \equiv \begin{pmatrix} \sum_{k=1}^{M} V_{,ijk}(Rs)\varphi_k^{\mu} & (V_{,ij}(Rs) - \lambda^{\mu}\delta_{ij})\delta^{\mu\nu} & -\varphi_i^{\mu}\delta^{\mu\nu} & 0 \\ V_{,ij}(Rs) & -\rho^{\nu}\delta_{ij} & 0 & -\varphi_i^{\nu} \\ 0 & \varphi_i^{\mu}\delta^{\mu\nu} & 0 & 0 \end{pmatrix}$$

Formula 23

where, $V_{,ij}(R_S)$ and $V_{,ijk}$ $(R_S)$ are defined by Formulae 24 and 25 given below respectively.

$$V_{,ij}(Rs) \equiv (m_i m_j)^{-1/2} \frac{\partial^2}{\partial Rs_i \partial Rs_j} V_{eff}(Rs)$$

Formula 24

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formula 25

where: i, j, and k each represents an integer in the range from 1 to M;

$\mu$ and v each represents an integer in the range from 1 to K;
$q^{\mu}$ represents $\mu^{th}$ collective coordinate;
$R_{Si}$ represents $i^{th}$ slow coordinate in the polyatomic system;
$R_S$ represents ($R_{S1}$, $R_{S2}$, ------, $R_{SM}$);
$m_i$ represents a mass of $i^{th}$ slow coordinate in the polyatomic system;
$\varphi_i^{\mu}$, $\lambda^{\mu}$, and $\rho^{\mu}$ each represents an auxiliary coordinate independent of $R_S$; and
$V_{eff}(R_S)$ represents effective potential energy.

**10.** The simulation apparatus (10) of claim 9, **characterized in that** the number K of the collective coordinate(s) satisfies K=1.

**11.** The simulation apparatus (10) of any of claims of 5 to 10, **characterized in that** the coordinate extraction means (18) is a means adapted to calculate the term of third derivative of the potential energy based on Formula 26 given below.

$$\sum_{k=1}^{M} V_{,ijk}(Rs) \cdot \phi_k = \lim_{\Delta x \to 0} \frac{V_{,ij}(Rs + \mathbf{n}\Delta x) - V_{,ij}(Rs - \mathbf{n}\Delta x)}{2\Delta x}$$

Formula 26

where, $\Phi_i$ represents $i^{th}$ component of an arbitrary vector, and $V_{,ij}$ $(R_S)$, $V_{,ijk}$ $(R_S)$ , and n are defined respectively by Formulae 27 to 29 given below.

$$V_{,ij}(Rs) \equiv (m_i m_j)^{-1/2} \frac{\partial^2}{\partial Rs_i \partial Rs_j} V_{eff}(Rs)$$

Formula 27

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formula 28

$$\mathbf{n} \equiv (m_1^{-1/2}\phi_1, \cdots, m_i^{-1/2}\phi_i, \cdots, m_M^{-1/2}\phi_M)$$

Formula 29

**12.** The simulation apparatus (10) of claim 1, **characterized in that** the coordinate setting means (16) is a means adapted to set representative coordinates extracted from and representing each characteristic partial structure of the structure of the polyatomic system as the slow coordinates,

the partial structure is a secondary structure, a building block or a main chain of the biological macromolecule;
the representative coordinate of each partial structure is a coordinate of each of atoms constituting the partial structure, a coordinate defined by combining coordinates of the atoms, or a pitch of the partial structures,
the biological macromolecule is a protein, the partial structure is a secondary structure of the protein, and the slow coordinate of the secondary structure is a coordinate of the center of gravity of a group of atoms constituting the secondary structure.

**13.** The simulation apparatus (10) of claim 12, **characterized in that** the secondary structure is at least one of helix structure, β sheet, turn, loop, and random coil.

**14.** The simulation apparatus (10) of claim 1, **characterized in that** the biological macromolecule is a protein, the partial structure is a residue of the protein, and the slow coordinate of the residue is a coordinate of the center of gravity of a group of atoms constituting the residue.

**15.** The simulation apparatus (10) of claim 1, **characterized in that** the biological macromolecule is a protein, the partial structure is a main chain of the protein, and the slow coordinate of the main chain is a coordinate of each atom constituting the main chain.

**16.** The simulation apparatus (10) of claim 1, **characterized in that** the biological macromolecule is a nucleic acid, the partial structure is a secondary structure of the nucleic acid, and the slow coordinate of the secondary structure is a coordinate of the center of gravity of a group of atoms constituting the secondary structure.

**17.** The simulation apparatus (10) of claim 16, **characterized in that** the secondary structure is a helical structure.

**18.** The simulation apparatus (10) of claim 1, **characterized in that** the biological macromolecule is a nucleic acid, the partial structure is a residue of the nucleic acid, and the slow coordinate of the residue is a coordinate of the center of gravity of a group of atoms constituting the residue.

**19.** The simulation apparatus (10) of claim 1, **characterized in that** the biological macromolecule is a nucleic acid, the partial structure is a main chain of the nucleic acid, and the slow coordinate of the main chain is a coordinate of each atom constituting the main chain.

**20.** A simulation method for use with the simulation apparatus (10) of any of claims 1 to 19 for predicting behavior of a modeled polyatomic system that includes a composite body constituted by N atoms of a biological macromolecule and a binding candidate molecule, the method comprising the steps of:

setting slow coordinates which are M coordinates given by coordinates of centers of gravity of partial structures of the polyatomic system based on 3N mass point coordinates describing a structure of the polyatomic system;
setting fast coordinates which are coordinates of atoms and coordinates describing the structure of the polyatomic system and being independent of the centers of gravity of the partial structures;
obtaining the fast coordinates as a function of the slow coordinates;
obtaining, by taking into account influence of a change of the position in the atoms on the partial structures due to a change of the position in the partial structures, the slow coordinates as a function of K collective coordinate(s) of a generalized coordinate which is associated with the slow coordinates by a canonical transformation, wherein the generalized coordinate is constituted by a variable component for which relative positional relationships between the partial structures vary with time, and an invariable component that serves as a constant with respect to time and the K collective coordinate(s) is the variable component of the generalized coordinate; and

predicting time evolution of the polyatomic system based on the collective coordinate(s) as a function of time, which can be obtained as a solution of a motion equation with respect to the collective coordinate(s), the slow coordinates, and the fast coordinates,

wherein, K, M, and N satisfy the relationship K<M<3N and each representing an integer not less than 1, wherein the structure of the slow coordinates are obtained by:

performing a first step for obtaining potential energy V represented as a function of the slow coordinates and the fast coordinates;

performing a second step for representing the fast coordinates as functions of the slow coordinates according to an adiabatic approximation condition using the potential energy;

performing, under a current state of the slow coordinates and the fast coordinates, a third step for obtaining a differential coefficient of the potential energy with respect to the slow coordinates by taking into account the influence;

performing, under the differential coefficient of the potential energy, a fourth step for obtaining a differential coefficient of the slow coordinates with respect to the collective coordinate(s) according to a basic equation of self-consistent collective coordinate method using the differential coefficient of the potential energy;

performing, under the differential coefficient of the slow coordinates, a fifth step for updating the collective coordinate(s) by a small amount and obtaining updated slow coordinates;

performing, under the updated slow coordinates, a sixth step for performing structural relaxation on the fast coordinates subordinated to the slow coordinates; and

thereafter, repeating the third to sixth steps based on the slow coordinates and the fast coordinates in a state after the structural relaxation of the fast coordinates.

**21.** A computer readable recording medium on which is recorded a simulation program for causing a computer to perform the simulation method of claim 20.

## Patentansprüche

**1.** Simulationsvorrichtung (10), die so ausgelegt ist, dass sie Verhalten eines modellierten polyatomaren Systems, das einen Verbundkörper, der aus N Atomen eines biologischen Makromoleküls und einem Bindungskandidatenmolekül aufgebaut ist, enthält, vorhersagt, wobei die Vorrichtung umfasst:

ein Koordinateneinstellmittel (16), das so ausgelegt ist, dass es langsame Koordinaten, die M Koordinaten sind, die durch Koordinaten von Schwerpunkten von Teilstrukturen des polyatomaren Systems auf der Grundlage von 3N Massenpunkt-Koordinaten, die eine Struktur des polyatomaren Systems beschreiben, gegeben sind, und schnelle Koordinaten, die Koordinaten von Atomen und Koordinaten, die die Struktur des polyatomaren Systems beschreiben, sind und unabhängig von den Schwerpunkten der Teilstrukturen sind, einstellt;

ein Koordinatenextraktionsmittel (18), das so ausgelegt ist, dass es eine Struktur der schnellen Koordinaten als eine Funktion der langsamen Koordinaten durch Unterordnen der schnellen Koordinaten unter die langsamen Koordinaten erhält und durch Berücksichtigen von Einfluss einer Änderung der Position in den Atomen auf die Teilstrukturen aufgrund einer Änderung der Position in den Teilstrukturen eine Struktur der langsamen Koordinaten als eine Funktion von K kollektiver Koordinate(n) einer verallgemeinerten Koordinate, die den langsamen Koordinaten durch eine kanonische Transformation zugeordnet ist, erhält, wobei die verallgemeinerte Koordinate durch eine variable Komponente, für die relative Positionsbeziehungen zwischen den Teilstrukturen sich mit der Zeit ändern, und eine invariable Komponente, die als eine Konstante in Bezug auf Zeit dient, gebildet ist und die K kollektive Koordinate(n) die variable Komponente der verallgemeinerten Koordinate ist; und

ein Inversetransformationsmittel (20), das so ausgelegt ist, dass es zeitliche Entwicklung des polyatomaren Systems auf der Grundlage der kollektiven Koordinate(n) als eine Funktion von Zeit vorhersagt, die als eine Lösung einer Bewegungsgleichung in Bezug auf die kollektive Koordinate(n), die langsamen Koordinaten und die schnellen Koordinaten erhalten werden kann, wobei K, M und N die Beziehung K < M < 3N erfüllen und jeweils eine ganze Zahl, die nicht kleiner als 1 ist, darstellen, wobei das Koordinatenextraktionsmittel (18) so ausgelegt ist, dass es die Struktur der langsamen Koordinaten erhält durch:

Durchführen eines ersten Schritts zum Erhalten von potenzieller Energie V, die als eine Funktion der langsamen Koordinaten und der schnellen Koordinaten dargestellt wird;
Durchführen eines zweiten Schritts zum Darstellen der schnellen Koordinaten als Funktionen der langsamen Koordinaten gemäß einer adiabatischen Näherungsbedingung unter Verwendung der potenziellen Energie;
Durchführen, unter einem aktuellen Zustand der langsamen Koordinaten und der schnellen Koordinaten, eines dritten Schritts zum Erhalten eines Differentialkoeffizienten der potenziellen Energie in Bezug auf die langsamen Koordinaten durch Berücksichtigen des Einflusses;
Durchführen, unter dem Differentialkoeffizienten der potenziellen Energie, eines vierten Schritts zum Erhalten eines Differentialkoeffizienten der langsamen Koordinaten in Bezug auf die kollektive Koordinate(n) gemäß einer Grundgleichung eines selbstkonsistenten kollektiven Koordinatenverfahrens unter Verwendung des Differentialkoeffizienten der potenziellen Energie;
Durchführen, unter dem Differentialkoeffizienten der langsamen Koordinaten, eines fünften Schritts zum Aktualisieren der kollektiven Koordinate(n) um einen kleinen Betrag und zum Erhalten von aktualisierten langsamen Koordinaten;
Durchführen, unter den aktualisierten langsamen Koordinaten, eines sechsten Schritts zum Durchführen von struktureller Relaxation an den schnellen Koordinaten, die den langsamen Koordinaten untergeordnet sind; und
anschließend Wiederholen des dritten bis sechsten Schritts auf der Grundlage der langsamen Koordinaten und der schnellen Koordinaten in einem Zustand nach der strukturellen Relaxation der schnellen Koordinaten.

2. Simulationsvorrichtung (10) nach Anspruch 1, **dadurch gekennzeichnet, dass** der Einfluss durch ein Verfahren berücksichtigt wird, das mindestens eine von unten angegebenen Formeln 1 bis 3 verwendet:

$$\frac{\partial}{\partial Rs_i}V_{eff}(Rs)=\frac{\partial}{\partial Rs_i}V(Rs,Rf)\bigg|_{Rf=Rf(Rs)} \qquad \text{Formel 1}$$

$$\begin{aligned}\frac{\partial^2}{\partial Rs_i\partial Rs_j}V_{eff}(Rs)&=\frac{\partial^2}{\partial Rs_i\partial Rs_j}V(Rs,Rf)\bigg|_{Rf=Rf(Rs)}\\&+\left(\sum_{\beta=1}^{3N-M}X_\beta{}^j(Rs)\frac{\partial^2}{\partial Rs_i\partial Rf_\beta}V(Rs,Rf)\bigg|_{Rf=Rf(Rs)}+(i\leftrightarrow j)\right)\\&+\sum_{\alpha=1}^{3N-M}\sum_{\beta=1}^{3N-M}X_\alpha{}^i(Rs)X_\beta{}^j(Rs)\frac{\partial^2}{\partial Rf_\alpha\partial Rf_\beta}V(Rs,Rf)\bigg|_{Rf=Rf(Rs)}\end{aligned} \qquad \text{Formel 2}$$

$$\begin{aligned}\frac{\partial^3}{\partial Rs_i\partial Rs_j\partial Rs_k}V_{eff}(Rs)&=\frac{\partial^3}{\partial Rs_i\partial Rs_j\partial Rs_k}V(Rs,Rf)\bigg|_{Rf=Rf(Rs)}\\&+\left(\sum_{\gamma=1}^{3N-M}X_\gamma{}^k(Rs)\frac{\partial^3}{\partial Rs_i\partial Rs_j\partial Rf_\gamma}V(Rs,Rf)\bigg|_{Rf=Rf(Rs)}+(i\leftrightarrow k)+(j\leftrightarrow k)\right)\\&+\left(\sum_{\alpha=1}^{3N-M}\sum_{\beta=1}^{3N-M}X_\alpha{}^i(Rs)X_\beta{}^j(Rs)\frac{\partial^3}{\partial Rf_\alpha\partial Rf_\beta\partial Rs_k}V(Rs,Rf)\bigg|_{Rf=Rf(Rs)}+(i\leftrightarrow k)+(j\leftrightarrow k)\right)\\&+\sum_{\alpha=1}^{3N-M}\sum_{\beta=1}^{3N-M}\sum_{\gamma=1}^{3N-M}X_\alpha{}^i(Rs)X_\beta{}^j(Rs)X_\gamma{}^k(Rs)\frac{\partial^3}{\partial Rf_\alpha\partial Rf_\beta\partial Rf_\gamma}V(Rs,Rf)\bigg|_{Rf=Rf(Rs)}\end{aligned} \qquad \text{Formel 3}$$

wobei: i, j und k jeweils eine ganze Zahl in dem Bereich von 1 bis M darstellen;

$\alpha$, $\beta$ und $\gamma$ jeweils eine ganze Zahl in dem Bereich von 1 bis 3N-M darstellen;
$R_{Si}$ $i^{-te}$ langsame Koordinate in dem polyatomaren System darstellt;
$R_{F\alpha}$ $\alpha^{-te}$ schnelle Koordinate in dem polyatomaren System darstellt;
$R_{F\beta}$ $\beta^{-te}$ schnelle Koordinate in dem polyatomaren System darstellt;
$R_S$ ($R_{S1}$, $R_{S2}$, ------, $R_{SM}$) darstellt;
$R_F$ ($R_{F1}$, $R_{F2}$, ------, $R_{F(3N-M)}$) darstellt;
$R_F(R_{sS})$ die schnellen Koordinaten als Funktionen der langsamen Koordinaten darstellt;
$V(R_S, R_F)$ die potenzielle Energie darstellt, die durch die langsamen Koordinaten und die schnellen Koordinaten dargestellt wird;
$V_{eff}(R_S)$ effektive potenzielle Energie darstellt, die durch Substituieren von $R_F(R_{sS})$ in $V(R_S, R_F)$ zu erhalten ist;
in Formel 2 ($i\leftrightarrow j$) in dem dritten Term einen Term darstellt, der durch gegenseitiges Ersetzen von Buchstaben i und j in dem zweiten Term abgeleitet wird;
in Formel 3 ($i\leftrightarrow k$) in dem dritten Term einen Term darstellt, der durch gegenseitiges Ersetzen von Buchstaben i und k in dem zweiten Term abgeleitet wird, ($j\leftrightarrow k$) in dem vierten Term einen Term darstellt, der durch gegenseitiges Ersetzen von Buchstaben j und k in dem zweiten Term abgeleitet wird, ($i\leftrightarrow k$) in dem sechsten Term einen Term darstellt, der durch gegenseitiges Ersetzen von Buchstaben i und k in dem fünften Term abgeleitet wird, und ($j\leftrightarrow k$) in dem siebten Term einen Term darstellt, der durch gegenseitiges Ersetzen von Buchstaben j und k in dem fünften Term abgeleitet wird; und ferner
in Formeln 1 bis 3 die unten angegebene Formel 4 verwendet wird,

$$X_\alpha{}^i(Rs) \equiv -\sum_{\beta=1}^{3N-M} K_{\alpha\beta}^{-1}(Rs) J^{\alpha i}(Rs)$$

Forme' 4

wobei: $K_{\alpha\beta}{}^{-1}(R_S)$ eine inverse Matrix von $K^{\alpha\beta}(R_S)$ darstellt, und
$K^{\alpha\beta}(R_S)$ und $J^{\alpha i}(R_S)$ entsprechend durch unten angegebene Formeln 5 und 6 definiert sind.

$$K^{\alpha\beta}(Rs) \equiv \frac{\partial^2}{\partial R_{F_\alpha} \partial R_{F_\beta}} V(Rs, R_F)\Big|_{R_F = R_F(Rs)}$$

Forme 5

$$J^{\alpha j}(Rs) \equiv \frac{\partial^2}{\partial R_{F_\alpha} \partial Rs_j} V(Rs, R_F)\Big|_{R_F = R_F(Rs)}$$

Forme 6

3. Simulationsvorrichtung (10) nach Anspruch 1 oder 2, **dadurch gekennzeichnet, dass** die adiabatische Näherungsbedingung die unten angegebene Formel 7 ist,

$$\frac{\partial}{\partial R_{F_\alpha}} V(Rs, R_F) = 0 \qquad \text{für} \qquad \alpha = 1, \cdots, 3N - M$$

Forme! 7

wobei: $R_{F\alpha}$ $\alpha^{-te}$ schnelle Koordinate in dem polyatomaren System darstellt;
$R_S$ ($R_{S1}$, $R_{S2}$, ------, $R_{SM}$) darstellt;
$R_F$ ($R_{F1}$, $R_{F2}$, ------, $R_{F(3N-M)}$) darstellt; und
$V(R_S, R_F)$ die potenzielle Energie darstellt, die durch die langsamen Koordinaten und die schnellen Koordinaten dargestellt wird.

4. Simulationsvorrichtung (10) nach einem der Ansprüche 1 bis 3, **dadurch gekennzeichnet, dass** die Anzahl K der kollektiven Koordinate(n) K = 1 erfüllt und die Grundgleichung durch unten angegebene Formeln 8 und 9 dargestellt wird,

$$\frac{dRs_i}{dq^1} = m_i^{-1/2}\varphi_i(Rs) \qquad \text{für} \qquad i = 1, \cdots, M$$

Formel 8

$$\sum_{j=1}^{M}\left(m_i^{-1/2}m_j^{-1/2}\frac{\partial^2}{\partial Rs_i \partial Rs_j}V_{eff}(Rs) - \Lambda(Rs)\,\delta_{ij}\right)\varphi_j(Rs) = 0 \qquad \text{für} \qquad i = 1, \cdots, M$$

Formel 9

wobei: i und j jeweils eine ganze Zahl in dem Bereich von 1 bis M darstellen;
$R_{Si}$ $i^{-te}$ langsame Koordinate in dem polyatomaren System darstellt;
$R_S$ ($R_{S1}$, $R_{S2}$, ------, $R_{SM}$) darstellt;
$q^1$ die kollektive Koordinate darstellt;
$m_i$ eine Masse von $i^{-ter}$ langsamer Koordinate in dem polyatomaren System darstellt;
$\varphi_i(R_S)$ $i^{-te}$ Komponente einer Funktion (Eigenvektor) darstellt, die Formel 9 erfüllt;
$\Lambda(R_S)$ eine Funktion (Eigenwert) darstellt, die Formel 9 erfüllt; und
$V_{eff}(R_S)$ effektive potenzielle Energie darstellt.

**5.** Simulationsvorrichtung (10) nach einem der Ansprüche 1 bis 3, **dadurch gekennzeichnet, dass** die Anzahl K der kollektiven Koordinate(n) K = 1 erfüllt und die Grundgleichung durch unten angegebene Formeln 10 bis 12 dargestellt wird,

$$\frac{dRs_i}{dq^1} = m_i^{-1/2}\varphi_i(Rs,\lambda) \qquad \text{für} \qquad i = 1, \cdots, M$$

Formel 10

$$\frac{d\lambda}{dq^1} = \kappa(Rs,\lambda)$$

Formel 11

$$\sum_{j=1}^{M} m_i^{-1/2}m_j^{-1/2}\frac{\partial^2}{\partial Rs_i \partial Rs_j}\left(\frac{1}{2}\sum_{k=1}^{M}\left(m_k^{-1/2}\frac{\partial}{\partial Rs_k}V_{eff}(Rs)\right)^2 - \lambda\, V_{eff}(Rs)\right)\varphi_j(Rs,\lambda) = m_i^{-1/2}\frac{\partial}{\partial Rs_i}V_{eff}(Rs)\,\kappa(Rs,\lambda)$$

$$\text{für} \qquad i = 1, \cdots, M$$

Formel 12

wobei: i, j und k jeweils eine ganze Zahl in dem Bereich von 1 bis M darstellen;
$R_{Si}$ $i^{-te}$ langsame Koordinate in dem polyatomaren System darstellt;
$R_S$ ($R_{S1}$, $R_{S2}$, ------, $R_{SM}$) darstellt;
$q^1$ die kollektive Koordinate darstellt;
$m_i$ eine Masse von $i^{-ter}$ langsamer Koordinate in dem polyatomaren System darstellt;
$\varphi_i(R_S, \lambda)$ und $\kappa(R_S, \lambda)$ $i^{-te}$ Komponente einer Funktion darstellen, die Formel 12 erfüllt, und $\lambda$ eine Hilfskoordinate darstellt; und
$V_{eff}(R_S)$ effektive potenzielle Energie darstellt.

**6.** Simulationsvorrichtung (10) nach einem der Ansprüche 1 bis 3, **dadurch gekennzeichnet, dass** das Koordinatenextraktionsmittel (18) ein Mittel ist, das so ausgelegt ist, dass es Berechnung bei dem vierten Schritt durch Erhöhen der Anzahl an Variablen, die als unabhängig von den langsamen Koordinaten und als Funktionen der kollektiven Koordinate(n) behandelt werden, bei Lösen der Grundgleichung durchführt, um die Willkürlichkeit von Vorzeichen eines Differentialkoeffizienten der langsamen Koordinaten oder Hilfskoordinaten in Bezug auf die kollektive Koordinate(n) bei der Grundgleichung zu beseitigen.

**7.** Simulationsvorrichtung (10) nach Anspruch 6, **dadurch gekennzeichnet, dass** das Koordinatenextraktionsmittel (18) ein Mittel ist, das so ausgelegt ist, dass es Berechnung gemäß einer durch unten angegebene Formel 13 dargestellten Grundgleichung, die als ein Ergebnis von Erhöhen der Anzahl an Variablen, die als unabhängig von den langsamen Koordinaten und als Funktionen der kollektiven Koordinate(n) behandelt werden, erhalten wird, durchführt,

$$\frac{d\mathbf{Y}}{dq^{\mu}} = \mathbf{v}^{\mu} \qquad \text{für} \qquad \mu = 1, \cdots, K$$

Formel 13

wobei Y ein MK + M + K-dimensionaler Vektor ist, der durch unten angegebene Formel 14 definiert ist, und $v^{\mu}$ ein Lösungsvektor der inhomogenen linearen Gleichung von unten angegebenen Formel 15 ist,

$$\mathbf{Y} \equiv \begin{pmatrix} \sqrt{m_i} Rs_i \\ \varphi_i^{\mu} \\ \Lambda^{\mu} \end{pmatrix}$$

Formel 14

$$C\mathbf{v}^{\mu} = \mathbf{s}^{\mu} \qquad \text{für} \qquad \mu = 1, \cdots, K$$

Formel 15

C und $s^{\mu}$ in Formel 15 entsprechend durch unten angegebene Formeln 16 und 17 definiert sind,

$$C \equiv \begin{pmatrix} \sum_{k=1}^{M} V_{,ijk}(Rs)\varphi_i^{\mu} & (V_{,ij}(Rs) - \Lambda^{\mu}\delta_{ij})\delta^{\mu\nu} & -\varphi_i^{\mu}\delta^{\mu\nu} \\ 0 & \varphi_j^{\mu}\delta^{\mu\nu} & 0 \\ \delta_{ij} & 0 & 0 \end{pmatrix}$$

Formel 16

$$\mathbf{s}^{\mu} \equiv \begin{pmatrix} 0 \\ 0 \\ \varphi_i^{\mu} \end{pmatrix} \qquad \text{für} \qquad \mu = 1, \cdots, K$$

Formel 17

wobei $V_{,ij}(R_S)$ und $V_{,ijk}(R_S)$ entsprechend durch unten angegebene Formeln 18 und 19 definiert sind,

$$V_{,ij}(Rs) \equiv (m_i m_j)^{-1/2} \frac{\partial^2}{\partial Rs_i \partial Rs_j} V_{eff}(Rs)$$

Formel 18

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formel 19

wobei: i, j und k jeweils eine ganze Zahl in dem Bereich von 1 bis M darstellen;
$\mu$ und v jeweils eine ganze Zahl in dem Bereich von 1 bis K darstellen;
$q^\mu$ $\mu$-te kollektive Koordinate darstellt;
$R_{Si}$ i-te langsame Koordinate in dem polyatomaren System darstellt;
$R_S$ ($R_{S1}$, $R_{S2}$, ------, $R_{SM}$) darstellt;
$m_i$ eine Masse von i-ter langsamer Koordinate in dem polyatomaren System darstellt;
$\varphi_i^\mu$ und $\Lambda^\mu$ jeweils eine von $R_S$ unabhängige Hilfskoordinate darstellen; und
$V_{eff}(R_S)$ effektive potenzielle Energie darstellt.

**8.** Simulationsvorrichtung (10) nach Anspruch 7, **dadurch gekennzeichnet, dass** die Anzahl K der kollektiven Koordinate(n) K = 1 erfüllt.

**9.** Simulationsvorrichtung (10) nach Anspruch 6, **dadurch gekennzeichnet, dass** das Koordinatenextraktionsmittel (18) ein Mittel ist, das so ausgelegt ist, dass es Berechnung gemäß einer durch unten angegebene Formel 20 dargestellten Grundgleichung, die als ein Ergebnis von Erhöhen der Anzahl an Variablen, die als unabhängig von den langsamen Koordinaten und als Funktionen der kollektiven Koordinate(n) behandelt werden, erhalten wird, durchführt,

$$\frac{\mathrm{d}Z}{\mathrm{d}q^\mu} = \sum_{\nu=1}^{K} c^{\mu\nu}\,\mathbf{w}^\mu \qquad \text{für} \qquad \mu = 1,\cdots,K$$

Forme 20

wobei: Z ein MK + M + 2K-dimensionaler Vektor ist, der durch unten angegebene Formel 21 definiert ist;
$c^{\mu\nu}$ eine Konstante ist, die eindeutig so bestimmt ist, dass der durch unten angegebene Formel 22 für jedes $\mu$ definierte Wert minimiert wird; und
$w^\mu$ MK + M + 2K-dimensionalen K-Einheitsvektor(en) darstellt, der einen K-dimensionalen Singulärwertraum von Matrix D, die durch unten angegebene Formel 23 definiert ist, aufspannt,

$$Z \equiv \begin{pmatrix} \sqrt{m_i}\,Rs_i \\ \varphi_i^\mu \\ \lambda^\mu \\ \rho^\mu \end{pmatrix}$$

Forme 21

$$\sum_{i=1}^{M}\left(m_i^{1/2}\frac{dRs_i}{dq^\mu} - \varphi_i^\mu\right)^2$$

Forme 22

$$D \equiv \begin{pmatrix} \sum_{k=1}^{M} V_{,ijk}(Rs)\varphi_k^\mu & (V_{,ij}(Rs) - \lambda^\mu\delta_{ij})\delta^{\mu\nu} & -\varphi_i^\mu\delta^{\mu\nu} & 0 \\ V_{,ij}(Rs) & -\rho^\nu\delta_{ij} & 0 & -\varphi_i^\nu \\ 0 & \varphi_i^\mu\delta^{\mu\nu} & 0 & 0 \end{pmatrix}$$

Forme 23

wobei $V_{,ij}(R_S)$ und $V_{,ijk}(R_S)$ entsprechend durch unten angegebene Formeln 24 und 25 definiert sind,

$$V_{,ij}(Rs) \equiv (m_i m_j)^{-1/2}\frac{\partial^2}{\partial Rs_i \partial Rs_j}V_{eff}(Rs)$$

Forme 24

$$V_{,ijk}(Rs) \equiv (m_i m_j m_k)^{-1/2} \frac{\partial^3}{\partial Rs_i \partial Rs_j \partial Rs_k} V_{eff}(Rs)$$

Forme' 25

wobei: i, j und k jeweils eine ganze Zahl in dem Bereich von 1 bis M darstellen;
$\mu$ und v jeweils eine ganze Zahl in dem Bereich von 1 bis K darstellen;
$q^\mu$ $\mu$-te kollektive Koordinate darstellt;
$R_{Si}$ i-te langsame Koordinate in dem polyatomaren System darstellt;
$R_S$ ($R_{S1}$, $R_{S2}$, ------, $R_{SM}$) darstellt;
$m_i$ eine Masse von i-ter langsamer Koordinate in dem polyatomaren System darstellt;
$\varphi_i^\mu$, $\lambda^\mu$ und $\rho^\mu$ jeweils eine von $R_S$ unabhängige Hilfskoordinate darstellen; und
$V_{eff}(R_S)$ effektive potenzielle Energie darstellt.

**10.** Simulationsvorrichtung (10) nach Anspruch 9, **dadurch gekennzeichnet, dass** die Anzahl K der kollektiven Koordinate(n) K = 1 erfüllt.

**11.** Simulationsvorrichtung (10) nach einem der Ansprüche 5 bis 10, **dadurch gekennzeichnet, dass** das Koordinatenextraktionsmittel (18) ein Mittel ist, das so ausgelegt ist, dass es den Term von dritter Ableitung der potenziellen Energie auf der Grundlage von Formel 26, die unten gegeben ist, berechnet,

$$\sum_{k=1}^{M} V_{,ijk}(Rs) \cdot \phi_k = \lim_{\Delta x \to 0} \frac{V_{,ij}(Rs + \mathbf{n}\Delta x) - V_{,ij}(Rs - \mathbf{n}\Delta x)}{2\Delta x}$$

Forme 26

wobei $\varphi_i$ i-te Komponente eines beliebigen Vektors darstellt und $V_{,ij}(R_S)$, $V_{ijk}(R_S)$ und n entsprechend durch unten angegebene Formeln 27 bis 29 definiert sind.

$$V_{,ij}(Rs) \equiv (m_i m_j)^{1/2} \frac{\partial^2}{\partial Rs_i \partial Rs_j} V_{eff}(Rs)$$

Forme' 27

$$V_{,ijk}(Rs) \equiv (m_i m_j m_k)^{-1/2} \frac{\partial^3}{\partial Rs_i \partial Rs_j \partial Rs_k} V_{eff}(Rs)$$

Forme' 28

$$\mathbf{n} \equiv (m_1^{-1/2}\phi_1, \cdots, m_i^{-1/2}\phi_i, \cdots, m_M^{-1/2}\phi_M)$$

Forme' 29

**12.** Simulationsvorrichtung (10) nach Anspruch 1, **dadurch gekennzeichnet, dass** das Koordinateneinstellmittel (16) ein Mittel ist, das so ausgelegt ist, dass es repräsentative Koordinaten, die aus jeder charakteristischen Teilstruktur der Struktur des polyatomaren Systems extrahiert sind und jeweils diese darstellen, als die langsamen Koordinaten einstellt,

die Teilstruktur eine Sekundärstruktur, ein Baustein oder eine Hauptkette des biologischen Makromoleküls ist, die repräsentative Koordinate jeder Teilstruktur eine Koordinate jedes von Atomen, die die Teilstruktur bilden, eine Koordinate, die durch Kombinieren von Koordinaten der Atome definiert ist, oder ein Abstand der Teilstrukturen ist,
das biologische Makromolekül ein Protein ist, die Teilstruktur eine Sekundärstruktur des Proteins ist und die langsame Koordinate der Sekundärstruktur eine Koordinate des Schwerpunkts einer Gruppe von Atomen, die Sekundärstruktur bilden, ist.

**13.** Simulationsvorrichtung (10) nach Anspruch 12, **dadurch gekennzeichnet, dass** die Sekundärstruktur mindestens eine von Helixstruktur, β-Faltblatt, Windung, Schleife und Zufallswendel ist.

**14.** Simulationsvorrichtung (10) nach Anspruch 1, **dadurch gekennzeichnet, dass** das biologische Makromolekül ein Protein ist, die Teilstruktur ein Residuum des Proteins ist und die langsame Koordinate des Residuums eine Koordinate des Schwerpunkts einer Gruppe von Atomen, die das Residuum bilden, ist.

**15.** Simulationsvorrichtung (10) nach Anspruch 1, **dadurch gekennzeichnet, dass** das biologische Makromolekül ein Protein ist, die Teilstruktur eine Hauptkette des Proteins ist und die langsame Koordinate der Hauptkette eine Koordinate jedes Atoms, das die Hauptkette bildet, ist.

**16.** Simulationsvorrichtung (10) nach Anspruch 1, **dadurch gekennzeichnet, dass** das biologische Makromolekül eine Nukleinsäure ist, die Teilstruktur eine Sekundärstruktur der Nukleinsäure ist und die langsame Koordinate der Sekundärstruktur eine Koordinate des Schwerpunkts einer Gruppe von Atomen, die die Sekundärstruktur bilden, ist.

**17.** Simulationsvorrichtung (10) nach Anspruch 16, **dadurch gekennzeichnet, dass** die Sekundärstruktur eine helikale Struktur ist.

**18.** Simulationsvorrichtung (10) nach Anspruch 1, **dadurch gekennzeichnet, dass** das biologische Makromolekül eine Nukleinsäure ist, die Teilstruktur ein Residuum der Nukleinsäure ist und die langsame Koordinate des Residuums eine Koordinate des Schwerpunkts einer Gruppe von Atomen, die das Residuum bilden, ist.

**19.** Simulationsvorrichtung (10) nach Anspruch 1, **dadurch gekennzeichnet, dass** das biologische Makromolekül eine Nukleinsäure ist, die Teilstruktur eine Hauptkette der Nukleinsäure ist und die langsame Koordinate der Hauptkette eine Koordinate jedes Atoms, das die Hauptkette bildet, ist.

**20.** Simulationsverfahren zum Verwenden mit der Simulationsvorrichtung (10) nach einem der Ansprüche 1 bis 19 zum Vorhersagen von Verhalten eines modellierten polyatomaren Systems, das einen zusammengesetzten Körper, der aus N Atomen eines biologischen Makromoleküls und einem Bindungskandidatenmolekül aufgebaut ist, enthält, wobei das Verfahren die folgenden Schritte umfasst:

Einstellen von langsamen Koordinaten, die M Koordinaten sind, die durch Koordinaten von Schwerpunkten von Teilstrukturen des polyatomaren Systems auf der Grundlage von 3N Massenpunkt-Koordinaten, die eine Struktur des polyatomaren Systems beschreiben, gegeben sind;
Einstellen von schnellen Koordinaten, die Koordinaten von Atomen und Koordinaten, die die Struktur des polyatomaren Systems beschreiben, sind und unabhängig von den Schwerpunkten der Teilstrukturen sind;
Erhalten der schnellen Koordinaten als eine Funktion der langsamen Koordinaten;
Erhalten, durch Berücksichtigen von Einfluss einer Änderung der Position in den Atomen auf die Teilstrukturen aufgrund einer Änderung der Position in den Teilstrukturen, der langsamen Koordinaten als eine Funktion von K kollektiver Koordinate(n) einer verallgemeinerten Koordinate, die den langsamen Koordinaten durch eine kanonische Transformation zugeordnet ist, wobei die verallgemeinerte Koordinate durch eine variable Komponente, für die relative Positionsbeziehungen zwischen den Teilstrukturen sich mit der Zeit ändern, und eine invariable Komponente, die als eine Konstante in Bezug auf Zeit dient, gebildet ist und die K kollektive Koordinate(n) die variable Komponente der verallgemeinerten Koordinate ist; und
Vorhersagen von zeitlicher Entwicklung des polyatomaren Systems auf der Grundlage der kollektiven Koordinate(n) als einer Funktion von Zeit, die als eine Lösung einer Bewegungsgleichung in Bezug auf die kollektive Koordinate(n), die langsamen Koordinaten und die schnellen Koordinaten erhalten werden kann,
wobei K, M und N die Beziehung $K < M < 3N$ erfüllen und jeweils eine ganze Zahl, die nicht kleiner als 1 ist, darstellen, wobei die Struktur der langsamen Koordinaten erhalten wird durch:

Durchführen eines ersten Schritts zum Erhalten von potenzieller Energie V, die als eine Funktion der langsamen Koordinaten und der schnellen Koordinaten dargestellt wird;
Durchführen eines zweiten Schritts zum Darstellen der schnellen Koordinaten als Funktionen der langsamen Koordinaten gemäß einer adiabatischen Näherungsbedingung unter Verwendung der potenziellen Energie;
Durchführen, unter einem aktuellen Zustand der langsamen Koordinaten und der schnellen Koordinaten, eines dritten Schritts zum Erhalten eines Differentialkoeffizienten der potenziellen Energie in Bezug auf die langsamen Koordinaten durch Berücksichtigen des Einflusses;

Durchführen, unter dem Differentialkoeffizienten der potenziellen Energie, eines vierten Schritts zum Erhalten eines Differentialkoeffizienten der langsamen Koordinaten in Bezug auf die kollektive Koordinate(n) gemäß einer Grundgleichung eines selbstkonsistenten kollektiven Koordinatenverfahrens unter Verwendung des Differentialkoeffizienten der potenziellen Energie;
Durchführen, unter dem Differentialkoeffizienten der langsamen Koordinaten, eines fünften Schritts zum Aktualisieren der kollektiven Koordinate(n) um einen kleinen Betrag und zum Erhalten von aktualisierten langsamen Koordinaten;
Durchführen, unter den aktualisierten langsamen Koordinaten, eines sechsten Schritts zum Durchführen von struktureller Relaxation an den schnellen Koordinaten, die den langsamen Koordinaten untergeordnet sind; und
anschließend Wiederholen des dritten bis sechsten Schritts auf der Grundlage der langsamen Koordinaten und der schnellen Koordinaten in einem Zustand nach der strukturellen Relaxation der schnellen Koordinaten.

**21.** Computerlesbares Aufzeichnungsmedium, auf dem ein Simulationsprogramm aufgezeichnet ist, um einen Computer zu veranlassen, das Simulationsverfahren nach Anspruch 20 durchzuführen.

## Revendications

**1.** Appareil de simulation (10) adapté pour prédire un comportement d'un système polyatomique modélisé qui inclut un corps composite constitué de N atomes d'une macromolécule biologique et d'une molécule candidate de liaison, l'appareil comprenant :

un moyen de réglage de coordonnées (16) adapté pour régler des coordonnées lentes qui sont des M coordonnées données par des coordonnées de centres de gravité de structures partielles du système polyatomique sur la base de 3N coordonnées de point de masse décrivant une structure du système polyatomique, et des coordonnées rapides qui sont des coordonnées d'atomes et des coordonnées décrivant la structure du système polyatomique et étant indépendantes des centres de gravité des structures partielles ;
un moyen d'extraction de coordonnées (18) adapté pour obtenir une structure des coordonnées rapides en fonction des coordonnées lentes en subordonnant les coordonnées rapides aux coordonnées lentes, et pour obtenir, en tenant compte de l'influence d'un changement de la position des atomes sur les structures partielles dû à un changement de la position dans les structures partielles, une structure des coordonnées lentes en fonction de K coordonnée(s) collective(s) d'une coordonnée généralisée qui est associée aux coordonnées lentes par une transformation canonique, où la coordonnée généralisée est constituée d'une composante variable pour laquelle des relations de position relative entre les structures partielles varient dans le temps et d'une composante invariable qui sert de constante par rapport au temps, et la/les K coordonnée(s) collective(s) est/sont la composante variable de la coordonnée généralisée ; et
un moyen de transformation inverse (20) adapté pour prédire une évolution temporelle du système polyatomique sur la base de la/les coordonnée(s) collective(s) en fonction du temps, qui peut être obtenue comme solution d'une équation de mouvement par rapport à la/les coordonnée(s) collective(s), aux coordonnées lentes et aux coordonnées rapides, où K, M et N satisfont la relation $K < M < 3N$ et représentant chacun un entier supérieur ou égal à 1, le moyen d'extraction de coordonnées (18) est adapté pour obtenir la structure des coordonnées lentes par :

effectuer une première étape pour obtenir une énergie potentielle V représentée en fonction des coordonnées lentes et des coordonnées rapides ;
effectuer une deuxième étape pour représenter les coordonnées rapides en fonction des coordonnées lentes selon une condition d'approximation adiabatique en utilisant l'énergie potentielle ;
effectuer, dans un état actuel des coordonnées lentes et des coordonnées rapides, une troisième étape pour obtenir un coefficient différentiel de l'énergie potentielle par rapport aux coordonnées lentes en tenant compte de l'influence ;
effectuer, sous le coefficient différentiel de l'énergie potentielle, une quatrième étape pour obtenir un coefficient différentiel des coordonnées lentes par rapport à la/les coordonnée(s) collective(s) selon une équation de base de la méthode des coordonnées collectives auto-cohérentes en utilisant le coefficient différentiel de l'énergie potentielle ;
effectuer, sous le coefficient différentiel des coordonnées lentes, une cinquième étape pour mettre à jour la/les coordonnée(s) collective(s) d'une petite quantité et obtenir des coordonnées lentes mises à jour ;

effectuer, sous les coordonnées lentes mises à jour, une sixième étape pour effectuer une relaxation structurale sur les coordonnées rapides subordonnées aux coordonnées lentes ; et
ensuite, répéter les troisièmes à sixièmes étapes sur la base des coordonnées lentes et des coordonnées rapides dans un état après la relaxation structurale des coordonnées rapides.

**2.** Appareil de simulation (10) selon la revendication 1, **caractérisé en ce que** l'influence est prise en compte par un procédé qui utilise au moins l'une des Formules 1 à 3 données ci-dessous :

$$\frac{\partial}{\partial Rs_i} V_{eff}(Rs) = \frac{\partial}{\partial Rs_i} V(Rs, R_F)\Big|_{R_F = R_F(Rs)}$$

Formule 1

$$\frac{\partial^2}{\partial Rs_i \partial Rs_j} V_{eff}(Rs) = \frac{\partial^2}{\partial Rs_i \partial Rs_j} V(Rs, R_F)\Big|_{R_F = R_F(Rs)} + \left( \sum_{\beta=1}^{3N-M} X_\beta{}^j(Rs) \frac{\partial^2}{\partial Rs_i \partial R_{F_\beta}} V(Rs, R_F)\Big|_{R_F = R_F(Rs)} + (i \leftrightarrow j) \right) + \sum_{\alpha=1}^{3N-M} \sum_{\beta=1}^{3N-M} X_\alpha{}^i(Rs) X_\beta{}^j(Rs) \frac{\partial^2}{\partial R_{F_\alpha} \partial R_{F_\beta}} V(Rs, R_F)\Big|_{R_F = R_F(Rs)}$$

Formule 2

$$\frac{\partial^3}{\partial Rs_i \partial Rs_j \partial Rs_k} V_{eff}(Rs) = \frac{\partial^3}{\partial Rs_i \partial Rs_j \partial Rs_k} V(Rs, R_F)\Big|_{R_F = R_F(Rs)} + \left( \sum_{\gamma=1}^{3N-M} X_\gamma{}^k(Rs) \frac{\partial^3}{\partial Rs_i \partial Rs_j \partial R_{F_\gamma}} V(Rs, R_F)\Big|_{R_F = R_F(Rs)} + (i \leftrightarrow k) + (j \leftrightarrow k) \right) + \left( \sum_{\alpha=1}^{3N-M} \sum_{\beta=1}^{3N-M} X_\alpha{}^i(Rs) X_\beta{}^j(Rs) \frac{\partial^3}{\partial R_{F_\alpha} \partial R_{F_\beta} \partial Rs_k} V(Rs, R_F)\Big|_{R_F = R_F(Rs)} + (i \leftrightarrow k) + (j \leftrightarrow k) \right) + \sum_{\alpha=1}^{3N-M} \sum_{\beta=1}^{3N-M} \sum_{\gamma=1}^{3N-M} X_\alpha{}^i(Rs) X_\beta{}^j(Rs) X_\gamma{}^k(Rs) \frac{\partial^3}{\partial R_{F_\alpha} \partial R_{F_\beta} \partial R_{F_\gamma}} V(Rs, R_F)\Big|_{R_F = R_F(Rs)}$$

Formule 3

où : i, j et k représentent chacun un entier compris entre 1 et M ;
$\alpha$, $\beta$ et $\gamma$ représentent chacun un entier compris entre 1 et 3N-M ;
$R_{Si}$ représente la i^e coordonnée lente dans le système polyatomique ;
$R_{F\alpha}$ représente la $\alpha^e$ coordonnée rapide dans le système polyatomique ;
$R_{F\beta}$ représente la $\beta^e$ coordonnée rapide dans le système polyatomique ;
$R_S$ représente ($R_{S1}$, $R_{S2}$, ------, $R_{SM}$) ;
$R_F$ représente ($R_{F1}$, $R_{F2}$, ------, $R_{F(3N-M)}$) ;
$R_F(R_S)$ représente les coordonnées rapides en fonction des coordonnées lentes ;
$V(R_S, R_F)$ représente l'énergie potentielle représentée par les coordonnées lentes et les coordonnées rapides ;
$V_{eff}(R_S)$ représente une énergie potentielle effective à obtenir en substituant $R_F(R_S)$ dans $V(R_S, R_F)$ ;
dans Formule 2, (i↔j) dans le troisième terme représente un terme dérivé en échangeant mutuellement les indices i et j dans le deuxième terme ;
dans Formule 3, (i↔k) dans le troisième terme représente un terme dérivé en échangeant mutuellement les indices i et k dans le deuxième terme, (j↔k) dans le quatrième terme représente un terme dérivé en échangeant mutuellement les indices j et k dans le deuxième terme, (i↔k) dans le sixième terme représente un terme dérivé en échangeant mutuellement les indices i et k dans le cinquième terme, et (j↔k) dans le septième terme représente un terme dérivé en échangeant mutuellement les indices j et k dans le cinquième terme ; et de plus dans Formules 1 à 3, Formule 4 donnée ci-dessous est utilisée,

$$X_\alpha{}^i(Rs) \equiv -\sum_{\beta=1}^{3N-M} K_{\alpha\beta}^{-1}(Rs) J^{\alpha i}(Rs)$$

Formule 4

où : $K_{\alpha\beta}^{-1}(R_S)$ représente une matrice inverse de $K^{\alpha\beta}(R_S)$, et
$K^{\alpha\beta}(R_S)$ et $J^{\alpha i}(R_S)$ sont définis respectivement par Formules 5 et 6 données ci-dessous.

$$K^{\alpha\beta}(Rs) \equiv \frac{\partial^2}{\partial R_{F_\alpha} \partial R_{F_\beta}} V(Rs, R_F)\Big|_{R_F = R_F(Rs)}$$

Formule 5

$$J^{\alpha j}(Rs) \equiv \frac{\partial^2}{\partial R_{F_\alpha} \partial Rs_j} V(Rs, R_F)\Big|_{R_F = R_F(Rs)}$$

Formule 6

**3.** Appareil de simulation (10) selon la revendication 1 ou la revendication 2, **caractérisé en ce que** la condition d'approximation adiabatique est Formule 7 donnée ci-dessous,

$$\frac{\partial}{\partial R_{F_\alpha}} V(Rs, R_F) = 0 \qquad \text{pour} \qquad \alpha = 1, \cdots, 3N - M$$

Formule 7

où : $R_{F\alpha}$ représente la $\alpha^e$ coordonnée rapide dans le système polyatomique ;
$R_S$ représente $(R_{S1}, R_{S2}, \text{------}, R_{SM})$ ;
$R_F$ représente $(R_{F1}, R_{F2}, \text{------}, R_{F(3N-M)})$ ; et
$V(R_S, R_F)$ représente l'énergie potentielle représentée par les coordonnées lentes et les coordonnées rapides.

**4.** Appareil de simulation (10) selon l'une quelconque des revendications 1 à 3, **caractérisé en ce que** le nombre K de la/les coordonnée(s) collective(s) satisfait K = 1, et l'équation de base est représentée par Formules 8 et 9 données ci-dessous,

$$\frac{dRs_i}{dq^1} = m_i^{-1/2} \varphi_i(Rs) \qquad \text{pour} \qquad i = 1, \cdots, M$$

Formule 8

$$\sum_{j=1}^{M} \left( m_i^{-1/2} m_j^{-1/2} \frac{\partial^2}{\partial Rs_i \partial Rs_j} V_{eff}(Rs) - \Lambda(Rs)\,\delta_{ij} \right) \varphi_i(Rs) = 0 \qquad \text{pour} \qquad i = 1, \cdots, M$$

Formule 9

où : i et j représentent chacun un entier compris entre 1 et M ;
$R_{Si}$ représente la $i^e$ coordonnée lente dans le système polyatomique ;
$R_S$ représente $(R_{S1}, R_{S2}, \text{------}, R_{SM})$ ;
$q^1$ représente la coordonnée collective ;
$m_i$ représente une masse de la $i^e$ coordonnée lente dans le système polyatomique ;
$\varphi_i(R_S)$ représente la $i^e$ composante d'une fonction (vecteur propre) qui satisfait Formule 9 ;

$A(R_S)$ représente une fonction (valeur propre) qui satisfait Formule 9 ; et
$V_{eff}(R_S)$ représente une énergie potentielle effective.

5. Appareil de simulation (10) selon l'une quelconque des revendications 1 à 3, **caractérisé en ce que** le nombre K de la/les coordonnée(s) collective(s) satisfait K = 1, et l'équation de base est représentée par Formules 10 à 12 données ci-dessous,

$$\frac{dRs_i}{dq^1} = m_i^{-1/2}\varphi_i(Rs,\lambda) \quad \text{pour} \quad i = 1,\cdots,M$$

Formule 10

$$\frac{d\lambda}{dq^1} = \kappa(Rs,\lambda)$$

Formule 11

$$\sum_{j=1}^{M} m_i^{-1/2} m_j^{-1/2} \frac{\partial^2}{\partial Rs_i \partial Rs_j}\left(\frac{1}{2}\sum_{k=1}^{M}\left(m_k^{-1/2}\frac{\partial}{\partial Rs_k}V_{eff}(Rs)\right)^2 - \lambda\, V_{eff}(Rs)\right)\varphi_j(Rs,\lambda) = m_i^{-1/2}\frac{\partial}{\partial Rs_i}V_{eff}(Rs)\,\kappa(Rs,\lambda)$$

$$\text{pour} \quad i = 1,\cdots,M$$

Formule 12

où : i, j et k représentent chacun un entier compris entre 1 et M ;
$R_{Si}$ représente la i[e] coordonnée lente dans le système polyatomique ;
$R_S$ représente ($R_{S1}$, $R_{S2}$, ------, $R_{SM}$) ;
$q^1$ représente la coordonnée collective ;
$m_i$ représente une masse de la i[e] coordonnée lente dans le système polyatomique ;
$\varphi_i(R_S, \lambda)$ et $\kappa(R_S, \lambda)$ représentent la i[e] composante d'une fonction qui satisfait Formule 12, et $\lambda$ représente une coordonnée auxiliaire ; et
$V_{eff}(R_S)$ représente une énergie potentielle effective.

6. Appareil de simulation (10) selon l'une quelconque des revendications 1 à 3, **caractérisé en ce que** le moyen d'extraction de coordonnées (18) est un moyen adapté pour effectuer un calcul dans la quatrième étape en augmentant le nombre de variables traitées comme indépendantes des coordonnées lentes et comme fonctions de la/les coordonnée(s) collective(s) lors de la résolution de l'équation de base afin de éliminer l'arbitraire du signe d'un coefficient différentiel des coordonnées lentes ou des coordonnées auxiliaires par rapport à la/les coordonnée(s) collective(s) dans l'équation de base.

7. Appareil de simulation (10) selon la revendication 6, **caractérisé en ce que** le moyen d'extraction de coordonnées (18) est un moyen adapté pour effectuer un calcul selon une équation de base représentée par Formule 13 donnée ci-dessous, obtenue à la suite de l'augmentation du nombre de variables traitées comme indépendantes des coordonnées lentes et comme fonctions de la/les coordonnée(s) collective(s),

$$\frac{d\mathbf{Y}}{dq^\mu} = \mathbf{v}^\mu \quad \text{pour} \quad \mu = 1,\cdots,K$$

Formule 13

où, Y est un vecteur de dimension MK + M + K défini par Formule 14 donnée ci-dessous, et $v^\mu$ est un vecteur solution de l'équation linéaire inhomogène de Formule 15 donnée ci-dessous,

$$\mathbf{Y} \equiv \begin{pmatrix} \sqrt{m_i} Rs_i \\ \varphi_i^\mu \\ \Lambda^\mu \end{pmatrix}$$

Formule 14

$$C\mathbf{v}^\mu = \mathbf{s}^\mu \qquad \text{pour} \qquad \mu = 1, \cdots, K$$

Formule 15

C et $s^\mu$ dans Formule 15 sont définis respectivement par Formules 16 et 17 données ci-dessous,

$$C \equiv \begin{pmatrix} \sum_{k=1}^{M} V_{,ijk}(Rs)\varphi_i^\mu & (V_{,ij}(Rs) - \Lambda^\mu \delta_{ij})\delta^{\mu\nu} & -\varphi_i^\mu \delta^{\mu\nu} \\ 0 & \varphi_j^\mu \delta^{\mu\nu} & 0 \\ \delta_{ij} & 0 & 0 \end{pmatrix}$$

Formule 16

$$\mathbf{s}^\mu \equiv \begin{pmatrix} 0 \\ 0 \\ \varphi_i^\mu \end{pmatrix} \qquad \text{pour} \qquad \mu = 1, \cdots, K$$

Formule 17

où, $V_{,ij}(R_S)$ et $V_{,ijk}(R_S)$ sont définis respectivement par Formules 18 et 19 données ci-dessous,

$$V_{,ij}(Rs) \equiv (m_i m_j)^{-1/2} \frac{\partial^2}{\partial Rs_i \partial Rs_j} V_{eff}(Rs)$$

Formule 18

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formule 19

où : i, j et k représentent chacun un entier compris entre 1 et M ;
$\mu$ et v représentent chacun un entier compris entre 1 et K ;
$q^\mu$ représente la $\mu^e$ coordonnée collective ;
$R_{Si}$ représente la $i^e$ coordonnée lente dans le système polyatomique ;
$R_S$ représente ($R_{S1}$, $R_{S2}$, ------, $R_{SM}$) ;
$m_i$ représente une masse de la $i^e$ coordonnée lente dans le système polyatomique ;
$\varphi_i^\mu$ et $\Lambda^\mu$ représentent chacun une coordonnée auxiliaire indépendante de $R_S$ ; et
$V_{eff}(R_S)$ représente une énergie potentielle effective.

**8.** Appareil de simulation (10) selon la revendication 7, **caractérisé en ce que** le nombre K de la/les coordonnée(s) collective(s) satisfait K = 1.

**9.** Appareil de simulation (10) selon la revendication 6, **caractérisé en ce que** le moyen d'extraction de coordonnées (18) est un moyen adapté pour effectuer un calcul selon une équation de base représentée par Formule 20 donnée ci-dessous, obtenue à la suite de l'augmentation du nombre de variables traitées comme indépendantes des coordonnées lentes et comme fonctions de la/les coordonnée(s) collective(s),

$$\frac{dZ}{dq^{\mu}} = \sum_{\nu=1}^{K} c^{\mu\nu} \mathbf{w}^{\mu} \qquad \text{pour} \qquad \mu = 1, \cdots, K$$

Formule 20

où : Z est un vecteur de dimension MK + M + 2K défini par Formule 21 donnée ci-dessous ;
$c^{\mu\nu}$ est une constante déterminée de manière unique de sorte que la valeur représentée par Formule 22 donnée ci-dessous, à définir par rapport à chaque $\mu$ soit minimisée ; et
$w^{\mu}$ représente des vecteur(s) unitaire(s) de dimension MK + M + 2K engendrant un espace de valeurs singulières de dimension K de matrice D définie par Formule 23 donnée ci-dessous,

$$Z \equiv \begin{pmatrix} \sqrt{m_i} Rs_i \\ \varphi_i^{\mu} \\ \lambda^{\mu} \\ \rho^{\mu} \end{pmatrix}$$

Formule 21

$$\sum_{i=1}^{M} \left( m_i^{1/2} \frac{dRs_i}{dq^{\mu}} - \varphi_i^{\mu} \right)^2$$

Formule 22

$$D \equiv \begin{pmatrix} \sum_{k=1}^{M} V_{,ijk}(Rs)\varphi_k^{\mu} & (V_{,ij}(Rs) - \lambda^{\mu}\delta_{ij})\delta^{\mu\nu} & -\varphi_i^{\mu}\delta^{\mu\nu} & 0 \\ V_{,ij}(Rs) & -\rho^{\nu}\delta_{ij} & 0 & -\varphi_i^{\nu} \\ 0 & \varphi_i^{\mu}\delta^{\mu\nu} & 0 & 0 \end{pmatrix}$$

Formule 23

où, $V_{,ij}(R_S)$ et $V_{,ijk}(R_S)$ sont définis respectivement par Formules 24 et 25 données ci-dessous,

$$V_{,ij}(Rs) \equiv (m_i m_j)^{-1/2} \frac{\partial^2}{\partial Rs_i \partial Rs_j} V_{eff}(Rs)$$

Formule 24

$$V_{,ijk}(Rs) \equiv (m_i m_j m_k)^{-1/2} \frac{\partial^3}{\partial Rs_i \partial Rs_j \partial Rs_k} V_{eff}(Rs)$$

Formule 25

où : i, j et k représentent chacun un entier compris entre 1 et M ;
$\mu$ et v représentent chacun un entier compris entre 1 et K ;
$q^{\mu}$ représente la $\mu^e$ coordonnée collective ;
$R_{Si}$ représente la $i^e$ coordonnée lente dans le système polyatomique ;
$R_S$ représente ($R_{S1}$, $R_{S2}$, ------, $R_{SM}$) ;
$m_i$ représente une masse de la $i^e$ coordonnée lente dans le système polyatomique ;
$\varphi_i^{\mu}$, $\lambda^{\mu}$ et $\rho^{\mu}$ représentent chacun une coordonnée auxiliaire indépendante de $R_S$ ; et
$V_{eff}(R_S)$ représente une énergie potentielle effective.

**10.** Appareil de simulation (10) selon la revendication 9, **caractérisé en ce que** le nombre K de la/les coordonnée(s) collective(s) satisfait K = 1.

**11.** Appareil de simulation (10) selon l'une quelconque des revendications 5 à 10, **caractérisé en ce que** le moyen d'extraction de coordonnées (18) est un moyen adapté pour calculer le terme de troisième dérivée de l'énergie potentielle sur la base de Formule 26 donnée ci-dessous,

$$\sum_{k=1}^{M} V_{,ijk}(Rs)\cdot\phi_k = \lim_{\Delta x\to 0}\frac{V_{,ij}(Rs+\mathbf{n}\Delta x)-V_{,ij}(Rs-\mathbf{n}\Delta x)}{2\Delta x}$$

Formule 26

où, $\varphi_i$ représente la $i^e$ composante d'un vecteur arbitraire, et $V_{,ij}(R_S)$, $V_{ijk}(R_S)$ et n sont définis respectivement par Formules 27 à 29 données ci-dessous.

$$V_{,ij}(Rs) \equiv (m_i m_j)^{-1/2}\frac{\partial^2}{\partial Rs_i \partial Rs_j}V_{eff}(Rs)$$

Formule 27

$$V_{,ijk}(Rs) \equiv (m_i m_j m_k)^{-1/2}\frac{\partial^3}{\partial Rs_i \partial Rs_j \partial Rs_k}V_{eff}(Rs)$$

Formule 28

$$\mathbf{n} \equiv (m_1^{-1/2}\phi_1,\cdots,m_i^{-1/2}\phi_i,\cdots,m_M^{-1/2}\phi_M)$$

Formule 29

**12.** Appareil de simulation (10) selon la revendication 1, **caractérisé en ce que** le moyen de réglage de coordonnées (16) est un moyen adapté pour régler des coordonnées représentatives extraites et représentant chaque structure partielle caractéristique de la structure du système polyatomique comme les coordonnées lentes,

la structure partielle est une structure secondaire, un bloc constitutif ou une chaîne principale de la macromolécule biologique,
la coordonnée représentative de chaque structure partielle est une coordonnée de chacun des atomes constituant la structure partielle, une coordonnée définie en combinant des coordonnées des atomes ou un pas des structures partielles,
la macromolécule biologique est une protéine, la structure partielle est une structure secondaire de la protéine, et la coordonnée lente de la structure secondaire est une coordonnée du centre de gravité d'un groupe d'atomes constituant la structure secondaire.

**13.** Appareil de simulation (10) selon la revendication 12, **caractérisé en ce que** la structure secondaire est au moins l'une d'une structure hélicoïdale, d'un feuillet β, d'une coudure, d'une boucle et d'une chaîne aléatoire.

**14.** Appareil de simulation (10) selon la revendication 1, **caractérisé en ce que** la macromolécule biologique est une protéine, la structure partielle est un résidu de la protéine, et la coordonnée lente du résidu est une coordonnée du centre de gravité d'un groupe d'atomes constituant le résidu.

**15.** Appareil de simulation (10) selon la revendication 1, **caractérisé en ce que** la macromolécule biologique est une protéine, la structure partielle est une chaîne principale de la protéine, et la coordonnée lente de la chaîne principale est une coordonnée de chaque atome constituant la chaîne principale.

**16.** Appareil de simulation (10) selon la revendication 1, **caractérisé en ce que** la macromolécule biologique est un acide nucléique, la structure partielle est une structure secondaire de l'acide nucléique, et la coordonnée lente de la structure secondaire est une coordonnée du centre de gravité d'un groupe d'atomes constituant la structure secondaire.

**17.** Appareil de simulation (10) selon la revendication 16, **caractérisé en ce que** la structure secondaire est une structure hélicoïdale.

**18.** Appareil de simulation (10) selon la revendication 1, **caractérisé en ce que** la macromolécule biologique est un acide nucléique, la structure partielle est un résidu de l'acide nucléique, et la coordonnée lente du résidu est une coordonnée du centre de gravité d'un groupe d'atomes constituant le résidu.

**19.** Appareil de simulation (10) selon la revendication 1, **caractérisé en ce que** la macromolécule biologique est un acide nucléique, la structure partielle est une chaîne principale de l'acide nucléique, et la coordonnée lente de la chaîne principale est une coordonnée de chaque atome constituant la chaîne principale.

**20.** Procédé de simulation destiné à être utilisé avec l'appareil de simulation (10) selon l'une quelconque des revendications 1 à 19 pour prédire un comportement d'un système polyatomique modélisé qui inclut un corps composite constitué de N atomes d'une macromolécule biologique et d'une molécule candidate de liaison, le procédé comprenant les étapes de :

régler des coordonnées lentes qui sont des M coordonnées données par des coordonnées de centres de gravité de structures partielles du système polyatomique sur la base de 3N coordonnées de point de masse décrivant une structure du système polyatomique ;
régler des coordonnées rapides qui sont des coordonnées d'atomes et des coordonnées décrivant la structure du système polyatomique et étant indépendantes des centres de gravité des structures partielles ;
obtenir les coordonnées rapides en fonction des coordonnées lentes ;
obtenir, en tenant compte de l'influence d'un changement de la position des atomes sur les structures partielles dû à un changement de la position dans les structures partielles, les coordonnées lentes en fonction de K coordonnée(s) collective(s) d'une coordonnée généralisée qui est associée aux coordonnées lentes par une transformation canonique, où la coordonnée généralisée est constituée d'une composante variable pour laquelle des relations de position relative entre les structures partielles varient dans le temps et d'une composante invariable qui sert de constante par rapport au temps, et la/les K coordonnée(s) collective(s) est/sont la composante variable de la coordonnée généralisée ; et
prédire une évolution temporelle du système polyatomique sur la base de la/les coordonnée(s) collective(s) en fonction du temps, qui peut être obtenue comme solution d'une équation de mouvement par rapport à la/les coordonnée(s) collective(s), aux coordonnées lentes et aux coordonnées rapides,
dans lequel K, M et N satisfont la relation $K < M < 3N$ et représentant chacun un entier supérieur ou égal à 1, dans lequel la structure des coordonnées lentes est obtenue par :

effectuer une première étape pour obtenir une énergie potentielle V représentée en fonction des coordonnées lentes et des coordonnées rapides ;
effectuer une deuxième étape pour représenter les coordonnées rapides en fonction des coordonnées lentes selon une condition d'approximation adiabatique en utilisant l'énergie potentielle ;
effectuer, dans un état actuel des coordonnées lentes et des coordonnées rapides, une troisième étape pour obtenir un coefficient différentiel de l'énergie potentielle par rapport aux coordonnées lentes en tenant compte de l'influence ;
effectuer, sous le coefficient différentiel de l'énergie potentielle, une quatrième étape pour obtenir un coefficient différentiel des coordonnées lentes par rapport à la/les coordonnée(s) collective(s) selon une équation de base de la méthode des coordonnées collectives auto-cohérentes en utilisant le coefficient différentiel de l'énergie potentielle ;
effectuer, sous le coefficient différentiel des coordonnées lentes, une cinquième étape pour mettre à jour la/les coordonnée(s) collective(s) d'une petite quantité et obtenir des coordonnées lentes mises à jour ;
effectuer, sous les coordonnées lentes mises à jour, une sixième étape pour effectuer une relaxation structurale sur les coordonnées rapides subordonnées aux coordonnées lentes ; et
ensuite, répéter les troisièmes à sixièmes étapes sur la base des coordonnées lentes et des coordonnées rapides dans un état après la relaxation structurale des coordonnées rapides.

**21.** Support d'enregistrement lisible par ordinateur sur lequel est enregistré un programme de simulation pour amener un ordinateur à effectuer le procédé de simulation selon la revendication 20.

# FIG.1

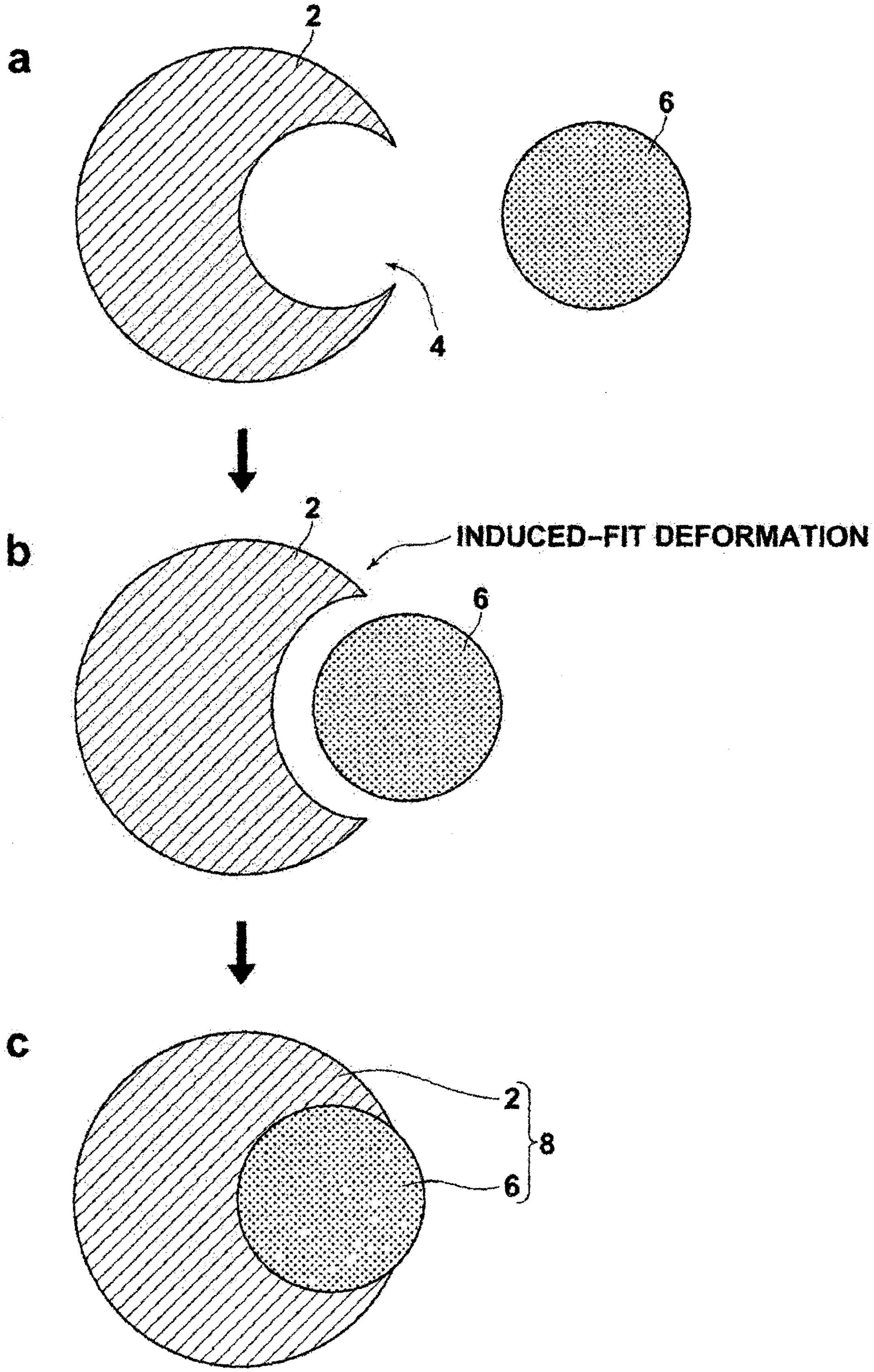

# FIG.2

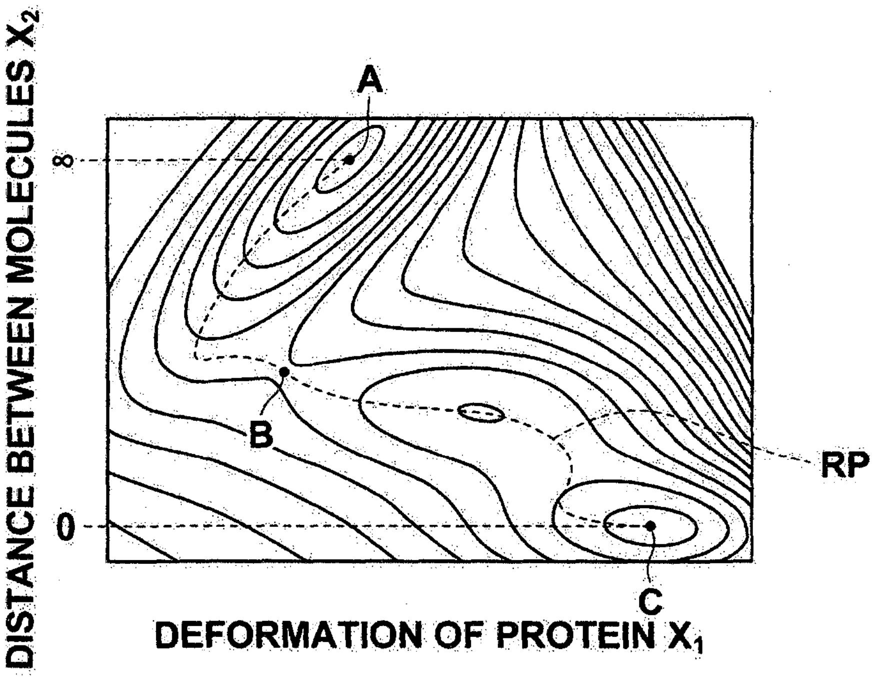
DISTANCE BETWEEN MOLECULES $X_2$
A
8
0
B
RP
C
DEFORMATION OF PROTEIN $X_1$

FIG.3

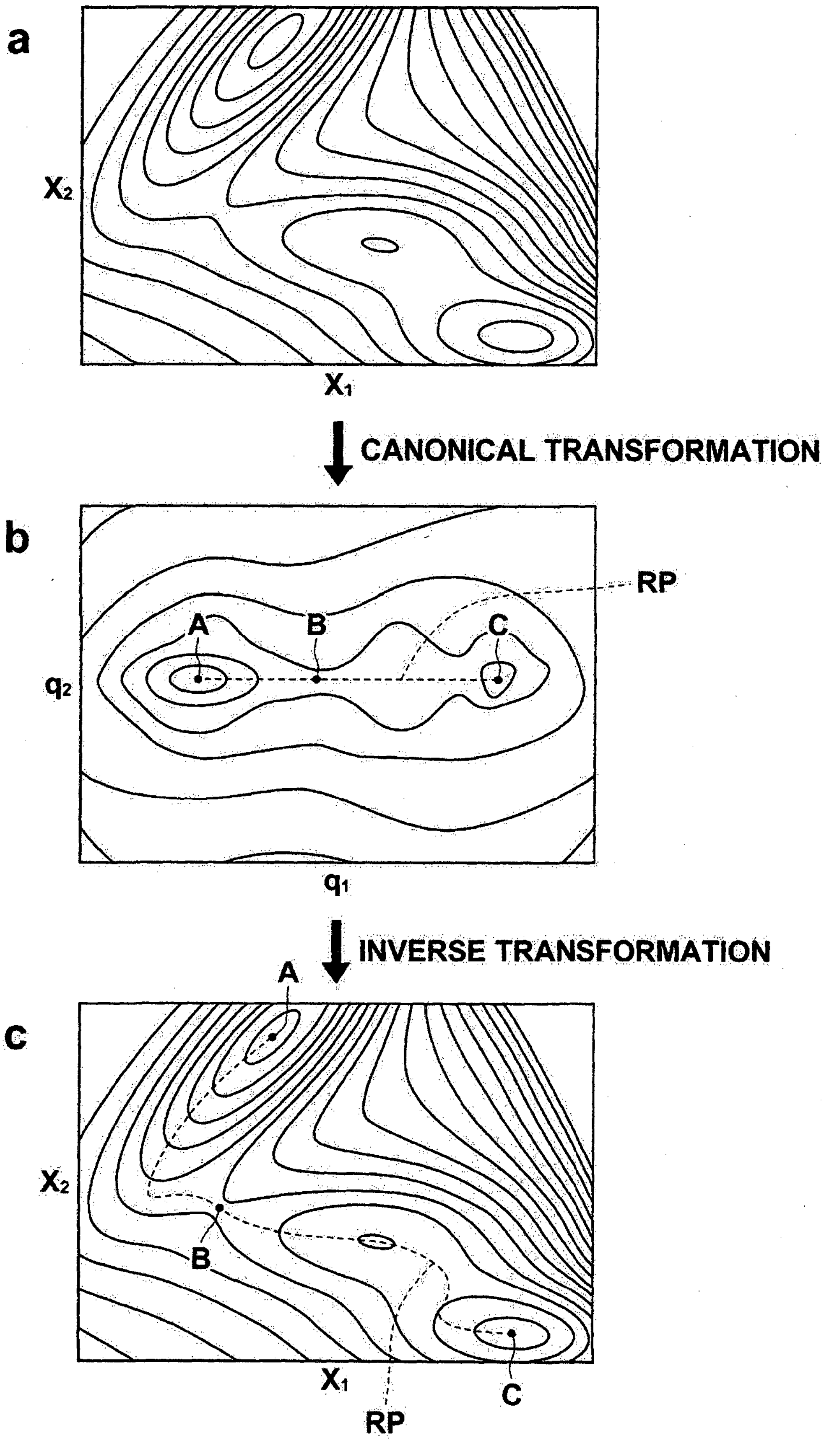
a
X2
X1
CANONICAL TRANSFORMATION
b
RP
A
B
C
q2
q1
INVERSE TRANSFORMATION
A
c
X2
B
X1
C
RP

FIG.4

a

$$x = (\underbrace{x_1, x_2, \cdots x_M}_{R_S}, \underbrace{x_{M+1}, x_{M+2}, \cdots x_{3N}}_{R_F})$$

↓

b

$$R_F = R_F(R_S)$$

↓

c

$$R_S = R_S(q^1)$$

↓

d

$$q^1 = q^1(t)$$

↓

e

$$x(t) = \begin{cases} x_1 = Rs_1(q^1 = q^1(t)) \\ x_2 = Rs_2(q^1 = q^1(t)) \\ \vdots \\ x_M = Rs_M(q^1 = q^1(t)) \\ x_{M+1} = R_{F_1}(Rs(q^1 = q^1(t))) \\ x_{M+2} = R_{F_2}(Rs(q^1 = q^1(t))) \\ \vdots \\ x_{3N} = R_{F_{3N-M}}(Rs(q^1 = q^1(t))) \end{cases}$$

FIG.5

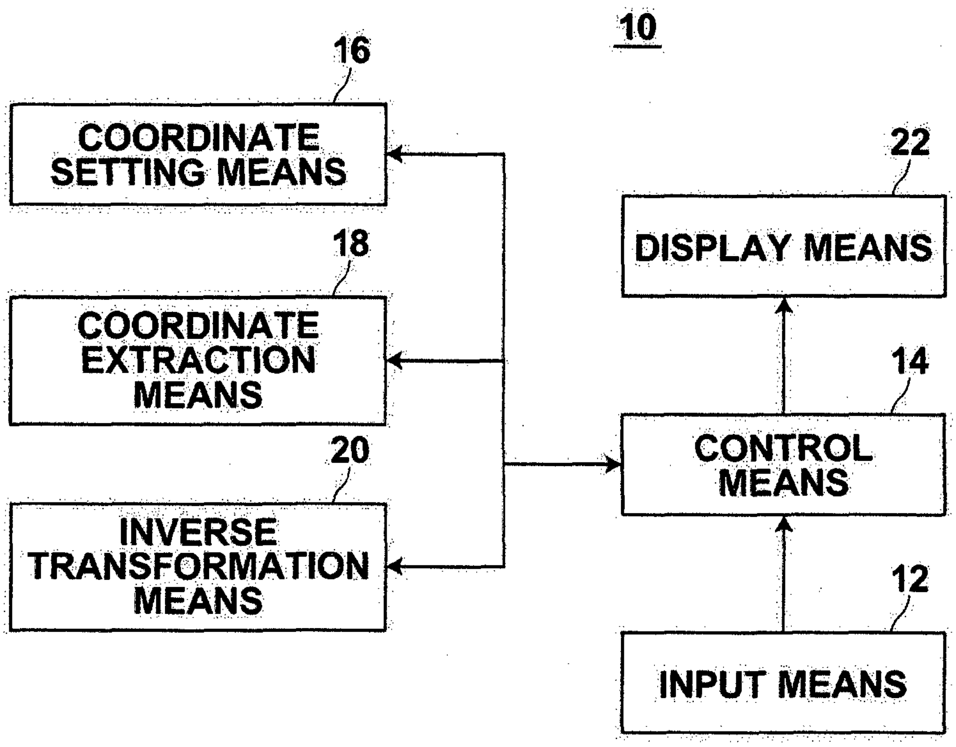
10
16
COORDINATE SETTING MEANS
18
COORDINATE EXTRACTION MEANS
20
INVERSE TRANSFORMATION MEANS
22
DISPLAY MEANS
14
CONTROL MEANS
12
INPUT MEANS

# FIG.6A

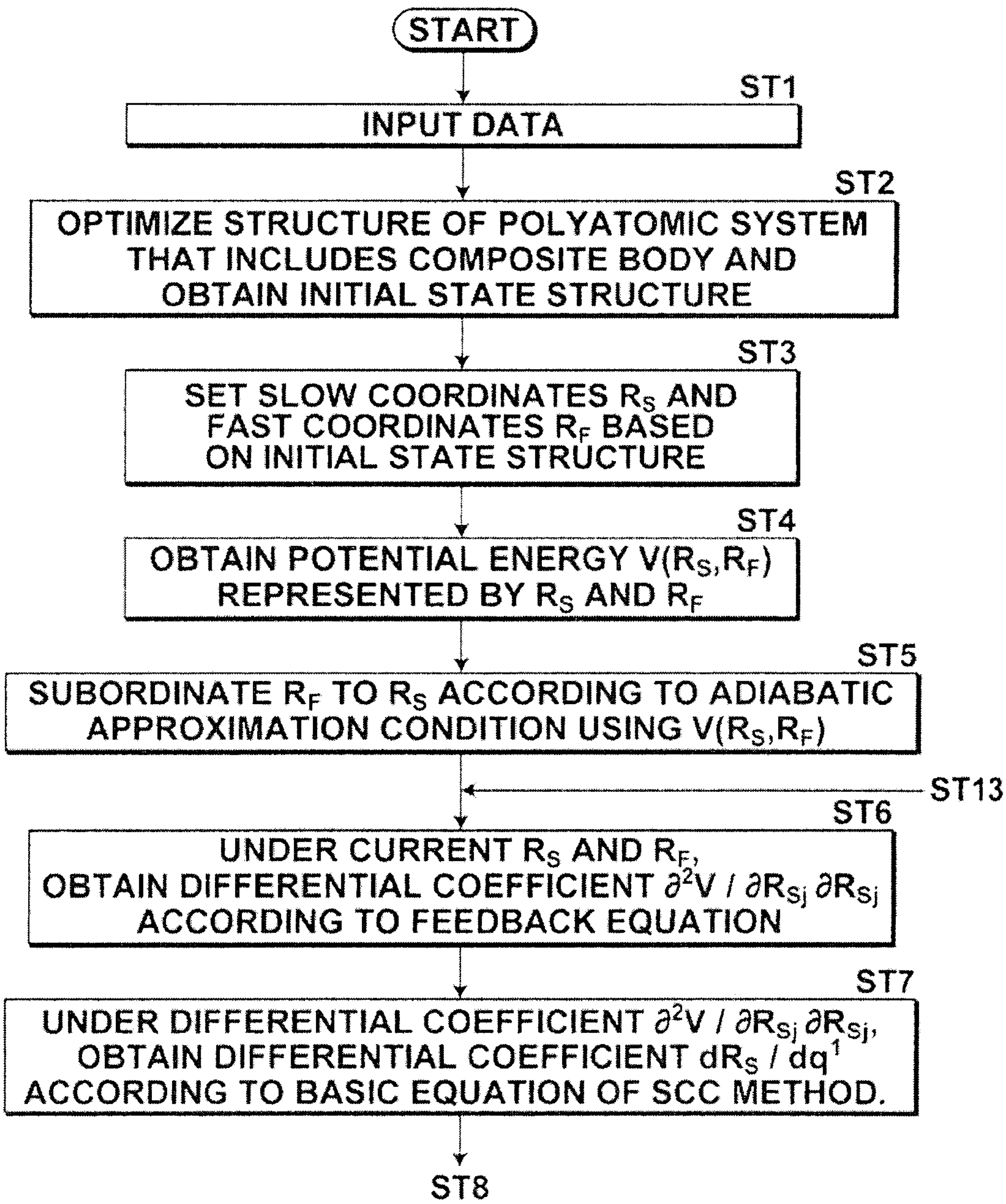
START
ST1
INPUT DATA
ST2
OPTIMIZE STRUCTURE OF POLYATOMIC SYSTEM THAT INCLUDES COMPOSITE BODY AND OBTAIN INITIAL STATE STRUCTURE
ST3
SET SLOW COORDINATES $R_S$ AND FAST COORDINATES $R_F$ BASED ON INITIAL STATE STRUCTURE
ST4
OBTAIN POTENTIAL ENERGY $V(R_S,R_F)$ REPRESENTED BY $R_S$ AND $R_F$
ST5
SUBORDINATE $R_F$ TO $R_S$ ACCORDING TO ADIABATIC APPROXIMATION CONDITION USING $V(R_S,R_F)$
ST13
ST6
UNDER CURRENT $R_S$ AND $R_F$, OBTAIN DIFFERENTIAL COEFFICIENT $\partial^2 V / \partial R_{Sj} \partial R_{Sj}$ ACCORDING TO FEEDBACK EQUATION
ST7
UNDER DIFFERENTIAL COEFFICIENT $\partial^2 V / \partial R_{Sj} \partial R_{Sj}$, OBTAIN DIFFERENTIAL COEFFICIENT $dR_S / dq^1$ ACCORDING TO BASIC EQUATION OF SCC METHOD.
ST8

FIG.6B

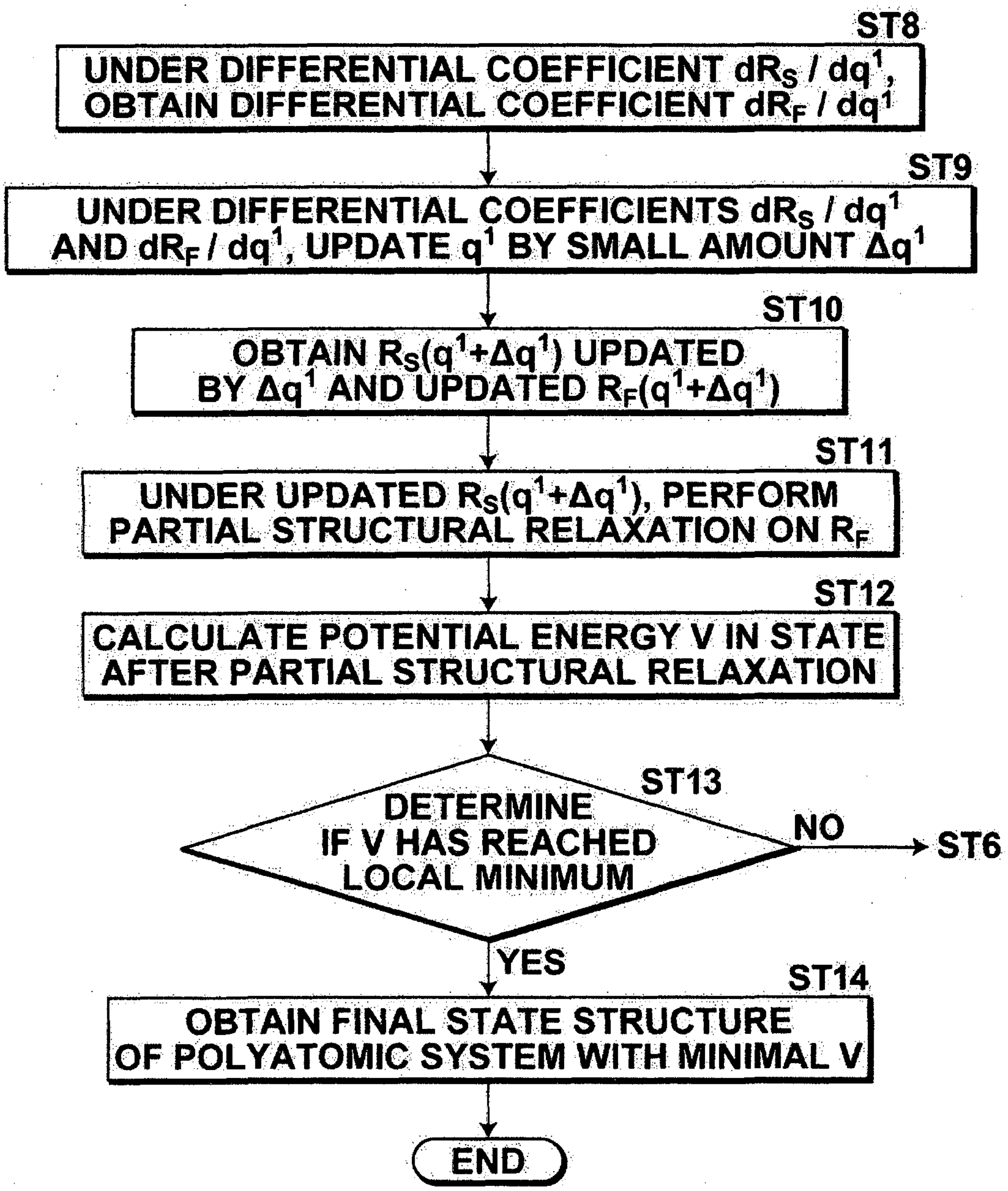
ST8
UNDER DIFFERENTIAL COEFFICIENT $dR_S / dq^1$, OBTAIN DIFFERENTIAL COEFFICIENT $dR_F / dq^1$
ST9
UNDER DIFFERENTIAL COEFFICIENTS $dR_S / dq^1$ AND $dR_F / dq^1$, UPDATE $q^1$ BY SMALL AMOUNT $\Delta q^1$
ST10
OBTAIN $R_S(q^1+\Delta q^1)$ UPDATED BY $\Delta q^1$ AND UPDATED $R_F(q^1+\Delta q^1)$
ST11
UNDER UPDATED $R_S(q^1+\Delta q^1)$, PERFORM PARTIAL STRUCTURAL RELAXATION ON $R_F$
ST12
CALCULATE POTENTIAL ENERGY V IN STATE AFTER PARTIAL STRUCTURAL RELAXATION
ST13
DETERMINE IF V HAS REACHED LOCAL MINIMUM
NO
ST6
YES
ST14
OBTAIN FINAL STATE STRUCTURE OF POLYATOMIC SYSTEM WITH MINIMAL V
END

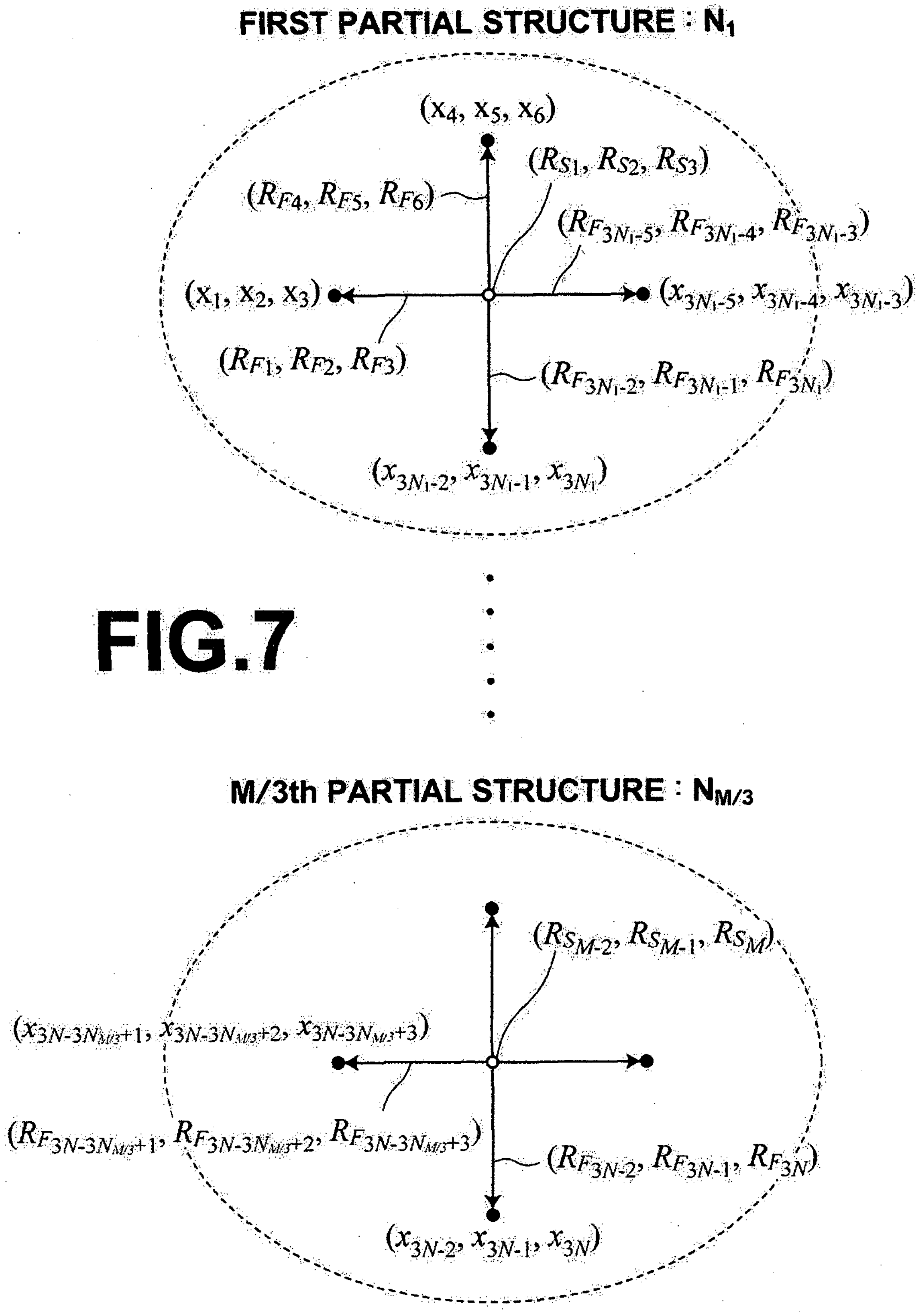
FIRST PARTIAL STRUCTURE : $N_1$
$(x_4, x_5, x_6)$
$(R_{S1}, R_{S2}, R_{S3})$
$(R_{F4}, R_{F5}, R_{F6})$
$(R_{F_{3N_1-5}}, R_{F_{3N_1-4}}, R_{F_{3N_1-3}})$
$(x_1, x_2, x_3)$
$(x_{3N_1-5}, x_{3N_1-4}, x_{3N_1-3})$
$(R_{F1}, R_{F2}, R_{F3})$
$(R_{F_{3N_1-2}}, R_{F_{3N_1-1}}, R_{F_{3N_1}})$
$(x_{3N_1-2}, x_{3N_1-1}, x_{3N_1})$
M/3th PARTIAL STRUCTURE : $N_{M/3}$
$(R_{S_{M-2}}, R_{S_{M-1}}, R_{S_M})$
$(x_{3N-3N_{M/3}+1}, x_{3N-3N_{M/3}+2}, x_{3N-3N_{M/3}+3})$
$(R_{F_{3N-3N_{M/3}+1}}, R_{F_{3N-3N_{M/3}+2}}, R_{F_{3N-3N_{M/3}+3}})$
$(R_{F_{3N-2}}, R_{F_{3N-1}}, R_{F_{3N}})$
$(x_{3N-2}, x_{3N-1}, x_{3N})$

FIG.7

# FIG.8

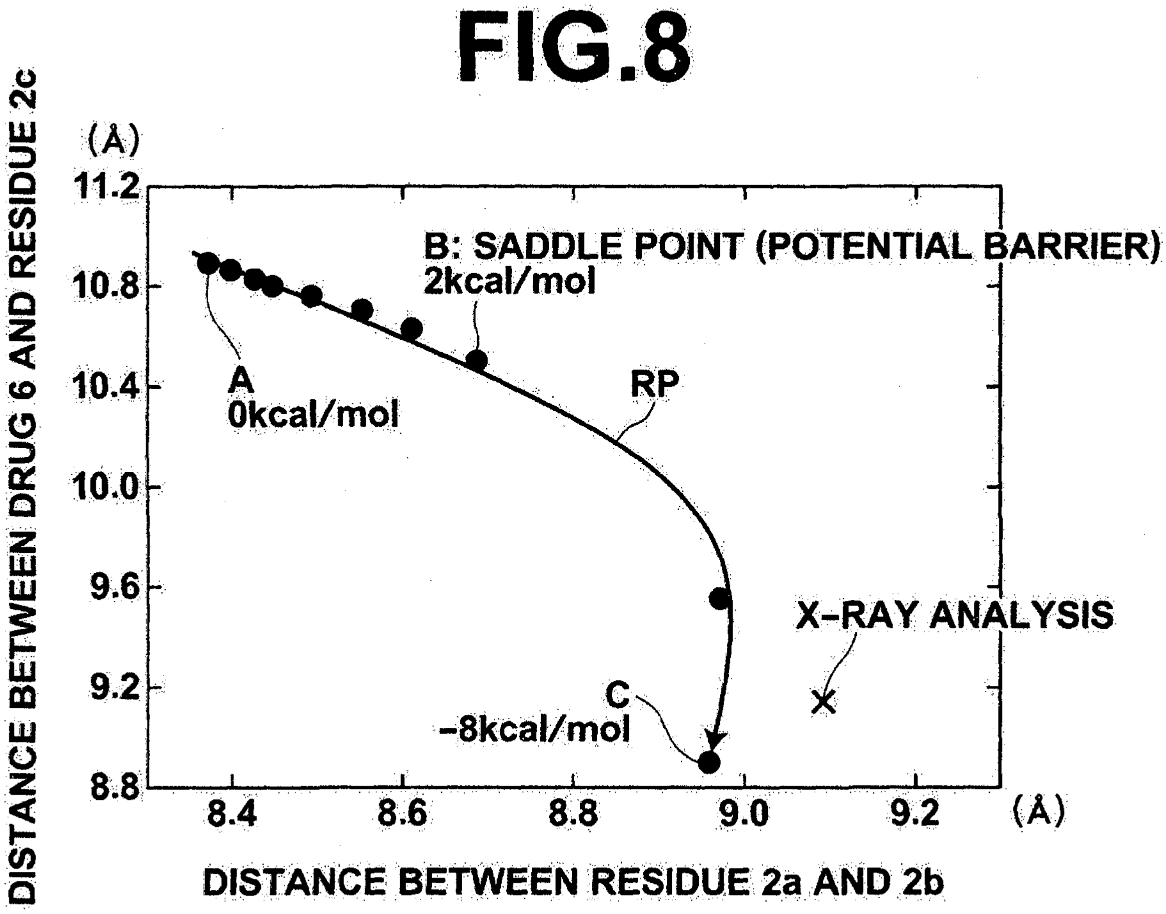
DISTANCE BETWEEN DRUG 6 AND RESIDUE 2c
(Å)
11.2
10.8
10.4
10.0
9.6
9.2
8.8
B: SADDLE POINT (POTENTIAL BARRIER)
2kcal/mol
A
0kcal/mol
RP
X-RAY ANALYSIS
C
-8kcal/mol
8.4
8.6
8.8
9.0
9.2
(Å)
DISTANCE BETWEEN RESIDUE 2a AND 2b

# FIG.9

a

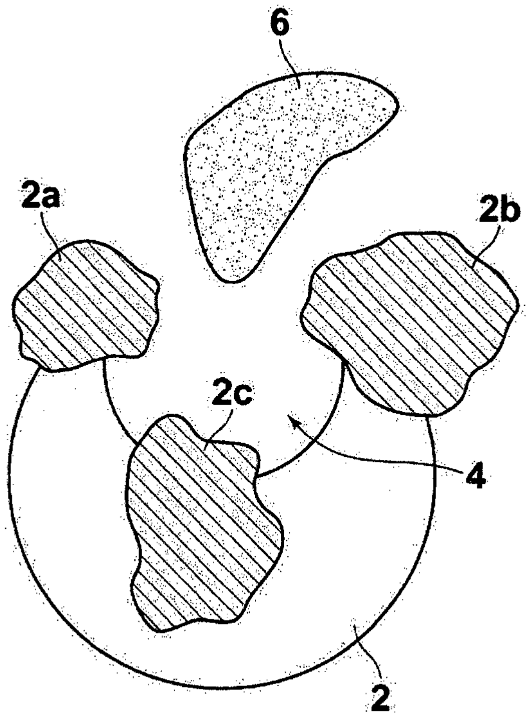


BINDING

b

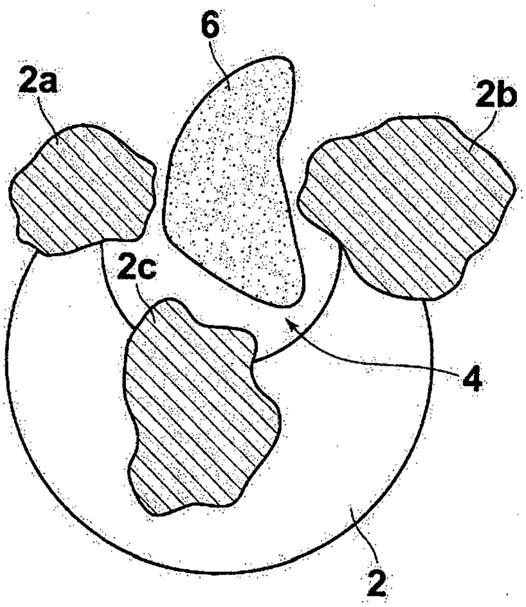

# FIG.10

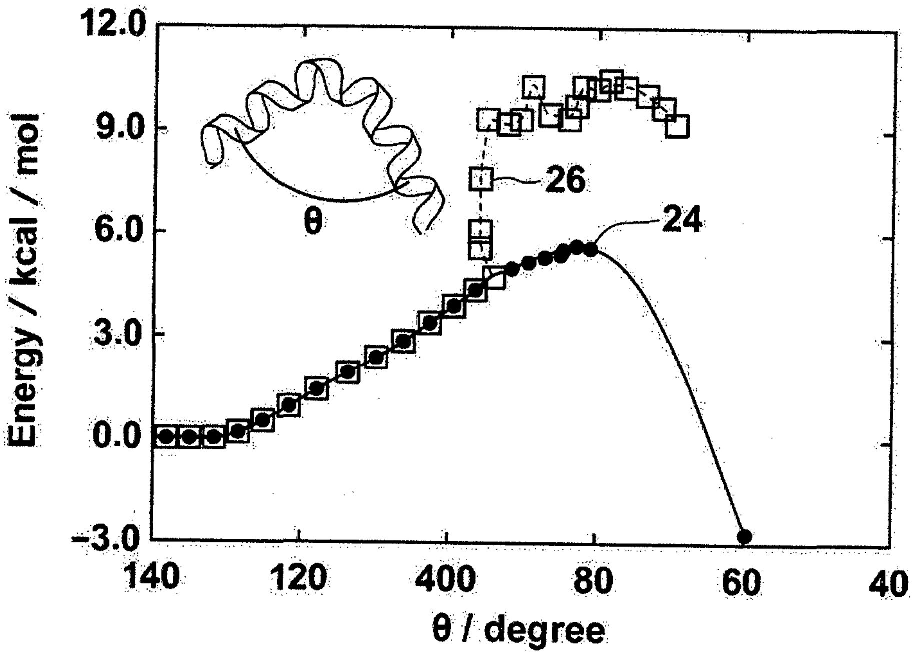
12.0
9.0
6.0
3.0
0.0
-3.0
Energy / kcal / mol
140
120
400
80
60
40
θ / degree
θ
26
24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005267592 A
- JP 2005524129 W
- JP 2006068271 W


### Non-patent literature cited in the description


- **T.J.A. EWING** ; **I.D. KUNTZ**. Critical evaluation of search algorithms for automated molecular docking and database screening. *Journal of Computational Chemistry*, 1997, vol. 18 (9), 1175-1189
- **G.M. MORRIS et al.** Automated docking using a Lamarckian genetic algorithm and an empirical binding free energy function. *Journal of Computational Chemistry*, 1998, vol. 19 (14), 1639-1662
- **M. RAREY et al.** A Fast Flexible Docking Method using an Incremental Construction. *Journal of Molecular Biology*, 1996, vol. 261 (3), 470-489
- **R. ABAGYAN et al.** ICM - A new method for protein modeling and design: Applications to docking and structure prediction from the distorted native conformation. *Journal of Computational Chemistry*, 1994, vol. 15 (5), 488-506
- **G. JONES et al.** Development and validation of a genetic algorithm for flexible docking. *Journal of Molecular Biology*, 1997, vol. 267 (3), 727-748
- **R.A. FRIESNER et al.** Glide: A New Approach for Rapid, Accurate Docking and Scoring. 1. Method and Assessment of Docking Accuracy. *Journal of Medicinal Chemistry*, 2004, vol. 47 (7), 1739-1749
- **T.A. HALGREN et al.** Glide: A New Approach for Rapid, Accurate Docking and Scoring. 2. Enrichment Factors in Database Screening. *Journal of Medicinal Chemistry*, 2004, vol. 47 (7), 1750-1759
- **Y. FUKUNISHI et al.** The Filling Potential Method: A Method for Estimating the Free Energy Surface for Protein-Ligand Docking. *THE JOURNAL OF PHYSICAL CHEMISTRY B*, 2003, vol. 107 (47), 13201-13210
- **Y. SUGITA** ; **Y. OKAMOTO**. Replica-exchange molecular dynamics method for protein folding. *Chemical Physics Letters*, 1999, vol. 314 (1-2), 141-151
- **S. TOMONAGA**. Elementary Theory of Quantum-Mechanical Collective Motion of Particles, II. *Progress of Theoretical Physics*, 1955, vol. 13 (5), 482-496
- **T. MARUMORI et al.** Self-Consistent Collective-Coordinate Method for the Large-Amplitude Nuclear Collective Motion. *Progress of Theoretical Physics*, 1980, vol. 64 (4), 1294-1314
- **G.D. DANG et al.** Self-consistent theory of large-amplitude collective motion: applications to approximate quantization of nonseparable systems and to nuclear physics. *Physics Reports*, 2000, vol. 335 (3-5), 93-274
- **M. BORN** ; **J.R. OPPENHEIMER**. On the Quantum Theory of Molecules. *Ann. Physik*, 1927, vol. 84, 457-484
- Nonequilibrium Phase Transitions and Self-Organization in Physics, Chemistry, and Biology. **H. HAKEN**. Synergetics. Springer-Verlag, 1978